(19) Europäisches Patentamt
European Patent Office
Office européen des brevets

(11) EP 4 232 128 B1

(12) EUROPEAN PATENT SPECIFICATION

(45) Date of publication and mention of the grant of the patent:
**22.04.2026 Bulletin 2026/17**

(21) Application number: **21883437.2**

(22) Date of filing: **22.10.2021**

(51) International Patent Classification (IPC):
***A61M 16/06*** (2006.01) ***A61B 5/00*** (2006.01)
***F03G 5/00*** (2006.01) ***H02K 7/18*** (2006.01)
***A61B 5/296*** (2021.01) ***A61B 5/097*** (2006.01)
***A61B 5/297*** (2021.01) ***A61M 16/00*** (2006.01)
***F03G 7/00*** (2006.01) ***H02K 7/14*** (2006.01)
***G16H 20/40*** (2018.01) ***A61B 5/291*** (2021.01)

(52) Cooperative Patent Classification (CPC):
(C-Sets available)
**A61M 16/06; A61B 5/0077; A61B 5/291; A61B 5/296; A61B 5/297; A61B 5/4848; A61B 5/6803; A61B 5/7405; A61B 5/7455; A61M 16/0066; A61M 16/024; A61M 16/0616; A61M 16/0633; A61M 16/0683; A61M 16/0816;**
(Cont.)

(86) International application number:
**PCT/SG2021/050643**

(87) International publication number:
**WO 2022/086452 (28.04.2022 Gazette 2022/17)**

# (54) PATIENT INTERFACE

PATIENTENSCHNITTSTELLE

INTERFACE PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2020 SG 10202010467U**

(43) Date of publication of application:
**30.08.2023 Bulletin 2023/35**

(73) Proprietor: **ResMed Asia Pte. Ltd.**
**Singapore 639443 (SG)**

(72) Inventors:
- **VALIYAMBATH, Mohankumar Krishnan**
  **Singapore 639443 (SG)**
- **ESPINOSA, Xyrone Royd**
  **Singapore 639443 (SG)**

(74) Representative: **Vossius & Partner Patentanwälte Rechtsanwälte mbB**
**Siebertstraße 3**
**81675 München (DE)**

(56) References cited:
**WO-A1-2010/080709**
**WO-A1-2016/156594**
**US-A1- 2008 022 920**
**US-A1- 2012 234 323**
**US-A1- 2012 325 215**
**US-A1- 2014 261 425**
**US-A1- 2014 332 010**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**A61M 16/0833; A61M 16/0875; A61M 16/16; A61M 21/00; A61M 21/02; F03G 5/06; F03G 7/008; F03G 7/0252; G16H 20/40; H02K 7/14; H02K 11/215;** A61B 5/0075; A61B 5/097; A61B 5/4812; A61B 5/4815; A61B 2505/07; A61B 2505/09; A61B 2560/0214; A61B 2560/0242; A61B 2562/0204; A61B 2562/0219; A61B 2562/0271; A61B 2562/029; A61M 16/0051; A61M 16/0075; A61M 16/0622; A61M 16/0666; A61M 16/0672; A61M 16/0688; A61M 16/0825; A61M 16/0883; A61M 16/101; A61M 16/1055; A61M 16/107; A61M 16/1085; A61M 16/109; A61M 16/1095; A61M 16/12; A61M 16/161; A61M 16/20; A61M 2016/0021; A61M 2016/0024; A61M 2016/0027; A61M 2016/003; A61M 2021/0022; A61M 2021/0027; A61M 2202/0208; A61M 2202/0225; A61M 2205/0294; A61M 2205/15; A61M 2205/18; A61M 2205/21; A61M 2205/3306; A61M 2205/3317; A61M 2205/332; A61M 2205/3331; A61M 2205/3334; A61M 2205/3365; A61M 2205/3368; A61M 2205/3375; A61M 2205/3379; A61M 2205/3553; A61M 2205/3561; A61M 2205/3584; A61M 2205/3592; A61M 2205/3633; A61M 2205/3653; A61M 2205/3673; A61M 2205/42; A61M 2205/505; A61M 2205/52; A61M 2205/581; A61M 2205/582; A61M 2205/583; A61M 2205/7518; A61M 2205/8206; A61M 2205/8243; A61M 2205/825; A61M 2205/8256; A61M 2205/8262; A61M 2205/8275; A61M 2205/8287; A61M 2209/045; A61M 2209/086; A61M 2209/088; A61M 2210/04; A61M 2230/04; A61M 2230/06; A61M 2230/10; A61M 2230/14; A61M 2230/205; A61M 2230/30; A61M 2230/40; A61M 2230/432; A61M 2230/435; A61M 2230/50; A61M 2230/60; A61M 2230/62; A61M 2230/63; H02J 7/42; H02J 7/82; H02J 7/96; H10N 10/00

C-Sets
A61M 2202/0208, A61M 2202/0007;
A61M 2202/0225, A61M 2202/0085;
A61M 2230/04, A61M 2230/005;
A61M 2230/06, A61M 2230/005;
A61M 2230/10, A61M 2230/005;
A61M 2230/14, A61M 2230/005;
A61M 2230/205, A61M 2230/005;
A61M 2230/30, A61M 2230/005;
A61M 2230/40, A61M 2230/005;
A61M 2230/432, A61M 2230/005;
A61M 2230/435, A61M 2230/005;
A61M 2230/50, A61M 2230/005;
A61M 2230/60, A61M 2230/005;
A61M 2230/62, A61M 2230/005;
A61M 2230/63, A61M 2230/005

## Description

2 BACKGROUND OF THE TECHNOLOGY

2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hypoventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hypoventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

#### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

#### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

#### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched air at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

#### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory Therapy Systems

These respiratory therapies may be provided by a respiratory therapy system or device. Such systems and devices may also be used to screen, diagnose, or monitor a condition without treating it.
A respiratory therapy system may comprise a Respiratory Pressure Therapy Device (RPT device), an air circuit, a humidifier, a patient interface, an oxygen source, and data management.

#### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.
Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.
Certain masks may be clinically unfavourable for the present technology e.g. if they block airflow via the nose and only allow it via the mouth.
Certain masks may be uncomfortable or impractical for the present technology if they require a patient to insert a portion of a mask structure in their mouth to create and maintain a seal via their lips.
Certain masks may be impractical for use while sleeping, e.g. for sleeping while lying on one's side in bed with a head on a pillow.
The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.
As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.
CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.
While a mask for other applications (e.g. aviators) may not be suitable for use in treating sleep disordered breathing, a mask designed for use in treating sleep disordered breathing may be suitable for other applications.
For these reasons, patient interfaces for delivery of CPAP during sleep form a distinct field.

#### 2.2.3.1.1 Seal-forming structure

Patient interfaces may include a seal-forming structure. Since it is in direct contact with the patient's face, the shape and configuration of the seal-forming structure can have a direct impact on the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design can fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT™ nasal pillows mask, SWIFT™ II nasal pillows mask, SWIFT™ LT nasal pillows mask, SWIFT™ FX nasal pillows mask and MIRAGE LIBERTY™ full-face mask. The following patent applications, assigned to ResMed Limited, describe examples of nasal pillows masks: International Patent Application WO2004/073,778 (describing amongst other things aspects of the ResMed Limited SWIFT™ nasal pillows), US Patent Application 2009/0044808 (describing amongst other things aspects of the ResMed Limited SWIFT™ LT nasal pillows); International Patent Applications WO 2005/063,328 and WO 2006/130,903 (describing amongst other things aspects of the ResMed Limited MIRAGE LIBERTY™ full-face mask); International Patent Application WO 2009/052,560 (describing amongst other things aspects of the ResMed Limited SWIFT™ FX nasal pillows).

#### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 $cmH_2O$).

| RPT Device name | A-weighted sound pressure level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

#### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the onset of inhalation. This therapy mode is known as pulsed oxygen delivery (POD) or demand mode, in contrast with traditional continuous flow delivery more suited to stationary oxygen concentrators.

#### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system. For example, it would be useful to have performance data, such as data indicative of the effects of therapy on a patient, and/or data indicative of functioning of the patient interface, to enable greater control to be exercised over therapy.

Existing processes to communicate and manage such data can be one or more of costly, time-consuming, and error-prone.

#### 2.2.3.7 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 $cmH_2O$ pressure at 1m)

(* one specimen only, measured using test method specified in ISO 3744 in CPAP mode at 10 $cmH_2O$)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage™ (*) | nasal | 29.5 | 21.5 | 1998 |
| ResMed UltraMirage™ | nasal | 36 (3) | 28 (3) | 2000 |
| ResMed Mirage Activa™ | nasal | 32 (3) | 24 (3) | 2002 |
| ResMed Mirage Micro™ | nasal | 30 (3) | 22 (3) | 2008 |
| ResMed Mirage™ SoftGel | nasal | 29 (3) | 22 (3) | 2008 |
| ResMed Mirage™ FX | nasal | 26 (3) | 18 (3) | 2010 |
| ResMed Mirage Swift™ (*) | nasal pillows | 37 | 29 | 2004 |
| ResMed Mirage Swift™ II | nasal pillows | 28 (3) | 20 (3) | 2005 |
| ResMed Mirage Swift™ LT | nasal pillows | 25 (3) | 17 (3) | 2008 |
| ResMed AirFit P10 | nasal pillows | 21 (3) | 13 (3) | 2014 |

Sound pressure values of a variety of objects are listed below

| Object | A-weighted sound pressure dB(A) | Notes |
|---|---|---|
| Vacuum cleaner: Nilfisk<br>Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

US 2014/261425 A1 relates to a wearable self-contained device that provides energy-efficient Positive Airway Pressure (PAP) to treat Obstructive Sleep Apnea (OSA). This device includes a primary airflow member and a bolus airflow member which operate together in a coordinated manner to provide just the right amount of pressure that is needed at any given time in order to keep the person's airway open in the most energy-efficient manner. The bolus airflow member can accumulate energy over multiple respiratory cycles and use this energy to release a bolus of air when needed. Energy efficiency is particularly critical for wearable self-contained Positive Airway Pressure (PAP) systems that a person can wear on their head without a tube connection to a bedside unit or a continuous wire connection to an external power source.

US 2012/234323 A1 relates to a method, device, and system to provide respiratory assistance to people with Obstructive Sleep Apnea (OSA), or other respiratory conditions, comprising harvesting and storing energy from gas outflow during exhalation and using that stored energy to increase gas inflow during inhalation. In an example, this disclosure may provide Positive End-Expiratory Pressure (PEEP) or Continuous positive Airway Pressure (CPAP). This disclosure can be embodied in a self-contained energy-harvesting positive airway pressure mask, nasal inserts, or mouth appliance. This disclosure offers a combination of the following five benefits for Obstructive Sleep Apnea (OSA) treatment: minimally-invasive; energy self-sufficient; freedom of movement; hypercapnia avoidance; and adjustable energy harvesting over multiple respiratory cycles

The invention is defined by the appended claims.

## 3 BRIEF SUMMARY OF THE TECHNOLOGY

The invention is set out in the appended set of claims. The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a patient interface that comprises one or more electronic components for monitoring, diagnosing and/or treating a patient, the one or more electronic components being provided in, or on, one or more of a plenum chamber, a seal-forming structure and a positioning and stabilising structure of the patient interface; wherein the patient interface comprises and/or is communicable with a power system for powering the one or more electronic components.

In some forms of the present technology, a patient interface comprises: a plenum chamber pressurisable to a therapeutic pressure of at least 4 cmH2O above ambient air pressure, said plenum chamber including a plenum chamber inlet port sized and structured to receive a flow of air at the therapeutic pressure for breathing by a patient; a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; a positioning and stabilising structure to provide a force to hold the seal-forming structure in a therapeutically effective position on the patient's head; and one or more electronic components for monitoring, diagnosing and/or treating the patient, the one or more electronic components being provided in, or on, one or more of the plenum chamber, the seal-forming structure and the positioning and stabilising structure; wherein the patient interface comprises and/or is communicable with a power system for powering the one or more electronic components.

In one form, the power system comprises an energy-harvesting system for converting energy generated during use of the patient interface to electrical energy for powering the one or more electronic components. The energy-harvesting system may provide power directly to the one or more electronic components and/or charge an electrical energy storage device, such as a battery or capacitor, that does so. Accordingly, the need for a separate power source or recharging mechanism may be reduced or even eliminated.

For example, the energy generated during use of the patient interface may be one or more of: mechanical energy generated by or during breathing; mechanical energy generated by movement of the patient; mechanical energy from air flow within the patient interface; and thermal energy from the patient's skin and/or from exhaled air.

In one form of the present technology, the energy harvesting system comprises at least one energy-harvesting device positioned in an air flow path of the patient interface. For example, this may be positioned at an air inlet of the patient interface, such as within a plenum chamber inlet port, or adjacent to the plenum chamber inlet port and within the plenum chamber.

The at least one energy-harvesting device may comprise a turbine generator. The turbine generator may comprise a rotor that comprises a plurality of turbine blades and a plurality of magnets disposed around a periphery of the rotor. In some forms, the turbine generator comprises a sealed stator assembly housing a plurality of coils, the stator assembly being disposed about the rotor.

The at least one energy-harvesting device may comprise a turbine generator. The turbine generator may comprise a rotor that comprises a plurality of turbine blades and a plurality of magnets that are positioned toward the base of the turbine blades. In some forms, the stator coils may be embedded inside the fixed axle around which the rotor rotates.

In some forms, positioning the magnets proximate to the base of the turbine blades may create a low rotor inertia (e.g., as compared to positioning the mass of the magnets towards the periphery of the turbine blades). This may allow the turbine blades to rotate even at a relatively low air flow.

The energy-harvesting system may alternatively, or additionally, comprise at least one piezoelectric film. For example, at least one said piezoelectric film may be attached to or disposed within the plenum chamber and/or the seal-forming structure and/or the positioning and stabilising structure.

The energy-harvesting system may alternatively, or additionally, comprise at least one thermoelectric generator (TEG) module.

For example, at least one TEG module may be disposed within the positioning and stabilising structure. The at least one TEG module may be arranged to contact skin of the patient.

In another example, at least one TEG module is arranged such that a first surface of said TEG module is exposed to an interior of the plenum chamber, and a second surface that is opposite the first surface is exposed to ambient.

In some forms of the present technology, the power system comprises an external charging circuit that is configured to charge the electrical energy storage device.

For example, the external charging circuit may be a component of a respiratory pressure therapy device that is configured to deliver the flow of air to the plenum chamber. In some forms, the external charging circuit is connectable to the electrical energy storage device via one or more cables incorporated in or on an air circuit that connects the respiratory pressure therapy device and the plenum chamber.

In some forms of the present technology, the one or more electronic components comprise one or more sensors and/or one or more actuators.

In some forms, at least one sensor and/or at least one actuator may be partly exposed to ambient at an exterior surface of the positioning and stabilising structure; and/or be partly exposed at a patient-contacting surface of the positioning and stabilising structure, so as to contact skin of the patient in use.

In some forms, at least one sensor and/or at least one actuator is at least partly embedded between an outer layer and a patient-contacting layer of the positioning and stabilising structure.

In some forms of the present technology, at least one sensor and/or at least one actuator comprises circuitry that is at least partially formed by one or more conductive threads, and/or one or more conductive ink traces.

In some forms of the present technology, the one or more electronic components comprise a wireless communications interface for transmitting data from the one or more sensors to one or more external computing devices, and/or for receiving data at the one or more actuators from the one or more external computing devices.

For example, the one or more sensors and/or one or more actuators may comprise one or more of: an accelerometer; a gyroscope; a humidity sensor; a temperature sensor; a microphone; a camera; a pulse oximeter; an EEG sensor; an EMG sensor; an EOG sensor; a touch sensor; a vibration device; and an audio output device.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Another aspect of one form of the present technology comprises a patient interface comprising: a plenum chamber pressurisable to a therapeutic pressure of at least 4 $cmH_2O$ above ambient air pressure, said plenum chamber including a plenum chamber inlet port sized and structured to receive a flow of air at the therapeutic pressure for breathing by a patient; a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use; a positioning and stabilising structure to provide a force to hold the seal-forming structure in a therapeutically effective position on the patient's head; and a power system for converting energy generated during use of the patient interface to converted electrical energy for powering at least one portion of the patient interface, wherein the energy generated during use of the patient interface is one or more of: mechanical energy generated by or during breathing; mechanical energy generated by movement of the patient; mechanical energy from air flow within the patient interface; and thermal energy from the patient's skin and/or exhaled air.

In some forms the power system provides a DC voltage to the at least one portion of the patient interface.

In some forms: a) the at least one portion of the patient interface is an electrical energy storage device electrically connected to the power system and configured to provide electrical energy to the power system; b) the power system is configured to provide the converted electrical energy to the electrical storage device to recharge the electrical energy storage device; c) an external charging circuit is electrically connected to the electrical energy storage device and is configured to provide electrical energy to electrical energy storage device; d) the at least one portion of the patient interface further includes one or more electronic components for monitoring, diagnosing and/or treating the patient; e) the one or more electronic components receives electrical energy directly from the power system; and/or f) the one or more electronic components receives electrical energy directly from the electrical energy storage device.

In some forms the at least one portion of the patient interface further includes one or more electronic components for monitoring, diagnosing and/or treating the patient, the one or more electronic components being provided in, or on, one or more of the plenum chamber, the seal-forming structure and the positioning and stabilising structure.

In some forms: a) the at least one energy-harvesting device comprises a turbine generator; b) the turbine generator comprises a rotor that comprises a plurality of turbine blades and a plurality of magnets disposed around a periphery of the rotor; c) the turbine generator comprises a sealed stator assembly housing a plurality of coils, the stator assembly being disposed about the rotor; d) the sealed stator assembly further includes a plurality of bobbins, the plurality of coils wrapped around the plurality of bobbins; e) a number of bobbins in the plurality of bobbins is equal to a number of magnets in the plurality of magnets; f) the turbine generator is positioned in air inlet of the patient interface, upstream from the patient in use; and/or g) the turbine generator is positioned in air outlet of the patient interface, downstream from the patient in use.

In some forms: a) the at least one energy-harvesting system comprises at least one piezoelectric film; b) at least one said piezoelectric film is attached to or disposed within the plenum chamber and/or the seal-forming structure and/or the positioning and stabilising structure; c) said piezoelectric film is a sensor and is configured to measure patient snoring by sensing vibration and/or noise; d) the piezoelectric film is positioned in air inlet of the patient interface, upstream from the patient in use; e) the piezoelectric film is positioned in air outlet of the patient interface, downstream from the patient in use; f) the at least one piezoelectric film is sandwiched between layers of the positioning and stabilising structure; and/or g) the at least one piezoelectric film configured to flex as a result of movement in the positioning and stabilising structure.

In some forms: a) the at least one energy-harvesting system comprises at least one thermoelectric generator (TEG) module; b) at least one TEG module is at least partially exposed and is arranged to contact skin of the patient; c) at least one TEG module is disposed within the positioning and stabilising structure; d) at least one TEG module is arranged such that a first surface of said TEG module is exposed to an interior of the plenum chamber, and a second surface that is opposite the first surface is exposed to ambient.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

## 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

4.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

Fig. 2E is a further side view of a head. The approximate locations of the Frankfort horizontal and nasolabial angle are indicated. The coronal plane is also indicated.

Fig. 2F shows a base view of a nose with several features identified including naso-labial sulcus, lip inferior, upper Vermilion, naris, subnasale, columella, pronasale, the major axis of a naris and the midsagittal plane.

Fig. 2G shows a side view of the superficial features of a nose.

Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.

Fig. 2I shows a medial dissection of a nose, approximately several millimeters from the midsagittal plane, amongst other things showing the septum cartilage and medial crus of greater alar cartilage.

Fig. 2J shows a front view of the bones of a skull including the frontal, nasal and zygomatic bones. Nasal concha are indicated, as are the maxilla, and mandible.

Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

Fig. 2L shows an anterolateral view of a nose.

4.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows a cushion for a mask that includes two pillows. An exterior surface of the cushion is indicated. An edge of the surface is indicated. Dome and saddle regions are indicated.

Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

Fig. 3I shows the surface of a structure, with a one dimensional hole in the surface. The illustrated plane curve forms the boundary of a one dimensional hole.

Fig. 3J shows a cross-section through the structure of Fig.3I. The illustrated surface bounds a two dimensional hole in the structure of Fig. 3I.

Fig. 3K shows a perspective view of the structure of Fig. 3I, including the two dimensional hole and the one dimensional hole. Also shown is the surface that bounds a two dimensional hole in the structure of Fig. 3I.

Fig. 3L shows a mask having an inflatable bladder as a cushion.

Fig. 3M shows a cross-section through the mask of Fig. 3L, and shows the interior surface of the bladder. The interior surface bounds the two dimensional hole in the mask.

Fig. 3N shows a further cross-section through the mask of Fig. 3L. The interior surface is also indicated.

Fig. 3O illustrates a left-hand rule.

Fig. 3P illustrates a right-hand rule.

Fig. 3Q shows a left ear, including the left ear helix.

Fig. 3R shows a right ear, including the right ear helix.

Fig. 3S shows a right-hand helix.

Fig. 3T shows a view of a mask, including the sign of the torsion of the space curve defined by the edge of the sealing membrane in different regions of the mask.

Fig. 3U shows a view of a plenum chamber 3200 showing a sagittal plane and a mid-contact plane.

Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.

Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

Fig. 3Y shows a patient interface in the form of a nasal cannula in accordance with one form of the present technology.

Fig. 4 depicts a superior perspective view of a patient interface according to an example of the present technology.

Fig. 5 depicts an anterior perspective view of a patient interface according to an example of the present technology.

Fig. 6A depicts an anterior perspective view of a patient interface according to an example of the present technology worn by a patient.

Fig. 6B depicts an anterior view of a patient interface according to an example of the present technology worn by a patient.

Fig. 7A shows a positioning and stabilising structure for a patient interface in accordance with one form of the present technology, in a first in-use position on a patient's head.

Fig. 7B shows the positioning and stabilising structure of Fig. 7A, in a second in-use position on a patient's head as part of a patient interface.

Fig. 8 is a schematic cross-section through part of the positioning and stabilising structure of Fig. 7A.

Fig. 9A is a block diagram of a patient interface comprising a plurality of electronic modules that are powered by a rechargeable battery that is charged by an external charging circuit.

Fig. 9B is a schematic depiction of a first arrangement for supplying power to electronic components of the patient interface of Fig. 9A.

Fig. 9C is a schematic depiction of a second arrangement for supplying power to electronic components of the patient interface of Fig. 9A.

Fig. 9D is a schematic depiction of a third arrangement for supplying power to electronic components of the patient interface of Fig. 9A.

Fig. 10 is a block diagram of a patient interface comprising a plurality of electronic modules that are powered by an energy harvesting system.

Fig. 11A is an exploded view of an example of an energy-harvesting device for a patient interface.

Fig. 11B is another view showing various components of the energy-harvesting device of Fig. 11A.

Fig. 11C is a plan view of a partially assembled energy-harvesting device.

Fig. 11D is a plan view of the energy-harvesting device in fully assembled form.

Fig. 11E is a front view of an alternate example of a rotor.

Fig. 12A shows a first series of voltage signals from a patient interface having a piezoelectric film-based energy-harvesting device incorporated therein.

Fig. 12B shows a second series of voltage signals from the patient interface having a piezoelectric film-based energy-harvesting device incorporated therein.

4.4 RPT DEVICE

Fig. 13A shows an RPT device in accordance with one form of the present technology.

Fig. 13B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 13C is a schematic diagram of the electrical components of an RPT device in accordance with one form of the present technology.

Fig. 13D is a schematic diagram of the algorithms implemented in an RPT device in accordance with one form of the present technology.

Fig. 13E is a flow chart illustrating a method carried out by the therapy engine module of Fig. 13D in accordance with one form of the present technology.

4.5 HUMIDIFIER

Fig. 14A shows an isometric view of a humidifier in accordance with one form of the present technology.

Fig. 14B shows an isometric view of a humidifier in accordance with one form of the present technology, showing a humidifier reservoir 5110 removed from the humidifier reservoir dock 5130.

Fig. 14C shows a schematic of a humidifier in accordance with one form of the present technology.

5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 6000.

5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy. One example of such a patient interface 3000 is shown in Fig. 3A.

Figs. 4 and 5 depict a patient interface 3000 according to another example of the present technology. The patient interface 3000 includes a seal-forming structure 3100 that is connected to the plenum chamber 3200. The plenum chamber 3200 may be provided with one or more vents 3400.

The patient interface 3000 of Figs. 4 and 5 may include a positioning and stabilising structure 3300 that includes conduits 3301. The conduits 3301 serve two purposes: 1) to position and stabilize the patient interface 3000 on the patient's head in a therapeutically effective position during use and 2) to provide the pressurized, breathable gas to the plenum chamber 3200. As such, the conduits 3301 may be constructed of a flexible, biocompatible material and may also form a hollow structure. The conduits 3301 may be connected to the plenum chamber 3200 with clips 3303 to provide a pneumatic connection therebetween. The conduits 3301 may also include strap connectors 3302 to connect to a strap (not shown) that passes behind the patient's head in use. The conduits 3301 may also include flexible portions 3304 that provide flexibility to the conduits 3301 to accommodate different sizes and shapes of patient heads. The patient interface 3000 includes an elbow connector 3305 to connect the decoupling structure 3500. The elbow connector 3305 may be hollow to allow gas from the conduit 3301 to pass through the decoupling structure 3500, through the elbow connector 3305, and into the conduits 3301.

In one form the patient interface 3000 includes at least one decoupling structure 3500, for example, a swivel or a ball and socket. The decoupling structures 3500 may be in the form of an elbow. The decoupling structures 3500 may include a swivel that connects to the air circuit 4170 and a patient interface connector (not shown) that connects to the patient interface 3000. The patient interface connector may permit the decoupling structure 3500 to rotate relative to the patient interface 3000. The decoupling structures 3500 may also include vent holes 3401. These vent holes 3401 may allow for pressurized gas delivered to the patient interface 3000 to be bled off. It is also possible for exhaled $CO_2$ from the patient to escape to atmosphere via the vent holes 3401 in the decoupling structure 3500. In another example, the vent holes can be provided in an added component, e.g., an adapter in the form of a relatively short flexible or rigid conduit (2-10 cm) with holes attached to the patient interface 3000, e.g., connected to the decoupling structure 3500. The short conduit can be sold with a patient interface and include an appropriate number of holes to allow bleed off of the pressurized gas to tune the therapeutic pressure to an appropriate level for the given patient interface 3000. The $CO_2$ vent and the bleed off vent may have the same or different capacities, e.g., the bleed off vent may provide a higher or lower flow rate than the $CO_2$ vent.

Figs. 6A and 6B show this patient interface 3000 on a patient. A strap 3306 may be joined to the strap connectors 3302 to secure the patient interface 3000 in a desired sealing position for therapy.

Further description of exemplary patient interfaces 3000, attributes of which may be applied to the present technology, is provided by International Publication No. WO2017/185140.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula-type unsealed patient interface 3800. The lumens 3820a, 3820b lead from the nasal cannula-type unsealed patient interface 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the nasal cannula-type unsealed patient interface 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure above ambient pressure.

The patient interface 3000 in accordance with one form of the present technology is constructed and arranged to be able to provide a supply of air at a positive pressure of at least 2 $cmH_2O$ with respect to ambient.

The patient interface 3000 in accordance with one form of the present technology is constructed and arranged to be able to provide a supply of air at a positive pressure of at least 4 $cmH_2O$ with respect to ambient.

The patient interface 3000 in accordance with one form of the present technology is constructed and arranged to be able to provide a supply of air at a positive pressure of at least 6 $cmH_2O$ with respect to ambient.

The patient interface 3000 in accordance with one form of the present technology is constructed and arranged to be able to provide a supply of air at a positive pressure of at least 8 $cmH_2O$ with respect to ambient.

The patient interface 3000 in accordance with one form of the present technology is constructed and arranged to be able to provide a supply of air at a positive pressure of at least 10 $cmH_2O$ with respect to ambient.

The patient interface 3000 in accordance with one form of the present technology is constructed and arranged to be able to provide a supply of air at a positive pressure of at least 20 $cmH_2O$ with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

#### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Forehead region

In one form, the seal-forming structure that forms a seal in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap.

In one form of the present technology, as shown in Fig. 7A and 7B, a positioning and stabilising structure 6300 of a patient interface 6000 may have a connection port 6600 integrated therein, to facilitate transformation from a non-therapeutic configuration where the positioning and stabilising structure is worn as a headband, as in Fig. 7A, to a therapeutic configuration, as in Fig. 7B, where the positioning and stabilising structure 6300 is connectable to an air circuit 4170 which is in communication with a plenum chamber 6200, via the connection port 6600.

A number of different forms of connection between air circuit 4170 and connection port 6600 are possible, provided that a substantially air-tight seal is formed to prevent or significantly reduce pressure leakage during therapy. For example, the port 6600 may comprise magnetic elements 6610 located at an interior surface thereof for connection to corresponding magnetic elements located on a connector at one end of air circuit 4170. In another example, the connector of the air circuit may attach to connection port 6600 by a snap-fit (such as an annular snap-fit or cantilever snap-fit, either of which may be rigid-to-rigid or rigid-to-resilient) or friction fit.

As shown in Fig. 7A, the positioning and stabilising structure 6300 comprises an upper textile portion 6310 comprising a resilient circumferential band for fitting to the patient's head in use. A first lower textile portion 6320 is hingedly connected to (for example, integral with), and extends from, the upper textile portion 6310. The positioning and stabilising structure 6300 is used to apply a force to a seal-forming structure 6100, as shown in Fig. 7B. The seal-forming structure 6100 may be an nasal mask having a plenum chamber 6200. Other types of mask, such as full face masks and oro-nasal masks, may also be used with positioning and stabilising structure 6300 as part of a patient interface.

In this example, the positioning and stabilising structure 6300 is formed as a band having a front section 6302 and a back section 6304, where the front section 6302 has a first bifurcated section (spanning points 6312, 6314) such that a first fork forms a first part of the upper textile portion 6310 and a second fork forms the first lower textile portion 6320. Accordingly, the upper textile portion 6310 forms a first band or strap that can encircle the patient's forehead in use, as shown in Fig. 7A, while the first lower textile portion 6320 forms a second band or strap that is downwardly stretchable to engage directly or indirectly with seal-forming structure 6100 to provide a force to hold the seal-forming structure 6100 in a therapeutically effective position on the patient's head.

In some forms of the present technology, the back section 6304 may also comprise a bifurcated section, comprising a first rear portion 6306 that is a second part of the upper textile portion 6310 and a second rear portion or lower portion 6308 that is a second lower textile portion, as shown in Fig. 7B. The bifurcation of the back section 6304 enables a greater degree of support, since tension can be provided at spaced locations at the back of the patient's head or occiput or adjacent to the occipital bone, and also provides for a greater degree of adjustability such that the patient may better position the positioning and stabilising structure 6300 for greater comfort.

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

#### 5.3.3.1 Sensor and actuator arrangements for patient interfaces

In some forms of the present technology, a patient interface may have one or more sensors and/or actuators provided therein, for measurement of the patient's physiological and sleep data. One or more sensors and one or more actuators may respectively be embedded within the patient interface 3000 or 6000, for example between textile layers of headgear of the patient interface, or may be attached to internal and/or external surfaces of the headgear 3300, 6300 or other components of the patient interface. For example, one or more sensors and/or actuators may be integrated in a positioning and stabilising structure 3300, 6300, and/or in another component such as a seal-forming structure 3100, 6100 or plenum chamber 3200, 6200.

Advantageously, sensors embedded in the patient interface can help collect sleep-related data and physiological indicators such as vital data; this can be used to determine the improvement in sleep and health by comparing data before and after start of therapy. This data can be processed and the patient informed of how the therapy is improving sleep. For example, a patient may wear a positioning and stabilising structure 6300 with integrated sensors as a headband, as shown in Fig. 7A, before commencing therapy, and physiological and sleep data may be recorded while the patient is sleeping (and during the day, in the case of physiological data). After commencing therapy, with the positioning and stabilising structure 6300 in the therapeutic configuration shown in Fig. 7B, further physiological and sleep data may be recorded, and compared to the data recorded before commencement of therapy. The physiological and sleep data may be communicated to external computing devices such as a smartphone of the patient, and/or a monitoring server that is operated by or accessible to a clinician or other healthcare provider.

With a smoother acclimatisation due to the patient being able to wear the positioning and stabilising structure 6300 as a headband, and data-based feedback being obtained on how the therapy is actually helping (for example, via an application executing on the patient's smartphone), patient compliance is more likely. The data collected may also be useful in determining population-level sleep and/or physiological characteristics of one or more cohorts of patients undergoing respiratory therapy, thereby potentially enabling better customisation of therapy to patients falling within particular categories, or otherwise enabling optimisation of operation of an RPT device 4000.

In some forms of the present technology, measurement of functional parameters at the patient side using sensors integrated in and/or attached to the mask may provide improved, active feedback-based control of an RPT device 4000 to which the patient interface is connected, for example, improved feedback control of a pressure generator 4140 of the RPT device 4000 (Fig. 13B).

For example, referring to Fig. 7B and Fig. 8, upper textile portion 6310 of positioning and stabilising structure 6300 may have a plurality of electronic modules (actuators and/or sensors) 6354, 6356, 6358 and 6360 integrated therein. A processor module 6350 may also be integrated in the positioning and stabilising structure 6300, typically also in the upper textile portion 6310, though it will be appreciated that the processor module 6350 may be located elsewhere within the positioning and stabilising structure 6300. The processor module may be disposed within a retaining structure 6380. The processor module 6350 may have an integrated transceiver for transmitting data to, and receiving data from, external computing devices. An electrical energy storage device such as a battery module 6352 may also be included to power the various electronic components (sensors/actuators 6354-6360 and processor module 6350) of the positioning and stabilising structure 6300. In some forms of the present technology, other electrical energy storage devices such as supercapacitors may be used.

As shown in Fig. 8, the sensors and associated electronics may be integrated at least partly between fabric layers of the upper textile portion 6310. For example, various sensor/actuator modules and/or associated circuitry, the processor module 6350, and the battery module 6352 may lie between an inner, patient-contacting, fabric layer 6370, and an outer, non-patient-contacting, fabric layer 6372.

The sensor and/or actuator modules integrated in the positioning and stabilising structure 6300 may be in electrical communication with processor module 6350 and battery module 6352 via a bus 6365, for example. The bus 6365 may be provided between two insulating layers 6366 that provide electrical insulation and that also prevent moisture ingress, for example from perspiration absorbed by inner fabric layer 6370. The insulating layers 6366 may be non-conductive polymer or elastomer films, for example, though it will be appreciated that other electrically insulating materials may also be used.

In some forms of the present technology, a thermally insulating layer may be provided between at least some of the electronic components of the positioning and stabilising structure 6300, noting that those components will tend to generate heat during use. Accordingly, a thermally insulating layer helps to improve patient comfort. For example, layer 6366 that is closest to the patient-contacting inner layer 6370 may be thermally insulating as well as electrically insulating, or an additional thermally insulating layer may be interposed between electrically insulating layer 6366 and inner layer 6370. In some examples, inner layer 6370 may itself be thermally insulating.

In some forms of the present technology, the sensor and/or actuator modules 6354-6360 and their associated circuitry, and other modules including the processor module 6350 and battery module 6352, may be received within retaining structures 6380-6390 that are affixed to insulating layer 6366 and/or inner fabric layer 6370. Each retaining structure 6380-6390 is in electrical communication with bus 6365, for example, and may contain electrical contacts to electrically connect circuitry of (or associated with) the sensor modules to bus 6365, and thus also to battery module 6352 and processor module 6350. In some examples, communication between modules 6350-6360 and bus 6365 may be via conductive ink traces, and/or conductive threads that are woven into or otherwise integrated with fabric layers 6370 and/or 6372. In some embodiments, electrical contacts and/or circuit traces may be contained only in outer layer 6372, so as not to be affected by perspiration from the patient during use.

In some examples, the modules 6350-6360 may be detachable from the sensor-retaining structures 6380-6390, such that particular modules may be switched out for other modules with different functionality, or to replace modules that have ceased to function or are at the end of their lifecycle. For example, the modules 6350-6360 (and/or the circuitry modules 6355, 6357, 6359, 6361, 6363 to which they are electrically coupled, if applicable) may releasably attach to the sensor-retaining structures 6380-6392. To this end, an external surface of a module may form a friction fit with an internal surface of a wall of a sensor-retaining structure 6380-6390, or may form a snap fit, such as an annular snap fit or cantilever snap fit, with the wall or other internal or external part of the sensor-retaining structure. In some embodiments, a non-mechanical coupling, such as magnetic coupling, may be used to retain the modules 6350-6360 in respective retaining structures 6380-6390.

In some forms of the present technology, retaining structures 6380-6390 may comprise pockets formed in the upper textile portion 6310 (for example, by making incisions in outer layer 6372 or inner layer 6370), into which modules 6350-6360 (or their associated circuitry) are insertable to electrically couple with the bus 6365.

Battery module 6352 may comprise a rechargeable battery. The battery may be recharged by connecting it to an external power source, for example via a micro-USB or USB-C port of the battery module 6352 (the port being exposed via outer fabric layer 6372, for example), or by inductive charging. In some embodiments, the battery module 6352 may be a disposable battery, for example disposed within a pocket 6382 of the upper textile portion 6310, and may be removable by the patient for replacement with a fresh battery.

In some forms of the present technology, one or more sensor modules and/or actuator modules may be enclosed entirely between the fabric layers 6370, 6372, such that no part of the one or more sensor modules is exposed. For example, an actuator module 6360 may be coupled to associated circuitry 6361 that is received in a sensor-retaining structure 6390. Both the actuator module 6360 and circuitry module 6361 lie entirely between the fabric layers 6370, 6372. In another example, a sensor module 6356 and associated circuitry 6357 may lie entirely between fabric layers 6370, 6372. One example of a sensor module 6356 that may be fully embedded is an accelerometer or gyroscope.

In some forms of the present technology, a sensor module or actuator module may be at least partly exposed. For example, a humidity sensor 6358 coupled to circuitry module 6359 may be at least partly exposed to ambient through the outer fabric layer 6372 to measure humidity of the patient's environment. To this end, outer fabric layer 6372 may comprise an aperture through which a surface of the humidity sensor 6358 may be exposed. In another example, a sensor 6354 coupled to circuitry 6355 may have a surface thereof exposed through the inner fabric layer 6370 (e.g., through an aperture formed therein), such that the sensor surface can contact the skin of the patient when the positioning and stabilising structure 6300 is worn by the patient. The sensor 6354 may be a pulse oximeter, for example.

Although the electronic components are described above as being modular in construction, and in at least some cases able to be switched out for other components, in some forms of the present technology, one or more electronic components (such as sensors or actuators) may be woven or otherwise integrated into the material of the upper textile portion 6310, for example into the outer fabric layer 6372 or the inner fabric layer 6370, and/or into another part of the positioning and stabilising structure 6300, such as lower textile portion 6320, and/or either or both of the back portions 6306, 6308. This may enable distribution of a sensor over a larger area for more informative and/or accurate measurements to be made.

In some forms of the present technology, a sensor may comprise a touch sensor 6362, such as a capacitive or resistive sensor or tactile switch, and may have associated circuitry that enables the sensor to function as a "pause" button. For example, the touch sensor 6362 may be incorporated into an exposed region of the positioning and stabilising structure 6300, or of the seal-forming structure 6100. In one example, a touch sensor 6362 may be positioned on the upper textile portion 6310 in the manner shown in Fig. 8. Touch sensor 6362 may communicate with processor/transceiver 6350 as previously described, such that signals recorded by touch sensor 6362 and circuitry module 6363 (when the touch sensor 6362 is in an "on" mode) may be transmitted by processor/transceiver 6350 to an external device, such as pressure generator 4140 of RPT device 4000.

For example, if the patient has patient interface 6000 in place and wishes to talk, or wakes up in the middle of the night and is feeling uncomfortable due to the positive pressure in plenum chamber 6200, he or she may activate the touch sensor 6362 to a "pause" mode by a light continuous touch. Circuitry module 6359 may detect this touch and transmit a pause signal to pressure generator 4140 (for example, via data communication interface 4280, Fig. 13C) to cause the flow to immediately be reduced to a very low value (for example, just enough to avoid a feeling of suffocation). When the "pause" mode is released, this is detected by circuitry module 6363 and a further signal sent to pressure generator 4140 to cause a ramp up algorithm implemented by RPT device 4000 to be reset.

Some forms of the present technology may comprise one or more sensors for determining sleeping position and movements of a patient prior to, and/or during, respiratory therapy. In some forms, the determined sleeping position and movements may be used to regulate the operation of pressure generator 4140, and/or to provide a sensory stimulus to the patient to cause them to change position. For example, if a number of apnea and/or hypopnea events above a certain threshold, and/or a decrease in blood oxygenation, is detected by the one or more sensors (whether or not pressure generator 4140 is operational at the time), this may be indicative of back sleeping. One or more actuators may receive an activation signal based on the detection, and the activation signal may cause the one or more actuators to generate a vibration or other tactile stimulus to irritate the patient sufficiently to cause them to switch to another sleeping position.

For example, positioning and stabilising structure 6300 or seal-forming structure 6100 may incorporate an accelerometer and/or gyroscope (not shown). The accelerometer and/or gyroscope may be fully enclosed between fabric layers 6370 and 6372 of the upper textile portion 6310, for example as shown at 6360 in Fig. 8. Both the accelerometer and gyroscope are in communication with processor/transceiver 6350 such that data recorded by them may be transmitted to RPT device 4000 to regulate the operation of pressure generator 4140.

Measurements recorded by the accelerometer may be used to determine the patient's sleeping position and to adjust therapy accordingly. When it is detected that the patient is lying on his or her back, the therapy pressure can be ramped up slowly by pressure generator 4140 to prevent sleep apnea events. When side sleeping is detected, the therapy pressure can be lowered. When an upright (e.g. reading before sleep, with mask on) position is detected, the flow and pressure could be just sufficient to avoid a feeling of suffocation.

Measurements recorded by the gyroscope may be used to determine movements of the patient and to adjust therapy accordingly. When a lot of movement is detected, indicating that the patient is likely awake, the therapy pressure can be kept low enough to avoid a feeling of suffocation. When the movements subside, the therapy pressure can be very slowly ramped up to avoid discomfort.

In some forms of the present technology, accelerometer and/or gyroscope measurements may be used to determine a sleep stage of the patient, and to turn pressure generator 4140 on or off accordingly. For example, it may be difficult for a patient to fall asleep if therapy commences while they are still awake. Accordingly, pressure generator 4140 may remain in an "off" or "paused" state if the accelerometer and/or gyroscope measurements are indicative of an awake or light sleep stage, and then switched on (typically, with a gentle ramp-up) once measurements indicate that the patient is in a deep sleep stage. Conversely, if therapy has already commenced and it is detected that the patient has switched from deep to light sleep, where therapy may rouse the patient, the pressure generator 4140 may be paused until the patient is in deep sleep again, for example.

In some forms of the present technology, a pulse oximeter incorporated in the positioning and stabilising structure 6300 may be used to assess sleep health. For example, a pulse oximeter 6354 and associated circuitry 6355 may be incorporated in the upper textile portion 6310 of positioning and stabilising structure 6300, as shown in Fig. 8. The pulse oximeter 6354 is exposed through an aperture of inner fabric layer 6370 such that it can contact the skin of the patient's forehead. Measurements recorded by pulse oximeter 6354 may be used to determine blood oxygen saturation level and heart rate during the period that the patient interface 6000 is worn, and this data may be transmitted to RPT device 4000, or an external computing device such as a smartphone, other mobile computing device, or laptop or desktop computing system of the patient. The time series data may be consolidated to provide feedback to the patient on their health levels, and recommendations for follow-up (for example, by a clinician).

In one example, an Apnea Hypopnea Index (AHI), which is a measure that clinicians use to classify the severity of sleep apnea, may be determined based on sensor measurements. Computation of AHI may use a combination of data from different sensors, e.g. blood oxygen level and heart rate (for example, measured by a PPG sensor), and chest movement (for example, measured by an accelerometer and/or gyroscope). The AHI value may be used to determine when an "apnea" occurs.

Accordingly, by tracking the AHI over time, a clinician will be able to tell if the patient has sleep apnea, and provide details of how severe it is. Further, by analysis of the AHI data together with other sensor data, the clinician may be able to not only correlate the frequency of apneas with particular sleeping positions (e.g. sleeping on back or sleeping on side), but also to adjust the CPAP therapy to the specific needs of the patient. For example, the amount of mouth breathing may be detected using temperature and/or humidity sensors located inside the plenum chamber of the patient interface, and a nasal or full-face mask prescribed accordingly. Additionally, pressure generator 4140 settings that will produce a flow rate most suitable for the patient may be recommended based on the sensor measurements. For example, a clinician may prescribe higher pressure settings (or equivalently, higher flow rates) for patients with a high detected rate of apnea or hypopnea events. The prescribed flow rate may also depend on the anatomical structure of the patient, for example if the patient has a more collapsible upper airway.

In some forms of the present technology, an EEG sensor may be provided in positioning and stabilising structure 6300, for example in upper textile portion 6310. The EEG sensor may be partially exposed in the manner similar to the pulse oximeter 6354 in Fig. 8 such that it can contact the skin of the patient's forehead. Typically, the EEG sensor comprises a plurality of EEG electrodes that generate signals that may be analysed to detect sleep stages. The signals may be transmitted (via processor/transceiver 6350) to an external device, such as the patient's smartphone, and the sleep stage, cycle and duration information may be used to provide feedback to the patient on how well the sleep therapy is progressing, as well as recommendations for enhanced health. For example, the EEG sensor measurements may be used for accurate sleep staging, to enable a more accurate determination of when an apnea or arousal from sleep occurs, e.g. during a sleep study.

In some forms of the present technology, the sleep stage information may be transmitted to RPT device 4000, such that it can be used by pressure generator 4140 to adjust the therapy pressures to avoid arousal or obstruction events.

In some forms of the present technology, the sleep stage information may be used to activate sleep-enhancing white/pink noise, and/or binaural beats. These may be produced by audio devices embedded in the patient interface 6000 itself, or by external devices that receive trigger signals from the patient interface 6000 via processor/transceiver 6350. For example, one or more miniature bone-conduction speakers may be incorporated at temple regions of the upper textile portion 6310.

In some forms of the present technology, a positioning and stabilising structure 6300 may incorporate electromyography (EMG) and/or electrooculography (EOG) sensors. EMG and EOG sensor signals may be analysed to determine REM sleep stage occurrences. In similar fashion to examples that incorporate EEG sensors, the sleep stage information determined by the EMG/EOG sensors may be used to provide feedback to the patient on how well sleep therapy is progressing, and may also be used to regulate pressure generator 4140 to avoid arousal or obstruction events, or to activate one or more audio devices to produce sleep-enhancing noise.

At least some of the EMG/EOG sensors may be incorporated in upper textile portion 6310. For example, a ground electrode and reference electrode may be provided in the upper textile portion 6310, for example in a front section thereof, and exposed through respective apertures in inner layer 6370 so as to be able to contact the patient's forehead. In another example, a ground electrode may be provided in a rear portion 6306 of upper textile portion 6310, or in second lower textile portion 6308 such that the ground electrode sits behind the patient's ear in use. Further electrodes may be provided, each having a cable that attaches to and/or extends within upper textile portion 6310 or first 6320 or second 6308 lower textile portions at one end, and to an electrode patch at the other end, the electrode patch being positionable by the patient on the temple and below their eye to provide two additional measurement channels.

In some forms of the present technology, an acoustic sensor, for example a microphone, such as a MEMS microphone or Electret microphone, or a piezo film based transducer, may be incorporated in a patient interface 6000 to detect snoring. For example, the microphone may be located in or on an internal surface of plenum chamber 6200, or on an external surface thereof, adjacent the patient's nares. The microphone may be coupled to a communications interface to enable communication of data to the pressure generator 4140 of RPT device 4000, to regulate the pressure produced thereby. For example, when a light snoring noise pattern is detected, the therapy pressure can be gradually increased to prevent an obstructive event. When the snoring noise pattern subsides, the therapy pressure can be ramped down.

In some forms of the present technology, a patient interface 6000 may incorporate a humidity sensor and temperature sensor, for example on an internal surface of plenum chamber 6200, to monitor the temperature and humidity inside the plenum chamber 6200. The sensors may be coupled to a communications interface for transmitting humidity and temperature data to RPT device 4000 and humidifier 5000 to regulate the operation thereof. The pressure generator 4140 and humidifier 5000 power may be regulated to prevent condensate accumulation. For example, the humidifier 5000 may be activated in stages, and/or heater power levels may be controlled, followed by flushing with plain air, still keeping moisture levels (as measured by the humidity sensor) sufficient to prevent dry mouth.

In some forms of the present technology, a pressure sensor may be provided inside plenum chamber 6200, for example on an inner surface thereof. This enables the air pressure inside plenum chamber 6200 to be monitored and a signal sent to the pressure generator 4140 to adjust pressure and flow dynamically. This may optimise the pressure generator 4140 response to the patient's breathing pattern.

In some forms of the present technology, a $CO_2$ sensor may be provided inside plenum chamber 6200. For example, the $CO_2$ level inside the plenum chamber 6200 may be monitored. When a slight increase in $CO_2$ level is detected, an electromechanical vent (not shown) may be opened to allow higher flushing of air from the plenum chamber 6200. Further, a signal may be sent to the pressure generator 4140 to slightly increase the flow to flush out $CO_2$ when the $CO_2$ level increases slightly. This may be done dynamically so as to minimise patient discomfort.

In some forms of the present technology, a combination of sensors and actuators may be provided to effect localised temperature change to improve patient comfort. For example, an EEG sensor and/or pulse oximeter may be provided in upper textile portion 6310 (for example in the manner shown at 6354 in Fig. 8), and a temperature sensor and/or a humidity sensor may also be provided in upper textile portion 6310 (for example in the manner shown at 6358 in Fig. 9C). Signals from the EEG and/or PPG sensors may be analysed to detect sleep state, and signals from the temperature sensor and/or humidity sensor may be used to assess ambient comfort levels. One or more Peltier elements may be provided, for example in wearable form on a wristband, and may be coupled to circuitry that communicates with the EEG/PPG and temperature/humidity sensors to receive signals indicative of sleep state and ambient comfort level, and that causes the Peltier element to be activated to locally warm or cool the body (e.g. at the wrist) to help the patient remain in a comfortable sleep state.

In some forms of the present technology, a haptic feedback element (such as a miniature vibratory motor) may be incorporated in the patient interface 6000, for example in a temple region of the positioning and stabilising structure 6300 (e.g. of upper textile portion 6310). The haptic feedback element may deliver vibrations to the patient to produce a calming effect. For example, processor module 6350 may monitor heart rate data from a pulse oximeter 6354, and if this exceeds a threshold, transmit a trigger signal to the haptic feedback element to cause it to vibrate at a few beats lower than the patient's current heart rate, to help slow it down. In another example, as mentioned above, a haptic feedback element may be used to influence the sleeping position of the patient if it is detected that they are in a sleeping position that is correlated with apnea or hypopnea events.

In some forms of the present technology, one or more miniature thermo electric generators (TEGs) may be incorporated into the patient interface 6000, such that the difference between the patient's body temperature and the ambient temperature may be used to generate a potential difference and thus to provide power to the various electronic components (sensors, actuators, processor, etc.) of the patient interface 6000. For example, miniature TEGs may be located in the upper textile portion 6310, and exposed through an aperture of the inner layer 6370 such that they contact the patient's forehead.

In some forms of the present technology, multiple sensors may be incorporated in a single module. For example, an accelerometer and gyroscope may be incorporated in a single package.

While the various sensors and actuators have been described as being incorporated in the patient interface 6000 shown in Figs. 7A, 7B and 8, it will be appreciated that they may be incorporated in like fashion in any of the other patient interfaces 3000 disclosed herein.

### 5.3.4 Power systems for patient interfaces and headgear

As mentioned above, a patient interface 3000 or 6000, and/or a positioning and stabilising structure for a patient interface (also referred to herein as headgear), may comprise one or more electronic components such as sensors, actuators, processors and the like. Such components may be incorporated into the patient interface 3000, 6000, for example as one or more actuator modules 6354-6360 as depicted in Fig. 8.

As discussed, the one or more electronic components may be powered by an electrical energy storage device such as a rechargeable battery or supercapacitor. In some forms of the present technology, a patient interface may comprise and/or may be communicable with a power system that can directly power the one or more electrical components, and/or charge a battery or supercapacitor for doing so.

Figure 9A is a schematic block diagram of a patient interface 7000 that comprises one or more electronic components that are electrically powered by a power system. The patient interface 7000 comprises a battery 7020 (e.g., a rechargeable battery), a communications module 7030, and two sensor modules 7040 and 7050. Each sensor module 7040, 7050 may comprise multiple sensor components. For example, sensor module 7040 may comprise temperature, humidity, ambient light, and/or acoustic sensors incorporated therein. Sensor module 7050 may comprise an accelerometer, a gyroscope, a pulse oximeter, and/or a blood pressure sensor, for example. Any of these sensor components may alternatively be included on the other sensor, or may be separately included on an additional sensor (not shown). Additionally, other sensor components not explicitly mentioned above may be included in at least one sensor 7040, 7050, and/or in a separate sensor. The communications module 7030 may comprise one or more of a Bluetooth interface, a near field communication (NFC) interface, and a WiFi interface. The patient interface 7000 may comprise a positioning and stabilising structure in accordance with those of patient interface 3000 or patient interface 6000, and the sensors of modules 7040, 7050 may comprise any of the sensors described above with reference to patient interface 6000.

The battery 7020 supplies power to one or more of the modules 7030, 7040, 7050 via a power line 7072 and a ground line 7070. Modules 7030, 7040, 7050 are synchronised with each other via a clock signal 7076, and communicate data with each other via data line 7074.

In some examples, as shown in Figures 9A to 9D, the power system may comprise a charger circuit 7010 that is located external of the patient interface 7000, but is communicable with the rechargeable battery 7020 of the patient interface 7000, either in wired or wireless fashion. The charger circuit 7010 may form part of, or be in communication with, an RPT device 4000, for example.

In one example, as depicted schematically in Figure 9B, the charger circuit 7010 is connected to the rechargeable battery 7020 via a wired connection. For example, power cables may be wound (helically or otherwise) around an exterior of the air circuit 4170 such that ends thereof may be connected to terminals of the battery 7020. Data cables may also be wound around the exterior of the air circuit 4170 (for example, within an insulating extrusion) for connection to battery 7020 and/or any of the other modules 7030, 7040, 7050. Alternatively, data may be communicated between the battery 7020 and the charger circuit 7010 by other means, such as Bluetooth or NFC, and intermediated by communication module 7030, for example.

In some forms of the present technology, power and/or data cables may pass through a conduit located at an exterior surface of the air circuit 4170, or even within the air circuit 4170.

The data connection between charging circuit 7010 and battery 7020 may be used to control charging of the battery 7020 in various ways. For example, a charging algorithm may be implemented in the battery 7020 such that when a certain low battery threshold is reached, the battery 7020 sends an alert to the charging circuit 7010 to initiate charging. The battery 7020 may also communicate charge information (and other information) to other nearby devices by wireless communication, for example.

The connection of power and/or data cables extending from the charging circuit 7010 to the patient interface 7000 may be implemented in various ways. For example, the cables may terminate at an end of the air circuit 4170 that connects to electrical contacts of an air inlet connection port (such as the port 3600 of patient interface 3000 or port 6600 of patient interface 6000) via a slip ring connection, and terminals at the port may in turn be in electrical communication with the battery 7020, or other electrical components of the patient interface 7000. This is so that, as the end of air circuit 4170 rotates within the port, an electrical connection is maintained.

In some forms of the present technology, as shown in Figure 9C, the charger circuit 7010 may be configured to charge battery 7020 via a wireless power transfer method. For example, near-field wireless power transfer may be effected by an inductive charging circuit of the charger circuit 7010 that cooperates with charging coils located in the headgear or another part of patient interface 7000. The inductive charging circuit may be located in RPT device 4000, for example, such that the patient may rest the headgear on RPT device 4000 when not in use, so as to charge a rechargeable battery of the headgear. In some forms, the patient interface 7000 may comprise a magnetic alignment feature for automatically aligning the battery 7020 with the charging circuit 7010. For example, the magnetic alignment feature may comprise one or more magnets that are attracted by a magnetic field produced by RPT device 4000, for example by one or more magnets in the vicinity of the location of charging circuit 7010. Alternatively, charging circuit 7010 may use a far-field or radiative wireless power transfer method, such as WiFi-based power transfer, to charge the battery 7020. Data may be communicated from the battery 7020 to the charging circuit 7010 by Bluetooth, NFC, etc.

In some forms of the present technology, as shown in Figure 9D, the charger circuit 7010 may connect to the battery 7020 by any form of standard connection means, such as a USB-C or mini-USB cable 7080, for example.

Accordingly, although charger circuit 7010 may be used to charge the battery 7020, other standard charging devices that are independent of the RPT device 4000 (such as smartphone chargers or the like) may be used to charge the battery 7020. In these forms, the patient interface 7000 may be made essentially independent of the RPT device 4000 for the purposes of powering electronic components of the patient interface 7000. Thus, if the patient interface (or at least the headgear thereof) is to be used for only monitoring, rather than therapeutic, purposes, portability of the patient interface 7000 is improved. As for the forms shown in Figures 9B and 9C, data may be transferred to charger circuit 7010 via Bluetooth, NFC, etc. as desired.

In some forms of the present technology, the power system may comprise an energy harvesting system. The energy harvesting system is arranged to convert non-electrical energy, such as mechanical or thermal energy, that is generated during use of the patient interface, to electrical energy for powering the one or more electrical components. Advantageously, the provision of an energy harvesting system provides an alternative or additional energy source for powering the one or more electrical components, and/or for providing auxiliary power in the event that the electrical energy storage device is at low capacity. Further, the energy harvesting system may reduce the need to recharge a battery or supercapacitor that powers the one or more electronic components.

For example, as shown in Figure 10, a patient interface 7000' that is of the same general configuration as patient interface 7000 of Figure 9A is shown. However, in Figure 10, the patient interface 7000' is not connected to any external power source, but instead incorporates an energy harvesting system 7060. The energy harvesting system 7060 is electrically coupled to battery 7020 and to the electronic modules 7030, 7040, 7050. The energy harvesting system 7060 may be capable of directly powering the modules 7030, 7040, 7050 and/or charging the battery 7020 such that the battery 7020 can provide power to said modules.

Of course, it will be appreciated that in some forms of the present technology, a patient interface may both comprise an energy harvesting system 7060, and be configured to communicate with an external charging circuit 7010 to charge battery 7020.

In one form of the present technology, a turbine generator may be provided in an air flow path of the patient interface. The turbine generator is constructed and arranged to convert mechanical energy from air flow during use of the patient interface to electrical current. For example, the turbine generator may be positioned at an air inlet of the patient interface 3000, 6000 (e.g., and may rotate as a result of the flow of pressurized air toward the patient). In some forms of the present technology, the turbine generator is positioned within a connection port 3600 or 6600 that connects the patient interface 3000, 6000 to air circuit 4170. By doing so, the turbine generator receives the highest possible air flow, and thus can generate the highest possible current. Alternatively, the turbine generator may be positioned within decoupling structure 3500. In some forms of the present technology, the turbine generator may be positioned in line with a vent of the patient interface 3000, 6000, or may be part of a vent of the patient interface 3000, 6000. In some forms, the vent and turbine generator may be positioned upstream from the patient such that pressurized air flows through both the vent and the turbine generator. In other forms, the vent and turbine generator may be positioned downstream from the patient (e.g., a vent that exhausts exhaled gas to the ambient).

In one form, the turbine generator may be positioned at the air inlet of the patient interface 3000, 6000 or in the connection port 3600, 6600 substantially perpendicularly to a flow direction of the pressurized air. For example, the turbine generator may rotate about an axis that is substantially parallel to the flow direction of the pressurized air.

In another form, multiple turbine generators may be positioned the air inlet of the patient interface 3000, 6000 and/or in the connection port 3600, 6600. The turbine generators may be spaced apart from one another along the flow path of the pressurized air.

One form of turbine generator 8000 is shown in Figs. 11A-11D. The turbine generator 8000 comprises a rotor assembly 8100 that is constructed and arranged to rotate about an axle 8200 within a stator assembly that comprises an inner portion 8300 that is housed in a casing 8400 and sealed by a cover 8500. The cover 8500 that is assembled together with the casing 8400 to seal coil windings 8310 to protect them from ingress of moisture and particulate matter.

The ends of axle 8200 are received in respective apertures 8510 of the cover 8500 and 8330 of the inner portion 8300 such that the rotor 8100 can rotate within the stator assembly. The axle 8200 may form a friction fit with the apertures 8510 and 8330 and a clearance fit with an aperture 8120 that extends through the rotor assembly 8100. Alternatively, the axle 8200 may form a friction fit with the rotor aperture 8120, and a clearance fit with the apertures 8330, 8510, to provide the ability of the rotor assembly 8100 to rotate.

In some forms, the axle may be configured to be supported only on one end, like a cantilever. This arrangement helps restrict the supporting rib elements (e.g., ribs 8520) to one side of the energy harvester to reduce noise.

In some forms, the inner portion 8300 comprises a plurality of bobbins 8308 extending around its periphery (see e.g., Fig. 11A). Each bobbin 8308 has a cylindrical section about which a wire can be wound to form a coil of a winding of the stator assembly. Although in other examples, the shape of at least one bobbin 8308 may be different (e.g., have an angled shaped, or an elliptical perimeter).

In some forms of the present technology, a single-wire winding scheme is adopted to maximise the voltage that is generated. A single wire is first wound in a plurality of turns around one bobbin 8308 and then in a plurality of turns around the next bobbin 8308, continuing from one bobbin 8308 to the next with the turns of successive coils 8310 being in opposite directions to each other. As shown in Fig. 11C, once all coils have been wound, the wire may have a first end 8312 and a second end 8314 that can be threaded through the stator casing 8400 for connection to the one or more electronic components, for example via additional circuitry such as rectifier circuitry and/or voltage stabilising circuitry. In some forms of the present technology, the rectifier circuitry and/or voltage stabilising circuitry may be located within the housing of turbine generator 8000, for example in the casing 8400.

In other forms, instead of a single wire wound over all the bobbins 8308, two or more wires may be wound around the bobbins 8308 in a series and/or parallel configuration for the desired voltage and current outputs.

The circuitry may be connected to terminals that extend from, or are located on an external surface of, the turbine generator 8000 to contact terminals located within patient interface 3000 or 6000 and which enable electrical connection to the one or more electronic components. For example, the terminals located within the patient interface may be connected to wires or other conductors that extend within and/or along conduit 3301 of patient interface 3000 or upper textile portion 6310 or lower textile portion 6320 of patient interface 6000.

In some forms of the present technology, fine gauge wire may be used to form the coils of the stator, to accommodate as many turns as possible such that as high a voltage as possible can be generated. For example, 0.1mm wire may be used. In another example, 0.22mm wire may be used. In this way it is possible to generate a voltage above 5 volts, rendering a voltage booster unnecessary for operation of the one or more electronic components of the patient interface 3000, 6000.

The rotor assembly 8100 comprises a generally cylindrical rotor body that has the rotor aperture 8120 extending therethrough. In some forms, the rotor aperture 8120 may extend through a center of the cylindrical rotor body, although in other examples, the rotor aperture 8120 may be off-center. The rotor body comprises a plurality of turbine blades 8130. It will be appreciated that the shape (angle and profile) and number of blades 8130 may be optimised to ensure low noise operation during respiratory therapy, when pressurised air causes rotor 8100 to spin (e.g., as a result of pressurized air entering the air inlet of the patient interface 3000, 6000 via air circuit 4170 and/or exhaled air exiting the patient's nares or mouth). For example, the angle of attack of the blades 8130 may be configured such that the torque generated by the rotor assembly 8100 is sufficient to overcome the force associated with the induced magnetic field from the coils of the stator, and to generate a desired voltage.

The rotor body also comprises a plurality of peripheral recesses 8110 within which respective magnets can be carried. The recesses 8110 may be sealed once the magnets have been inserted, to guard against ingress of dirt and moisture.

In some forms of the present technology, the magnets of neighbouring recesses 8110 have opposite poles facing the winding. The number of coil bobbins 8308 may be the same as the number of magnets, and their angular pitches may also be the same. The coil axes and magnet axes may each be arranged radially. In some examples, the magnet axes may be arranged radially, and the coil axes arranged tangentially.

As illustrated in Fig. 11E, an alternate version of the rotor body 9000 may have magnets 9004 that are positioned towards the base of the blades 9008 (instead of in the recesses 8110 proximate to the periphery of the rotor illustrated in Figs. 11A and 11B). In some forms, the magnets 9004 of the rotor body 9000 may be similarly positioned in a recess (e.g., so that they are removable) and/or they may be fixed to the housing 9002 or other portion of the rotor body 9000.

In certain forms, the magnets 9004 may be covered, similar to the magnets in the recesses described above, in order to limit ingress of dirt and moisture.

In certain forms, the magnets 9004 are arranged in a similar configuration as the magnets described as being inserted into the recesses 8110. For example, the magnets 9004 of neighbouring blades 9008 have opposite poles facing the stator coils 9012.

In some forms, the stator coils 9012 of the rotor body 9000 may be embedded in the rotor body 9000. The fixed coils 9012 may act as the fixed axle around which the rotor 9000 rotates. The coils 9012 may be formed from one or more wires as described above.

In some forms, positioning the magnets 9004 closer to the base of the turbine blades 9008, as illustrated in Fig. 11E, may produce a lower rotor inertia (e.g., the location of the center of mass is located closer to a center of the rotor body 9000) and therefore, can rotate better even at a relatively low air flow (e.g., less torque is necessary to produce rotation in the turbine blades 9008).

To assemble the turbine generator 8000, the inner portion 8300 may be placed inside the casing 8400, and the rotor assembly 8100 then placed within the inner portion 8300. Axle 8200 may then be inserted into through-aperture 8120 of the rotor assembly 8100 and into aperture 8330 of the inner portion. At this stage, coils 8310 of the stator assembly remain exposed. Accordingly, the cover 8500 is then attached to the casing 8400, such that axle 8200 is located in the aperture 8510 of the cover, and the rim 8530 of the cover seals the space within which the coils 8310 reside. The cover 8500 may be affixed to the casing 8400 by any suitable means, such as by a snap fit or screw thread. In some forms of the present technology, a gasket (not shown) may be provided at the rim 8530 to further reduce the possibility of moisture ingress.

Because the rotor assembly 8100 and stator assembly (comprising the combination of casing 8400, inner portion 8300 and cover 8500) are both sealed, the ingress of moisture and dirt is minimised. Accordingly, it is possible for the turbine generator to remain in place in the patient interface 3000, 6000 during the usual washing cycle for the patient interface. This is more convenient for the patient, as disassembly is not required.

As mentioned above, the turbine generator 8000 operates by converting mechanical energy from air flow into rotational energy, which is in turn converted to electrical energy due to the current induced in coils 8310 by the rotating magnets of the rotor 8100. To this end, it is desirable to minimise flow impedance. In some forms of the present technology, this is achieved by providing a supporting structure for the axle 8200 of minimal surface area. For example, as shown in Fig. 11A-11D, the axle 8200 may be supported at the cover 8500 side by a trio of ribs 8520 that extend from a central ring 8515 to the rim 8530 of the cover 8500.

In some forms of the present technology, two ribs 8520 may extend from the central ring 8515 to the rim 8530. In other forms, more than three ribs 8520 may be provided. In general, if the number of blades 8130 is even, it may be advantageous to provide an odd number of ribs 8520 (and vice versa), since this provides an asymmetry that will tend to reduce the amount of noise that is generated as the blades 8130 pass over the ribs 8520.

The ribs 8520 are made as thin as possible so as to minimise the flow impedance while still providing the necessary support for the axle. As shown, the ribs 8520 do not extend radially from the ring 8515, but instead are slightly offset. This is to reduce the possibility of turbulence being generated as air flows through the turbine generator 8000, and thus the amount of noise generation. The offset arrangement of ribs 8520 tends to guide the air flow, thus smoothening it e.g. by changing the direction of the airflow or by reducing the velocity of the air flow so as to reduce the amount of turbulence thereby inducing more laminar flow. Further smoothening of the air flow may be achieved by providing curved or angled surfaces for the ribs 8520, for example.

Similar to the cover 8500, the inner portion 8300 of the stator assembly may have a supporting structure of minimal surface area, for example a trio of ribs 8320 having substantially identical configuration to the ribs 8520 of the cover 8500.

The turbine generator 8000 may be connected to circuitry for modulating and/or converting its output voltage prior to the connection to the one or more electronic components of the patient interface 3000, 6000. For example, a full-bridge rectifier with capacitor may be used to convert the output AC voltage to a DC power supply.

Test results with an 8-blade, 8-pole example indicated that a turbine generator may generate up to 8V when placed in an air circuit 4170 connected to an RPT device 4000 at 20 cm H2O pressure setting.

In some forms of the present technology, the energy harvesting system can comprise at least one piezoelectric film. A piezoelectric film can be used to generate current from air flow changes (e.g., due to input of pressurised air from RPT device 4000, for example, and/or from exhalation by the patient) and body movements of the patient during use. Deflection of the piezoelectric film generates a voltage that depends on the deflection amplitude and speed. Advantageously, a piezoelectric film can also itself act as a sensor, with the voltage output being indicative of vibration and/or noise (e.g. due to snoring).

A piezoelectric film may be placed in or on various components of the patient interface 3000, 6000. For example, a piezo film may be positioned within plenum chamber 3200, 6200, such as adjacent an air inlet to the plenum chamber such that it is in the path of the air flow created by RPT device 4000 during use. A piezo film thus positioned may be used to both harvest energy and to detect vibration. In another example, a piezo film may be attached to or integrated with an interior surface of the seal-forming structure 3100, for energy harvesting and movement sensing. In a further example, a piezo film may be attached to or sandwiched between layers of a tie or strap of a positioning and stabilising structure 3300, 6300, or another component that contacts the user's face, such that the movement of facial muscles of the user during breathing or other movements causes the piezo film to flex and thus generate electrical energy. In a further example, the piezo film may be positioned within the plenum chamber 3200, 6200, such as adjacent to an outlet (e.g., a vent that exhausts exhaled air to ambient) to the plenum chamber such that it is in the path of exhaled air (e.g., carbon dioxide) leaving the plenum chamber.

In some forms, the patient interface 3000, 6000 may include both the turbine generator 8000 and the piezo film (e.g., as opposed to one or the other). This may allow for a greater amount of energy harvesting and/or may allow energy harvesting at multiple locations in the flow path.

In some forms of the present technology, an energy harvesting system may comprise one or more thermoelectric generators (TEGs). The thermoelectric generator may be used to convert heat energy into electric energy, which may be used to power various electrical components of the patient interface 3000, 6000.

In some forms, the thermoelectric generator may be located on a portion of the patient interface 3000, 6000 that is adapted to contact the patient in use. For example, the thermoelectric generator may be located on a portion of the positioning and stabilising structure 3300, 6300. The thermoelectric generator may use heat emitted from the patient's skin and convert it into electrical energy. The thermoelectric generator may alternatively or additionally be located on the seal-forming structure 3100, 6100, and similarly capture heat emitted from the patient's skin.

Alternatively or in addition, thermoelectric generator may be located in the plenum chamber 3200, 6200 of the respective patient interface 3000, 6000. The thermoelectric generator may convert the heat from exhaled air into electrical energy that may be used to power various electrical components.

Fig. 12A and 12B show examples of measurements recorded by two piezo films forming part of an energy harvesting system of a patient interface. A first piezo film was positioned within plenum chamber 3200 in the air path, near the inlet, and a second piezo film was positioned along an inner surface of the seal-forming structure 3100.

In Fig. 12A, the patient's breathing pattern includes a sequence of normal breathing followed by a breathing stoppage and then a sudden inhalation. The breathing stoppage is detectable as a deviation from the normal breathing pattern, as the mean amplitude for the air path piezo film during the stoppage is zero. The sudden inhalation is detectable as a spike in the signal detected by the cushion (seal-forming structure) piezo film.

In some forms, this may allow the piezo film based harvester may function as a snoring and/or breath-stoppage detector (i.e. detect the stoppage of breathing).

In Fig. 12B, a sequence of normal breathing followed by simulated snoring is shown. During the snoring phase, the signal detected by the air path piezo film does not change, but spikes appear in the signal detected by the cushion piezo film.

### 5.3.5 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled $CO_2$ by the patient while maintaining the therapeutic pressure in the plenum chamber in use.

One form of vent 3400 in accordance with the present technology comprises a plurality of holes, for example. about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

As mentioned above, one or more of the energy harvesters mentioned above (e.g., the turbine generator 8000, the piezo film, and/or the thermoelectric generator) may be positioned proximate to the vent 3400 in order to convert washout gas into electrical energy.

In some forms, the energy harvester(s) may be positioned within the plenum chamber 3200, 6200 between the patient and the vent 3400. In other words, the patient may be upstream from the energy harvester and the vent 3400 may be downstream from the energy harvester. As described above, exhaled $CO_2$ may exhaust through the vent 3400 to prevent rebreathing. As the air flows from the patient's mouth toward the vent 3400, the air passes the energy harvester and allows for some recapture of energy

In some forms, a vent of the patient interface 3000, 6000 may be a bleed-off vent, which may exhaust pressurized air to the ambient prior to reaching the patient (i.e., this may be a different vent than the $CO_2$ exhaust vent described above). For example, the full face mask 3000 illustrated in Fig. 1C and the nasal mask illustrated in Fig. 1B and 3A may be connectable to the same RPT device 4000. In some forms, the RPT device 4000 may include a sensor or user input to determine which version of the mask is connected. In other version, the RPT device 4000 may provide a single output for a given pressure. In other words, in order to achieve a given pressure in the respective plenum chamber, the RPT device may output a determined pressure, regardless of which patient interface is connected.

In some forms, the volume of the plenum chamber 3200 of the full face mask 3000 may be larger than the volume of the plenum chamber 3200 of the nasal mask 3000. Therefore, the plenum chamber 3200 of the nasal mask may be over pressured (e.g., experience a higher pressure than requested). To address this problem, a vent 3400 may be located in the swivel so that air exhausts to the ambient prior to entering the plenum chamber 3200. This may allow the pressure in the plenum chamber 3200 to drop to the desired pressure.

Using a bleed-off vent in this manner may be inefficient because electrical energy to create the stronger flow may be wasted (i.e., the pressurized air is exhausted to the ambient before the patient can inhale it). The turbine generator 8000 (described above) may be positioned proximate to the swivel, and may complement and/or replace the bleed-off vent.

For example, airflow through the turbine generator 8000 may reduce the speed of the pressurized airflow, thereby reducing the pressure of the airflow. This may reduce the airflow pressure to the desired pressure and/or may require less airflow to exhaust through the vent.

In some forms, an energy harvester may be used with the vent 3400 and the bleed off vent in order to increase efficiency of the system (e.g., recapture more energy that would otherwise exit the system). However, the patient interface 3000, 6000 may only include one or none of these energy harvesters.

### 5.3.6 Decoupling structure(s)

In one form the patient interface 3000 includes at least one decoupling structure, for example, a swivel or a ball and socket.

### 5.3.7 Connection port

Connection port 3600 allows for connection to the air circuit 4170.

### 5.3.8 Forehead support

In one form, the patient interface 3000 includes a forehead support 3700.

### 5.3.9 Anti-asphyxia valve

In one form, the patient interface 3000 includes an anti-asphyxia valve.

### 5.3.10 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

## 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 $cmH_2O$, or at least 10$cmH_2O$, or at least 20 $cmH_2O$.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

#### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 5.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 $cmH_2O$ to about 20 $cmH_2O$, or in other forms up to about 30 $cmH_2O$ when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

A pressure generator may also be referred to in at least some contexts as a flow generator.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

#### *5.4.1.4.1 Flow rate sensor*

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### *5.4.1.4.2 Pressure sensor*

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 and representing a pressure is received by the central controller 4230.

#### *5.4.1.4.3 Motor speed transducer*

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

#### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

#### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000. In another form of the present technology, power supply 4210 provides electrical power to the one or more electronic components of patient interface 3000, 6000.

#### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

#### 5.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 which may be implemented with processor-control instructions, expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

#### 5.4.2.4 Clock

The RPT device 4000 may include a clock 4232 that is connected to the central controller 4230.

#### 5.4.2.5 Therapy device controller

In one form of the present technology, therapy device controller 4240 is a therapy control module 4330 that forms part of the algorithms 4300 executed by the central controller 4230.
In one form of the present technology, therapy device controller 4240 is a dedicated motor control integrated circuit. For example, in one form a MC33035 brushless DC motor controller, manufactured by ONSEMI is used.

#### 5.4.2.6 Protection circuits

The one or more protection circuits 4250 in accordance with the present technology may comprise an electrical protection circuit, a temperature and/or pressure safety circuit.

#### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.
Memory 4260 may be located on the PCBA 4202. Memory 4260 may be in the form of EEPROM, or NAND flash.
Additionally, or alternatively, RPT device 4000 includes a removable form of memory 4260, for example a memory card made in accordance with the Secure Digital (SD) standard.
In one form of the present technology, the memory 4260 acts as a non-transitory computer readable storage medium on which is stored computer program instructions expressing the one or more methodologies described herein, such as the one or more algorithms 4300.

#### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.
In one form, data communication interface 4280 is part of the central controller 4230. In another form, data communication interface 4280 is separate from the central controller 4230, and may comprise an integrated circuit or a processor.
In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.
In one form, local external communication network 4284 utilises one or more communication standards, such as Bluetooth, or a consumer infrared protocol.
In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.
The local external device 4288 may be a personal computer, mobile phone, tablet or remote control.

#### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

##### *5.4.2.9.1 Display driver*

A display driver 4292 receives as an input the characters, symbols, or images intended for display on the display 4294, and converts them to commands that cause the display 4294 to display those characters, symbols, or images.

##### *5.4.2.9.2 Display*

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

#### 5.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure *Pm,* the vent flow rate *Qv,* the respiratory flow rate *Qr,* and the leak flow rate *Ql.*

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

##### *5.4.3.1.1 Interface pressure estimation*

In one form of the present technology, an interface pressure estimation algorithm 4312 receives as inputs a signal from the pressure sensor 4272 indicative of the pressure in the pneumatic path proximal to an outlet of the pneumatic block (the device pressure *Pd*) and a signal from the flow rate sensor 4274 representative of the flow rate of the airflow leaving the RPT device 4000 (the device flow rate *Qd*). The device flow rate *Qd,* absent any supplementary gas 4180, may be used as the total flow rate *Qt.* The interface pressure algorithm 4312 estimates the pressure drop $\Delta P$ through the air circuit 4170. The dependence of the pressure drop $\Delta P$ on the total flow rate *Qt* may be modelled for the particular air circuit 4170 by a pressure drop characteristic $\Delta P(Q)$. The interface pressure estimation algorithm, 4312 then provides as an output an estimated pressure, *Pm,* in the patient interface 3000 or 3800. The pressure, *Pm,* in the patient interface 3000 or 3800 may be estimated as the device pressure *Pd* minus the air circuit pressure drop $\Delta P$.

##### *5.4.3.1.2 Vent flow rate estimation*

In one form of the present technology, a vent flow rate estimation algorithm 4314 receives as an input an estimated pressure, *Pm,* in the patient interface 3000 or 3800 from the interface pressure estimation algorithm 4312 and estimates a vent flow rate of air, *Qv,* from a vent 3400 in a patient interface 3000 or 3800. The dependence of the vent flow rate *Qv* on the interface pressure *Pm* for the particular vent 3400 in use may be modelled by a vent characteristic *Qv*(*Pm*).

##### *5.4.3.1.3 Leak flow rate estimation*

In one form of the present technology, a leak flow rate estimation algorithm 4316 receives as an input a total flow rate, *Qt,* and a vent flow rate *Qv,* and provides as an output an estimate of the leak flow rate *Ql.* In one form, the leak flow rate estimation algorithm estimates the leak flow rate *Ql* by calculating an average of the difference between total flow rate *Qt* and vent flow rate *Qv* over a period sufficiently long to include several breathing cycles, e.g. about 10 seconds.

In one form, the leak flow rate estimation algorithm 4316 receives as an input a total flow rate *Qt,* a vent flow rate *Qv,* and an estimated pressure, *Pm,* in the patient interface 3000 or 3800, and provides as an output a leak flow rate *Ql,* by calculating a leak conductance, and determining a leak flow rate *Ql* to be a function of leak conductance and pressure, *Pm.* Leak conductance is calculated as the quotient of low pass filtered non-vent flow rate equal to the difference between total flow rate *Qt* and vent flow rate *Qv,* and low pass filtered square root of pressure *Pm,* where the low pass filter time constant has a value sufficiently long to include several breathing cycles, e.g. about 10 seconds. The leak flow rate *Ql* may be estimated as the product of leak conductance and a function of pressure, *Pm.*

#### *5.4.3.1.4 Respiratory flow rate estimation*

In one form of the present technology, a respiratory flow rate estimation algorithm 4318 receives as an input a total flow rate, *Qt,* a vent flow rate, *Qv,* and a leak flow rate, *Ql,* and estimates a respiratory flow rate of *air, Qr,* to the patient, by subtracting the vent flow rate *Qv* and the leak flow rate *Ql* from the total flow rate *Qt.*

### 5.4.3.2 Therapy Engine Module

In one form of the present technology, a therapy engine module 4320 receives as inputs one or more of a pressure, *Pm,* in a patient interface 3000 or 3800, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

#### *5.4.3.2.1 Phase determination*

In one form of the present technology, the RPT device 4000 does not determine phase.

In one form of the present technology, a phase determination algorithm 4321 receives as an input a signal indicative of respiratory flow rate, *Qr,* and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate *Qr* has a value that exceeds a positive threshold, and a discrete value of 0.5 revolutions (thereby "cycling" the RPT device 4000) when a respiratory flow rate *Qr* has a value that is more negative than a negative threshold. The inhalation time *Ti* and the exhalation time *Te* may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate *Qr.* A continuous value of phase determined in this implementation is often referred to as "fuzzy phase". In one implementation of a fuzzy phase determination algorithm 4321, the following rules are applied to the respiratory flow rate *Qr*:

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate *Qr* as described above, as are the inhalation time *Ti* and the exhalation time *Te.* The continuous phase $\Phi$ at any instant may be determined as the half the proportion of the inhalation time *Ti* that has elapsed since the previous trigger instant, or 0.5 revolutions plus half the proportion of the exhalation time *Te* that has elapsed since the previous cycle instant (whichever instant was more recent).

### *5.4.3.2.2 Waveform determination*

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure *Pt* that varies as a function of phase $\Phi$ of a respiratory cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi(\Phi)$ in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation ($\Phi$ = 0 revolutions) or exhalation ($\Phi$ = 0.5 revolutions), the waveform determination algorithm 4322 computes a waveform template $\Pi$ "on the fly" as a function of both discrete phase $\Phi$ and time t measured since the most recent trigger instant. In one such form, the waveform determination algorithm 4322 computes the waveform template $\Pi(\Phi, t)$ in two portions (inspiratory and expiratory) as follows:

$$\Pi(\Phi,t) = \begin{cases} \Pi_i(t), & \Phi = 0 \\ \Pi_e(t - T_i), & \Phi = 0.5 \end{cases}$$

where $\Pi_i(t)$ and $\Pi_e(t)$ are inspiratory and expiratory portions of the waveform template $\Pi(\Phi, t)$. In one such form, the

inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

#### *5.4.3.2.3 Ventilation determination*

In one form of the present technology, a ventilation determination algorithm 4323 receives an input a respiratory flow rate *Qr,* and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, *Qr,* optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle K proportion (by time) of the inspiratory portion, where $0 < K < 1$. There is an arbitrarily large number of measures that are exactly proportional to ventilation if the flow rate shape is constant.

#### *5.4.3.2.4 Determination of Inspiratory Flow limitation*

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal *Qr* and provides as an output a metric of the extent to which the inspiratory portion of the breath exhibits inspiratory flow limitation.

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6A. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty five scaled data points are generated by the central controller 4230, and represent a moving average of the preceding several inspiratory events, e.g., three events. The moving average of continuously updated values of the (e.g., sixty five) points are hereinafter called the "scaled flow rate ", designated as *Qs*(*t*). Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

*5.4.3.2.5 Determination of apneas and hypopneas*

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal *Qr* and provides as an output a flag that indicates that an apnea or a hypopnea has been detected.

In one form, an apnea will be said to have been detected when a function of respiratory flow rate *Qr* falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate *Qr* falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

*5.4.3.2.6 Determination of snore*

In one form of the present technology, the central controller 4230 executes one or more snore determination algorithms 4326 for the determination of the extent of snore.

In one form, the snore determination algorithm 4326 receives as an input a respiratory flow rate signal *Qr* and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal *Qr* to reduce background noise, e.g., the sound of airflow in the system from the blower.

*5.4.3.2.7 Determination of airway patency*

In one form of the present technology, the central controller 4230 executes one or more airway patency determination algorithms 4327 for the determination of the extent of airway patency.

In one form, the airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure *Pt.* In one implementation, the forced oscillation is of frequency 2 Hz with amplitude about 1 $cmH_2O$.

In one form, airway patency determination algorithm 4327 receives as an input a respiratory flow rate signal *Qr,* and determines the presence or absence of a cardiogenic signal. The absence of a cardiogenic signal is taken to be an indication of a closed airway.

*5.4.3.2.8 Determination of target ventilation*

In one form of the present technology, the central controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

#### *5.4.3.2.9 Determination of therapy parameters*

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi\left(\Phi, t\right) + P_0 \qquad (1)$$

where:

- A is the amplitude,
- $\Pi(\Phi, t)$ is the waveform template value (in the range 0 to 1) at the current value $\Phi$ of phase and $t$ of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude A and the base pressure $P_0$ may be set by the therapy parameter determination algorithm 4329 depending on the chosen respiratory pressure therapy mode in the manner described below.

#### 5.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose interface pressure *Pm* at the patient interface 3000 or 3800 is equal to the treatment pressure *Pt.*

#### 5.4.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device

- Logging of the incident

5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,732,349.

In some forms, cabling to carry power or data may also be incorporated into the air circuit 4170, for example by winding the cabling around the external surface of the air circuit 4170. The cabling thus wound may be held in place by one or more layers of a textile material, for example.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

5.6 HUMIDIFIER

### 5.6.1 Humidifier overview

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.6.2 Humidifier components

#### 5.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

#### 5.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

#### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

#### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

#### 5.6.2.5 Humidifier transducer(s)

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 5C. A humidifier transducer 5210 may produce one or more output signals which may be communicated to a controller such as the central controller 4230 and/or the humidifier controller 5250. In some forms, a humidifier transducer may be located externally to the humidifier 5000 (such as in the *air* circuit 4170) while communicating the output signal to the controller.

##### *5.6.2.5.1 Pressure transducer*

One or more pressure transducers 5212 may be provided to the humidifier 5000 in addition to, or instead of, a pressure sensor 4272 provided in the RPT device 4000.

##### *5.6.2.5.2 Flow rate transducer*

One or more flow rate transducers 5214 may be provided to the humidifier 5000 in addition to, or instead of, a flow rate sensor 4274 provided in the RPT device 4000.

##### *5.6.2.5.3 Temperature transducer*

The humidifier 5000 may comprise one or more temperature transducers 5216. The one or more temperature transducers 5216 may be configured to measure one or more temperatures such as of the heating element 5240 and/or of the flow of *air* downstream of the humidifier outlet 5004. In some forms, the humidifier 5000 may further comprise a temperature sensor 5216 to detect the temperature of the ambient air.

##### *5.6.2.5.4 Humidity transducer*

In one form, the humidifier 5000 may comprise one or more humidity sensors 5218 to detect a humidity of a gas, such as the ambient air. The humidity sensor 5218 may be placed towards the humidifier outlet 5004 in some forms to measure a humidity of the gas delivered from the humidifier 5000. The humidity sensor may be an absolute humidity sensor or a relative humidity sensor.

#### 5.6.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/17072.

In some forms, the heating element 5240 may be provided in the humidifier base 5006 where heat may be provided to the humidifier reservoir 5110 primarily by conduction as shown in Fig. 5B.

#### 5.6.2.7 Humidifier controller

According to one arrangement of the present technology, a humidifier 5000 may comprise a humidifier controller 5250 as shown in Fig. 5C. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 5C, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

## 5.7 SCREENING, DIAGNOSIS, MONITORING SYSTEMS

### 5.7.1 Overview

At least some forms of the present technology allow for screening, diagnosis and/or monitoring of sleep health using sensors incorporated in a patient interface. The sensors may be provided in a positioning and stabilising structure of the patient interface, such as the positioning and stabilising structure 6300 of Fig. 4A and 4B, and/or in a plenum chamber of the patient interface.

For example, as mentioned, a sensor-enabled positioning and stabilising structure in the form of a headband, such as positioning and stabilising structure 6300, may be worn as a standalone headgear by the patient prior to commencing therapy, and sensor measurements recorded during sleep. The sensor measurements may be used to accurately monitor sleep stages and sleeping positions, to track vital signs and other physiological indicators, and to detect apnea and/or hypopnea events. The sleep data and physiological data may be used by clinicians to diagnose sleep disorders and to recommend appropriate therapy. Further, the same parameters may be monitored by the sensors during therapy, and compared to the parameters prior to commencing therapy (or at an earlier stage of therapy) to enable the patient and the clinician to assess the efficacy of the therapy.

As mentioned, physiological and sleep data may be recorded by one or more sensors of the patient interface, and communicated to external computing devices such as a smartphone of the patient, and/or a monitoring server that is operated by or accessible to a clinician or other healthcare provider.

In some forms of the present technology, a pulse oximeter incorporated in the positioning and stabilising structure 6300 may be used to determine blood oxygen saturation level and heart rate during the period that the patient interface 6000 is worn, and this data may be transmitted to an external computing device such as a smartphone, other mobile computing device, or laptop or desktop computing system of the patient. The time series data may be consolidated to provide feedback to the patient on their health levels, and recommendations for follow-up (for example, by a clinician), for example based on determination of an AHI as discussed above, which can be used in conjunction with other sensor measurements to determine when and how often apnea events are occurring, and an optimal treatment regimen for the patient.

Other sensors which may be incorporated in a screening, diagnosis and/or monitoring system that comprises a sensor-enabled patient interface include, without limitation: an EEG sensor for detection of sleep stages; EMG and EOG sensors for determining REM sleep stage occurrences; a microphone for detecting snoring or other sounds indicative of disturbed sleep; a humidity sensor, temperature sensor, pressure sensor, and/or $CO_2$ sensor, each provided internally of a plenum chamber 6200 of the patient interface.

Some example applications of sensor-enabled patient interfaces will now be described.

### 5.7.2 Polysomnography

Polysomnography (PSG) is a monitoring process that typically involves a variety of different sensors and associated equipment, and that can be challenging to set up, even for an expert. A typical PSG system comprises a headbox which receives and records signals from the following sensors: an EOG electrode; an EEG electrode; an ECG electrode; a submental EMG electrode; a snore sensor; a respiratory inductance plethysmogram (respiratory effort sensor) on a chest band; a respiratory inductance plethysmogram (respiratory effort sensor) on an abdominal band; an oro-nasal cannula with oral thermistor; a photoplethysmograph (pulse oximeter); and a body position sensor. The electrical signals are referred to a ground electrode (ISOG) positioned in the centre of the forehead.

A sensor-enabled patient interface, such as patient interface 6000, can replace some or all of the functions of an existing PSG system, since, as discussed above, some or all of the sensors used by a PSG system can be provided in the positioning and stabilising structure (e.g. 6300) and/or in the plenum chamber (e.g. 6200). For example, EOG, EEG, ECG and EMG electrodes, a microphone (acting as a snore sensor), a PPG sensor, and an accelerometer and/or gyroscope (acting as body position sensor(s)) can all be provided in upper textile portion 6310 and/or lower textile portions 6308, 6320 of the positioning and stabilising structure 6300. A ground electrode may also be provided (e.g. in the lower textile portion 6308 for placement behind the patient's ear, as described above).

By integrating the sensors with the patient interface 6000, in at least some examples, it becomes far more straightforward to implement PSG, since the patient is simply able to wear the patient interface 6000 in the manner in which they would do so for therapy, with no or minimal additional configuration required (e.g., no need to manually place various electrodes for the EEG/EMG/EOG sensors).

### 5.7.3 Non-obtrusive monitoring system

In one example, one or more accelerometers and/or one or more gyroscopes and/or one or more other motion sensors may be provided in the positioning and stabilising structure 6300. The motion sensor(s) is or are configured to generate one or more signals representing bodily movement of the patient, from which may be obtained a signal representing respiratory movement of the patient.

### 5.7.4 Respiratory polygraphy

Respiratory polygraphy (RPG) is a term for a simplified form of PSG without the electrical signals (EOG, EEG, EMG), snore, or body position sensors. Ordinarily, RPG comprises at least a thoracic movement signal from a respiratory inductance plethysmogram (movement sensor) on a chest band, a nasal pressure signal sensed via a nasal cannula, and an oxygen saturation signal from a pulse oximeter, e.g. the pulse oximeter. The three RPG signals, or channels, are received by an RPG headbox.

A sensor-enabled patient interface, such as patient interface 6000, can replace some or all of the functions of an existing RPG system. For example, accelerometer and/or gyroscope measurements from sensors mounted in headgear 6300 may be used as a proxy for a thoracic movement signal. A nasal pressure signal may be measured by a pressure sensor mounted inside the plenum chamber, for example on the internal surface thereof so as to lie adjacent to the patient's nares in use. An oxygen saturation signal may be measured by a PPG sensor mounted in headgear 6300, for example as shown at 6355 in Fig. 9C. The proxy thoracic movement signal, the nasal pressure signal, and the oxygen saturation signal may be received by an on-board processor 6350 of the patient interface 6000, and/or may be transmitted to an external computing device for analysis in the same manner as conventional RPG signals.

In certain configurations, a nasal pressure signal is a satisfactory proxy for a nasal flow rate signal generated by a flow rate transducer in-line with a sealed nasal mask, in that the nasal pressure signal is comparable in shape to the nasal flow rate signal. The nasal flow rate in turn is equal to the respiratory flow rate if the patient's mouth is kept closed, i.e. in the absence of mouth leaks.

### 5.8 PORTABLE OXYGEN CONCENTRATOR (POC)

Portable oxygen concentrators may take advantage of pressure swing adsorption (PSA). Pressure swing adsorption may involve using one or more compressors to increase gas pressure inside a canister that contains particles of a gas separation adsorbent arranged in a "sieve bed". As the pressure increases, certain molecules in the gas may become adsorbed onto the gas separation adsorbent. Removal of a portion of the gas in the canister under the pressurized conditions allows separation of the non-adsorbed molecules from the adsorbed molecules. The gas separation adsorbent may be regenerated by reducing the pressure, which reverses the adsorption of molecules from the adsorbent. Further details regarding oxygen concentrators may be found, for example, in U.S. Published Patent Application No. 2009-0065007, published March 12, 2009, and entitled "Oxygen Concentrator Apparatus and Method".

Ambient air usually includes approximately 78% nitrogen and 21% oxygen with the balance comprised of argon, carbon dioxide, water vapor and other trace gases. If a gas mixture such as air, for example, is passed under pressure through a canister containing a gas separation adsorbent bed that attracts nitrogen more strongly than it does oxygen, part or all of the nitrogen will stay in the bed, and the gas coming out of the canister will be enriched in oxygen. When the bed reaches the end of its capacity to adsorb nitrogen, it can be regenerated by reducing the pressure, thereby releasing the adsorbed nitrogen. It is then ready for another cycle of producing oxygen enriched air. By alternating canisters in a two-canister system, one canister can be separating oxygen while the other canister is being purged (resulting in a continuous separation of the oxygen from the nitrogen). In this manner, oxygen enriched air can be accumulated, such as in a storage container or other pressurizable vessel or conduit coupled to the canisters, for a variety of uses including providing supplemental oxygen to patients.

5.9 RESPIRATORY THERAPY MODES

Various respiratory therapy modes may be implemented by the disclosed respiratory therapy system.

**5.9.1 CPAP therapy**

In some implementations of respiratory pressure therapy, the central controller 4230 sets the treatment pressure *Pt* according to the treatment pressure equation (1) as part of the therapy parameter determination algorithm 4329. In one such implementation, the amplitude A is identically zero, so the treatment pressure *Pt* (which represents a target value to be achieved by the interface pressure *Pm* at the current instant of time) is identically equal to the base pressure $P_0$ throughout the respiratory cycle. Such implementations are generally grouped under the heading of CPAP therapy. In such implementations, there is no need for the therapy engine module 4320 to determine phase Φ or the waveform template Π (Φ).

In CPAP therapy, the base pressure $P_0$ may be a constant value that is hard-coded or manually entered to the RPT device 4000. Alternatively, the central controller 4230 may repeatedly compute the base pressure $P_0$ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

Fig. 4E is a flow chart illustrating a method 4500 carried out by the central controller 4230 to continuously compute the base pressure $P_0$ as part of an APAP therapy implementation of the therapy parameter determination algorithm 4329, when the pressure support A is identically zero.

The method 4500 starts at step 4520, at which the central controller 4230 compares the measure of the presence of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the central controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the central controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the central controller 4230 increases the base pressure $P_0$ by a predetermined pressure increment $\Delta P$, provided the resulting treatment pressure *Pt* would not exceed a maximum treatment pressure *Pmax.* In one implementation, the predetermined pressure increment $\Delta P$ and maximum treatment pressure *Pmax* are 1 $cmH_2O$ and 25 $cmH_2O$ respectively. In other implementations, the pressure increment $\Delta P$ can be as low as 0.1 $cmH_2O$ and as high as 3 $cmH_2O$, or as low as 0.5 $cmH_2O$ and as high as 2 $cmH_2O$. In other implementations, the maximum treatment pressure *Pmax* can be as low as 15 $cmH_2O$ and as high as 35 $cmH_2O$, or as low as 20 $cmH_2O$ and as high as 30 $cmH_2O$. The method 4500 then returns to step 4520.

At step 4560, the central controller 4230 decreases the base pressure $P_0$ by a decrement, provided the decreased base pressure $P_0$ would not fall below a minimum treatment pressure *Pmin.* The method 4500 then returns to step 4520. In one implementation, the decrement is proportional to the value of $P_0$-*Pmin,* so that the decrease in $P_0$ to the minimum treatment pressure *Pmin* in the absence of any detected events is exponential. In one implementation, the constant of proportionality is set such that the time constant $\tau$ of the exponential decrease of $P_0$ is 60 minutes, and the minimum treatment pressure *Pmin* is 4 $cmH_2O$. In other implementations, the time constant $\tau$ could be as low as 1 minute and as high as 300 minutes, or as low as 5 minutes and as high as 180 minutes. In other implementations, the minimum treatment pressure *Pmin* can be as low as 0 $cmH_2O$ and as high as 8 $cmH_2O$, or as low as 2 $cmH_2O$ and as high as 6

$cmH_2O$. Alternatively, the decrement in $P_0$ could be predetermined, so the decrease in $P_0$ to the minimum treatment pressure *Pmin* in the absence of any detected events is linear.

### 5.9.2 Bi-level therapy

In other implementations of this form of the present technology, the value of amplitude A in equation (1) may be positive. Such implementations are known as bi-level therapy, because in determining the treatment pressure *Pt* using equation (1) with positive amplitude A, the therapy parameter determination algorithm 4329 oscillates the treatment pressure *Pt* between two values or levels in synchrony with the spontaneous respiratory effort of the patient 1000. That is, based on the typical waveform templates $\Pi(\Phi, t)$ described above, the therapy parameter determination algorithm 4329 increases the treatment pressure *Pt* to $P_0 + A$ (known as the IPAP) at the start of, or during, or inspiration and decreases the treatment pressure *Pt* to the base pressure $P_0$ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude *A*, which has a "small" value (a few $cmH_2O$) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing returned by the respective algorithms in the therapy engine module 4320 in analogous fashion to the computation of the base pressure $P_0$ in APAP therapy described above.

In other forms of bi-level therapy, the amplitude *A* is large enough that the RPT device 4000 does some or all of the work of breathing of the patient 1000. In such forms, known as pressure support ventilation therapy, the amplitude A is referred to as the pressure support, or swing. In pressure support ventilation therapy, the IPAP is the base pressure $P_0$ plus the pressure support A, and the EPAP is the base pressure $P_0$.

In some forms of pressure support ventilation therapy, known as fixed pressure support ventilation therapy, the pressure support *A* is fixed at a predetermined value, e.g. 10 $cmH_2O$. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure *Vent* of ventilation) and a target value of that respiratory parameter (e.g. a target value *Vtgt* of ventilation) and repeatedly adjusts the parameters of equation (1) to bring the current measure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp*, as described above.

In some forms of servo-ventilation, the therapy parameter determination algorithm 4329 applies a control methodology to repeatedly compute the pressure support *A* so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp*, the pressure support *A* is repeatedly computed as:

$$A = G\int (Vent - Vtgt)dt \qquad (2)$$

where *G* is the gain of the PI control. Larger values of gain G can result in positive feedback in the therapy engine module 4320. Smaller values of gain *G* may permit some residual untreated CSR or central sleep apnea. In some implementations, the gain G is fixed at a predetermined value, such as -0.4 $cmH_2O/(L/min)/sec$. Alternatively, the gain G may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations In yet other implementations, the gain *G* may vary depending on the difference between the current measure *Vent* of ventilation and the target ventilation *Vtgt*.

Other servo-ventilation control methodologies that may be applied by the therapy parameter determination algorithm 4329 include proportional (P), proportional-differential (PD), and proportional-integral-differential (PID).

The value of the pressure support *A* computed via equation (2) may be clipped to a range defined as [*Amin, Amax*]. In this implementation, the pressure support *A* sits by default at the minimum pressure support *Amin* until the measure of current ventilation *Vent* falls below the target ventilation *Vtgt,* at which point *A* starts increasing, only falling back to *Amin* when *Vent* exceeds *Vtgt* once again.

The pressure support limits *Amin* and *Amax* are settings of the RPT device 4000, set for example by hard-coding during configuration of the RPT device 4000 or by manual entry through the input device 4220.

In pressure support ventilation therapy modes, the EPAP is the base pressure $P_0$. As with the base pressure $P_0$ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure $P_0$ in constant CPAP therapy.

Alternatively, the therapy parameter determination algorithm 4329 may repeatedly compute the base pressure $P_0$ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

### 5.9.3 High flow therapy

In other forms of respiratory therapy, the pressure of the flow of air is not controlled as it is for respiratory pressure therapy. Rather, the central controller 4230 controls the pressure generator 4140 to deliver a flow of air whose device flow rate *Qd* is controlled to a treatment or target flow rate *Qtgt* that is typically positive throughout the patient's breathing cycle. Such forms are generally grouped under the heading of flow therapy. In flow therapy, the treatment flow rate *Qtgt* may be a constant value that is hard-coded or manually entered to the RPT device 4000. If the treatment flow rate *Qtgt* is sufficient to exceed the patient's peak inspiratory flow rate, the therapy is generally referred to as high flow therapy (HFT). Alternatively, the treatment flow rate may be a profile *Qtgt*(*t*) that varies over the respiratory cycle.

## 5.10 GLOSSARY

For the purposes of the present technology disclosure, in certain forms of the present technology, one or more of the following definitions may apply. In other forms of the present technology, alternative definitions may apply.

### 5.10.1 General

*Air*: In certain forms of the present technology, air may be taken to mean atmospheric air, and in other forms of the present technology air may be taken to mean some other combination of breathable gases, e.g. oxygen enriched air.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q.* 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd*, is the flow rate of air leaving the RPT device. Total flow rate, *Qt*, is the flow rate of air and any supplementary gas reaching the patient interface via the air circuit. Vent flow rate, *Qv*, is the flow rate of air leaving a vent to allow washout of exhaled gases. Leak flow rate, *Ql*, is the flow rate of leak from a patient interface system or elsewhere. Respiratory flow rate, *Qr*, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air:* Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or greater.

*Patient:* A person, whether or not they are suffering from a respiratory condition.

*Pressure:* Force per unit area. Pressure may be expressed in a range of units, including $cmH_2O$, g-f/$cm^2$ and hectopascal. 1 $cmH_2O$ is equal to 1 g-f/$cm^2$ and is approximately 0.98 hectopascal (1 hectopascal = 100 Pa = 100 N/$m^2$ = 1 millibar ~ 0.001 atm). In this specification, unless otherwise stated, pressure is given in units of $cmH_2O$.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

#### 5.10.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240

*Polycarbonate:* a thermoplastic polymer of Bisphenol-A Carbonate.

#### 5.10.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component*: The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component*: A structure or component that will not substantially change shape when subject to the loads typically encountered in use. An example of such a use may be setting up and maintaining a patient interface in sealing relationship with an entrance to a patient's airways, e.g. at a load of approximately 20 to 30 $cmH_2O$ pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.10.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, *Ti* to total breath time, *Ttot.*

*Effort (breathing)*: The work done by a spontaneously breathing person attempting to breathe.

*Expiratory portion of a breathing cycle:* The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

*(i) Flattened*: Having a rise followed by a relatively flat portion, followed by a fall.

(ii) *M-shaped*: Having two local peaks, one at the leading edge, and one at the trailing edge, and a relatively flat portion between the two peaks.

(iii) *Chair-shaped*: Having a single local peak, the peak being at the leading edge, followed by a relatively flat portion.

(iv) *Reverse-chair shaped*: Having a relatively flat portion followed by single local peak, the peak being at the trailing edge.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate,* patient *airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of one inspiratory portion of a respiratory flow rate waveform and the start of the following inspiratory portion of the respiratory flow rate waveform.

*Typical recent ventilation*: The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent)*: A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.10.3 Ventilation

*Adaptive Servo-Ventilator (ASV)*: A servo-ventilator that has a changeable, rather than fixed target ventilation. The changeable target ventilation may be learned from some characteristic of the patient, for example, a respiratory characteristic of the patient.

*Backup rate*: A parameter of a ventilator that establishes the minimum breathing rate (typically in number of breaths per minute) that the ventilator will deliver to the patient, if not triggered by spontaneous respiratory effort.

*Cycled*: The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP)*: a base pressure, to which a pressure varying within the breath is added to produce the desired interface pressure which the ventilator will attempt to achieve at a given time.

*End expiratory pressure (EEP)*: Desired interface pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.

*Inspiratory positive airway pressure (IPAP):* Maximum desired interface pressure which the ventilator will attempt to achieve during the inspiratory portion of the breath.

*Pressure support*: A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous/Timed (S/T)*: A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator, or other respiratory therapy device such as an RPT device or portable oxygen concentrator, delivers a volume of breathable gas to a spontaneously breathing patient, it is said to be triggered to do so.

Triggering usually takes place at or near the initiation of the respiratory portion of the breathing cycle by the patient's efforts.

### 5.10.4 Anatomy

#### 5.10.4.1 Anatomy of the face

*Ala:* the external outer wall or "wing" of each nostril (plural: alar)

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

#### 5.10.4.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal*

*process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.10.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.10.5 Patient interface

*Anti-asphyxia valve (AAV):* The component or sub-assembly of a mask system that, by opening to atmosphere in a failsafe manner, reduces the risk of excessive $CO_2$ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.10.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p*. See Fig. 3B to Fig. 3F, which illustrate examples of cross-sections at point *p* on a surface, and the resulting plane curves. Figs. 3B to 3F also illustrate an outward normal vector at *p*. The outward normal vector at *p* points away from the surface. In some examples we describe the surface from the point of view of an imaginary small person standing upright on the surface.

#### 5.10.6.1 Curvature in one dimension

The curvature of a plane curve at *p* may be described as having a sign (e.g. positive, negative) and a magnitude (e.g. 1/radius of a circle that just touches the curve at *p).*

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

#### 5.10.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.10.6.3 Space curves

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).
Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.10.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.
A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

## 5.11 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.
Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.
Furthermore, where a value or values are stated herein as being implemented as part of the technology, it is understood that such values may be approximated, unless otherwise stated, and such values may be utilized to any suitable significant digit to the extent that a practical technical implementation may permit or require it.
Furthermore, "approximately", "substantially", "about", or any similar term used herein means +/- 5-10% of the recited value.
Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.
When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.
The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.
The subject headings used in the detailed description are included only for the ease of reference of the reader and should not be used to limit the subject matter found throughout the disclosure or the claims. The subject headings should not be used in construing the scope of the claims or the claim limitations.
Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

## Claims

1. A patient interface (3000, 6000, 7000) comprising:

   a plenum chamber pressurisable to a therapeutic pressure of at least 6 $cmH_2O$ above ambient air pressure, said plenum chamber (3200, 6200) including a plenum chamber inlet port sized and structured to receive a flow of air at the therapeutic pressure for breathing by a patient;
   a seal-forming structure (3100, 6100) constructed and arranged to form a seal with a region of the patient's face surrounding an entrance to the patient's airways, said seal-forming structure (3100, 6100) having a hole therein such that the flow of air at said therapeutic pressure is delivered to at least an entrance to the patient's nares, the seal-forming structure (3100, 6100) constructed and arranged to maintain said therapeutic pressure in the plenum chamber (3200, 6200) throughout the patient's respiratory cycle in use;
   a positioning and stabilising structure (3300, 6300) to provide a force to hold the seal-forming structure (3100, 6100) in a therapeutically effective position on the patient's head; and
   a power system for converting energy generated during use of the patient interface (3000, 6000, 7000) to converted electrical energy for powering at least one portion of the patient interface (3000, 6000, 7000), wherein the energy generated during use of the patient interface (3000, 6000, 7000) is one or more of: mechanical energy generated by movement of the patient and thermal energy from the patient's skin and/or exhaled air.

2. The patient interface (3000, 6000, 7000) according to claim 1, wherein the power system provides a DC voltage to the at least one portion of the patient interface (3000, 6000, 7000), preferably the at least one portion of the patient interface (3000, 6000, 7000) is an electrical energy storage device electrically connected to the power system and configured to provide electrical energy to the power system, more preferably the power system is configured to provide the converted electrical energy to the electrical energy storage device to recharge the electrical energy storage device.

3. The patient interface (3000, 6000, 7000) according to claim 2, wherein an external charging circuit is electrically connected to the electrical energy storage device and is configured to provide electrical energy to the electrical energy storage device.

4. The patient interface (3000, 6000, 7000) according to any one of claims 1-3, wherein:

   the at least one portion of the patient interface (3000, 6000, 7000) further includes one or more electronic components (7030, 7040, 7050) for monitoring, diagnosing and/or treating the patient, and
   the one or more electronic components (7030, 7040, 7050) receives electrical energy directly from the power system.

5. The patient interface (3000, 6000, 7000) according to any one of claims 2-3, wherein:

   the at least one portion of the patient interface (3000, 6000, 7000) further includes one or more electronic components (7030, 7040, 7050) for monitoring, diagnosing and/or treating the patient, and
   the one or more electronic components (7030, 7040, 7050) receives electrical energy directly from the electrical energy storage device.

6. The patient interface (3000, 6000, 7000) according to claim 4 or claim 5, wherein the one or more electronic components (7030, 7040, 7050) is provided in, or on, one or more of the plenum chamber (3200, 6200), the seal-forming structure (3100, 6100) and the positioning and stabilising structure (3300, 6300).

7. The patient interface (3000, 6000, 7000) according to any one of claims 1-6, wherein the power system comprises at least one energy-harvesting device.

8. The patient interface (3000, 6000, 7000) according to claim 7, wherein the at least one energy-harvesting device comprises a turbine generator (8000), preferably the turbine generator (8000) comprises a rotor (8100, 9000) that comprises a plurality of turbine blades and a plurality of magnets (9004) disposed around a periphery of the rotor (8100, 9000).

**9.** The patient interface (3000, 6000, 7000) according to claim 8, wherein the turbine generator (8000) comprises a sealed stator assembly housing a plurality of coils (8310), the sealed stator assembly being disposed about the rotor (8100, 9000), preferably the sealed stator assembly further includes a plurality of bobbins (8308), the plurality of coils (8310) wrapped around the plurality of bobbins (8308), more preferably a number of bobbins (8308) in the plurality of bobbins (8308) is equal to a number of magnets (9004) in the plurality of magnets (9004).

**10.** The patient interface (3000, 6000, 7000) according to claim 8 or claim 9, wherein the turbine generator (8000) is positioned in an air inlet of the patient interface (3000, 6000, 7000), upstream from the patient in use or the turbine generator (8000) is positioned in an air outlet of the patient interface (3000, 6000, 7000), downstream from the patient in use.

**11.** The patient interface (3000, 6000, 7000) according to any one of claims 7-10, wherein the at least one energy-harvesting device comprises at least one piezoelectric film, preferably at least one of said piezoelectric film is attached to or disposed within the plenum chamber (3200, 6200) and/or the seal-forming structure (3100, 6100) and/or the positioning and stabilising structure (3300, 6300).

**12.** The patient interface (3000, 6000, 7000) of claim 11, wherein said piezoelectric film is a sensor and is configured to measure patient snoring by sensing vibration and/or noise.

**13.** The patient interface (3000, 6000, 7000) according to claim 10 or claim 11, wherein the piezoelectric film is positioned in an air inlet of the patient interface (3000, 6000, 7000), upstream from the patient in use or the piezoelectric film is positioned in an air outlet of the patient interface (3000, 6000, 7000), downstream from the patient in use.

**14.** The patient interface (3000, 6000, 7000) according to any one of claims 11-13, wherein the at least one piezoelectric film is sandwiched between layers of the positioning and stabilising structure (3300, 6300), the at least one piezoelectric film configured to flex as a result of movement in the positioning and stabilising structure (3300, 6300).

**15.** The patient interface (3000, 6000, 7000) according to any one of claims 7-14, wherein the at least one energy-harvesting device comprises at least one thermoelectric generator (TEG) module, preferably at least one TEG module is at least partially exposed and is arranged to contact skin of the patient, or at least one TEG module is disposed within the positioning and stabilising structure (3300, 6300), or at least one TEG module is arranged such that a first surface of said TEG module is exposed to an interior of the plenum chamber (3200, 6200), and a second surface that is opposite the first surface is exposed to ambient.

## Patentansprüche

**1.** Patientenschnittstelle (3000, 6000, 7000), aufweisend:

eine Luftkammer, die auf einen therapeutischen Druck von mindestens 6 $cmH_2O$ über dem Umgebungsluftdruck gebracht werden kann, wobei die Luftkammer (3200, 6200) eine Luftkammereinlassöffnung aufweist, die zum Aufnehmen eines Luftstroms mit dem therapeutischem Druck zum Atmen durch einen Patienten bemessen und ausgebildet ist;
eine dichtungsbildende Struktur (3100, 6100), die so aufgebaut und angeordnet ist, dass sie eine Dichtung mit einem Bereich des Gesichts des Patienten bildet, der einen Eingang zu den Atemwegen des Patienten umgibt, wobei die dichtungsbildende Struktur (3100, 6100) ein Loch darin aufweist, sodass der Luftstrom mit dem therapeutischen Druck mindestens einem Eingang zu den Nasenlöchern des Patienten zugeführt wird, wobei die dichtungsbildende Struktur (3100, 6100) so aufgebaut und angeordnet ist, dass sie im Gebrauch den therapeutischen Druck in der Luftkammer (3200, 6200) während des gesamten Atemzyklus des Patienten aufrechterhält;
eine Positionierungs- und Stabilisierungsstruktur (3300, 6300) zum Bereitstellen einer Kraft zum Halten der dichtungsbildenden Struktur (3100, 6100) in einer therapeutisch wirksamen Position am Kopf des Patienten; und
ein Energiesystem zum Umwandeln von Energie, die während des Gebrauchs der Patientenschnittstelle (3000, 6000, 7000) erzeugt wird, in umgewandelte Elektroenergie zum Betreiben mindestens eines Teils der Patientenschnittstelle (3000, 6000, 7000), wobei die während des Gebrauchs der Patientenschnittstelle (3000, 6000, 7000) erzeugte Energie eine oder mehrere der folgenden ist: mechanische Energie, die durch die Bewegung des Patienten erzeugt wird, und Wärmeenergie von der Haut und/oder der Ausatemluft des Patienten.

2. Patientenschnittstelle (3000, 6000, 7000) nach Anspruch 1, wobei das Energiesystem dem mindestens einen Teil der Patientenschnittstelle (3000, 6000, 7000) eine Gleichspannung zuführt, wobei der mindestens eine Teil der Patientenschnittstelle (3000, 6000, 7000) bevorzugt eine Elektroenergiespeichervorrichtung ist, die elektrisch mit dem Energiesystem verbunden und zum Bereitstellen von Elektroenergie für das Energiesystem konfiguriert ist, wobei das Energiesystem stärker bevorzugt zum Zuführen der umgewandelten Elektroenergie zu der Elektroenergiespeichervorrichtung konfiguriert ist, um die Elektroenergiespeichervorrichtung aufzuladen.

3. Patientenschnittstelle (3000, 6000, 7000) nach Anspruch 2, wobei eine externe Ladeschaltung elektrisch mit der Elektroenergiespeichervorrichtung verbunden ist und so konfiguriert ist, dass sie der Elektroenergiespeichervorrichtung Elektroenergie zuführt.

4. Patientenschnittstelle (3000, 6000, 7000) nach einem der Ansprüche 1-3, wobei:

der mindestens eine Teil der Patientenschnittstelle (3000, 6000, 7000) ferner eine oder mehrere elektronische Komponenten (7030, 7040, 7050) zur Überwachung, Diagnose und/oder Behandlung des Patienten aufweist, und
die eine oder die mehreren elektronischen Komponenten (7030, 7040, 7050) Elektroenergie direkt vom Energiesystem erhalten.

5. Patientenschnittstelle (3000, 6000, 7000) nach einem der Ansprüche 2-3, wobei:

der mindestens eine Teil der Patientenschnittstelle (3000, 6000, 7000) ferner eine oder mehrere elektronische Komponenten (7030, 7040, 7050) zur Überwachung, Diagnose und/oder Behandlung des Patienten aufweist, und
die eine oder die mehreren elektronischen Komponenten (7030, 7040, 7050) Elektroenergie direkt von der Elektroenergiespeichervorrichtung erhalten.

6. Patientenschnittstelle (3000, 6000, 7000) nach Anspruch 4 oder Anspruch 5, wobei die eine oder die mehreren elektronischen Komponenten (7030, 7040, 7050) in oder auf der Luftkammer (3200, 6200), der dichtungsbildenden Struktur (3100, 6100) und/oder der Positionierungs- und Stabilisierungsstruktur (3300, 6300) vorgesehen sind.

7. Patientenschnittstelle (3000, 6000, 7000) nach einem der Ansprüche 1 bis 6, wobei das Energiesystem mindestens eine Energiegewinnungsvorrichtung aufweist.

8. Patientenschnittstelle (3000, 6000, 7000) nach Anspruch 7, wobei die mindestens eine Energiegewinnungsvorrichtung einen Turbinengenerator (8000) aufweist, wobei der Turbinengenerator (8000) bevorzugt einen Rotor (8100, 9000) aufweist, der mehrere Turbinenschaufeln und mehrere Magnete (9004) aufweist, die um einen Umfang des Rotors (8100, 9000) angeordnet sind.

9. Patientenschnittstelle (3000, 6000, 7000) nach Anspruch 8, wobei der Turbinengenerator (8000) eine abgedichtete Statorbaugruppe aufweist, die mehrere Spulen (8310) aufnimmt, wobei die abgedichtete Statorbaugruppe um den Rotor (8100, 9000) herum angeordnet ist, die abgedichtete Statorbaugruppe ferner bevorzugt mehrere Spulenkörper (8308) aufweist, wobei die mehreren Spulen (8310) um die mehreren Spulenkörper (8308) gewickelt sind, stärker bevorzugt eine Anzahl von Spulenkörpern (8308) von den mehreren Spulenkörpern (8308) gleich einer Anzahl von Magneten (9004) von den mehreren Magneten (9004) ist.

10. Patientenschnittstelle (3000, 6000, 7000) nach Anspruch 8 oder Anspruch 9, wobei der Turbinengenerator (8000) im Gebrauch in einem Lufteinlass der Patientenschnittstelle (3000, 6000, 7000) stromaufwärts vom Patienten angeordnet ist oder der Turbinengenerator (8000) im Gebrauch in einem Luftauslass der Patientenschnittstelle (3000, 6000, 7000) stromabwärts vom Patienten angeordnet ist.

11. Patientenschnittstelle (3000, 6000, 7000) nach einem der Ansprüche 7-10, wobei die mindestens eine Energiegewinnungsvorrichtung mindestens einen piezoelektrischen Film aufweist, wobei bevorzugt mindestens einer der piezoelektrischen Filme an der Luftkammer (3200, 6200) und/oder der dichtungsbildenden Struktur (3100, 6100) und/oder der Positionierungs- und Stabilisierungsstruktur (3300, 6300) angebracht oder darin angeordnet ist.

12. Patientenschnittstelle (3000, 6000, 7000) nach Anspruch 11, wobei der piezoelektrische Film ein Sensor ist und zum Messen des Schnarchens des Patienten durch Erfassen von Vibrationen und/oder Geräuschen konfiguriert ist.

13. Patientenschnittstelle (3000, 6000, 7000) nach Anspruch 10 oder Anspruch 11, wobei der piezoelektrische Film im Gebrauch in einem Lufteinlass der Patientenschnittstelle (3000, 6000, 7000) stromaufwärts vom Patienten angeordnet ist oder der piezoelektrische Film im Gebrauch in einem Luftauslass der Patientenschnittstelle (3000, 6000, 7000) stromabwärts vom Patienten angeordnet ist.

14. Patientenschnittstelle (3000, 6000, 7000) nach einem der Ansprüche 11 bis 13, wobei der mindestens eine piezoelektrische Film zwischen Schichten der Positionierungs- und Stabilisierungsstruktur (3300, 6300) eingeschlossen ist, wobei der mindestens eine piezoelektrische Film dazu konfiguriert ist, sich infolge von Bewegung in der Positionierungs- und Stabilisierungsstruktur (3300, 6300) zu biegen.

15. Patientenschnittstelle (3000, 6000, 7000) nach einem der Ansprüche 7-14, wobei die mindestens eine Energiegewinnungsvorrichtung mindestens ein thermoelektrisches Generatormodul (TEG-Modul) aufweist, wobei bevorzugt mindestens ein TEG-Modul mindestens teilweise freiliegt und so angeordnet ist, dass es die Haut des Patienten berührt, oder mindestens ein TEG-Modul innerhalb der Positionierungs- und Stabilisierungsstruktur (3300, 6300) angeordnet ist, oder mindestens ein TEG-Modul so angeordnet ist, dass eine erste Fläche des TEG-Moduls zu einem Innenraum der Luftkammer (3200, 6200) hin freiliegt und eine zweite, der ersten Fläche gegenüberliegende Fläche zur Umgebung hin freiliegt.

## Revendications

1. Interface patient (3000, 6000, 7000), comprenant :

une chambre de distribution pouvant être mise sous pression à une pression thérapeutique d'au moins 6 $cmH_2O$ au-dessus de la pression d'air ambiant, ladite chambre de distribution (3200, 6200) comprenant un orifice d'entrée de chambre de distribution dimensionné et structuré pour recevoir un flux d'air à la pression thérapeutique à des fins de respiration par un patient ;
une structure de formation d'étanchéité (3100, 6100) structurée et conçue pour former une étanchéité avec une région du visage du patient entourant une entrée des voies aériennes du patient, ladite structure de formation d'étanchéité (3100, 6100) comportant un trou en son sein de façon à distribuer le flux d'air à ladite pression thérapeutique à au moins une entrée des narines du patient, la structure de formation d'étanchéité (3100, 6100) étant structurée et conçue pour maintenir ladite pression thérapeutique dans la chambre de distribution (3200, 6200) sur tout le cycle respiratoire du patient en utilisation ;
une structure de positionnement et de stabilisation (3300, 6300) servant à établir une force pour maintenir la structure de formation d'étanchéité (3100, 6100) dans une position thérapeutiquement efficace sur la tête du patient ; et
un système de puissance destiné à convertir de l'énergie générée pendant l'utilisation de l'interface patient (3000, 6000, 7000) en une énergie électrique convertie destinée à alimenter au moins une partie de l'interface patient (3000, 6000, 7000), dans laquelle l'énergie générée pendant l'utilisation de l'interface patient (3000, 6000, 7000) est l'une ou les deux : d'une énergie mécanique générée par un mouvement du patient et d'une énergie thermique provenant de la peau et/ou de l'air exhalé du patient.

2. Interface patient (3000, 6000, 7000) selon la revendication 1, dans laquelle le système de puissance fournit une tension continue à l'au moins une partie de l'interface patient (3000, 6000, 7000), de préférence l'au moins une partie de l'interface patient (3000, 6000, 7000) est un dispositif d'accumulation d'énergie électrique connecté électriquement au système de puissance et configuré pour fournir de l'énergie électrique au système de puissance, plus préférablement le système de puissance est configuré pour fournir l'énergie électrique convertie au dispositif d'accumulation d'énergie électrique pour recharger le dispositif d'accumulation d'énergie électrique.

3. Interface patient (3000, 6000, 7000) selon la revendication 2, dans laquelle un circuit de charge externe est connecté électriquement au dispositif d'accumulation d'énergie électrique et est configuré pour fournir de l'énergie électrique au dispositif d'accumulation d'énergie électrique.

4. Interface patient (3000, 6000, 7000) selon l'une quelconque des revendications 1 à 3, dans laquelle :

l'au moins une partie de l'interface patient (3000, 6000, 7000) comprend en outre un ou plusieurs composants électroniques (7030, 7040, 7050) destinés à surveiller, à diagnostiquer et/ou à traiter le patient, et
les un ou plusieurs composants électroniques (7030, 7040, 7050) reçoivent de l'énergie électrique directement du système de puissance.

5. Interface patient (3000, 6000, 7000) selon l'une quelconque des revendications 2 à 3, dans laquelle :

   l'au moins une partie de l'interface patient (3000, 6000, 7000) comprend en outre un ou plusieurs composants électroniques (7030, 7040, 7050) destinés à surveiller, à diagnostiquer et/ou à traiter le patient, et
   les un ou plusieurs composants électroniques (7030, 7040, 7050) reçoivent de l'énergie électrique directement du dispositif d'accumulation d'énergie électrique.

6. Interface patient (3000, 6000, 7000) selon la revendication 4 ou la revendication 5, dans laquelle les un ou plusieurs composants électroniques (7030, 7040, 7050) sont disposés dans, ou sur, un ou plusieurs éléments parmi la chambre de distribution (3200, 6200), la structure de formation d'étanchéité (3100, 6100) et la structure de positionnement et de stabilisation (3300, 6300).

7. Interface patient (3000, 6000, 7000) selon l'une quelconque des revendications 1 à 6, dans laquelle le système de puissance comprend au moins un dispositif de récupération d'énergie.

8. Interface patient (3000, 6000, 7000) selon la revendication 7, dans laquelle l'au moins un dispositif de récupération d'énergie comprend un turbogénérateur (8000), de préférence le turbogénérateur (8000) comprend un rotor (8100, 9000) qui comprend une pluralité d'aubes de turbine et une pluralité d'aimants (9004) disposés autour d'une périphérie du rotor (8100, 9000).

9. Interface patient (3000, 6000, 7000) selon la revendication 8, dans laquelle le turbogénérateur (8000) comprend un ensemble stator étanche logeant une pluralité d'enroulements (8310), l'ensemble stator étanche étant disposé autour du rotor (8100, 9000), de préférence l'ensemble stator étanche comprend en outre une pluralité de bobines (8308), la pluralité d'enroulements (8310) étant enroulée autour de la pluralité de bobines (8308), plus préférablement un nombre de bobines (8308) de la pluralité de bobines (8308) est égal à un nombre d'aimants (9004) de la pluralité d'aimants (9004).

10. Interface patient (3000, 6000, 7000) selon la revendication 8 ou la revendication 9, dans laquelle le turbogénérateur (8000) est positionné dans une entrée d'air de l'interface patient (3000, 6000, 7000), en amont du patient en utilisation, ou le turbogénérateur (8000) est positionné dans une sortie d'air de l'interface patient (3000, 6000, 7000), en aval du patient en utilisation.

11. Interface patient (3000, 6000, 7000) selon l'une quelconque des revendications 7 à 10, dans laquelle l'au moins un dispositif de récupération d'énergie comprend au moins un film piézoélectrique, de préférence au moins l'un dudit film piézoélectrique est fixé à la chambre de distribution (3200, 6200) et/ou à la structure de formation d'étanchéité (3100, 6100) et/ou à la structure de positionnement et de stabilisation (3300, 6300), ou est disposé dans une ou plusieurs de ces dernières.

12. Interface patient (3000, 6000, 7000) selon la revendication 11, dans laquelle ledit film piézoélectrique est un capteur et est configuré pour mesurer un ronflement de patient par une détection de vibration et/ou de bruit.

13. Interface patient (3000, 6000, 7000) selon la revendication 10 ou la revendication 11, dans laquelle le film piézoélectrique est positionné dans une entrée d'air de l'interface patient (3000, 6000, 7000), en amont du patient en utilisation, ou le film piézoélectrique est positionné dans une sortie d'air de l'interface patient (3000, 6000, 7000), en aval du patient en utilisation.

14. Interface patient (3000, 6000, 7000) selon l'une quelconque des revendications 11 à 13, dans laquelle l'au moins un film piézoélectrique est pris en sandwich entre des couches de la structure de positionnement et de stabilisation (3300, 6300), l'au moins un film piézoélectrique étant configuré pour fléchir consécutivement à un mouvement de la structure de positionnement et de stabilisation (3300, 6300).

15. Interface patient (3000, 6000, 7000) selon l'une quelconque des revendications 7 à 14, dans laquelle l'au moins un dispositif de récupération d'énergie comprend au moins un module générateur thermoélectrique (TEG), de préférence au moins un module TEG est au moins partiellement exposé et est disposé pour contacter la peau du patient, ou au moins un module TEG est disposé à l'intérieur de la structure de positionnement et de stabilisation (3300, 6300), ou au moins un module TEG est disposé de sorte qu'une première surface dudit module TEG est exposée à l'intérieur de la chambre de distribution (3200, 6200), et qu'une seconde surface qui est opposée à la première surface est exposée à l'environnement ambiant.

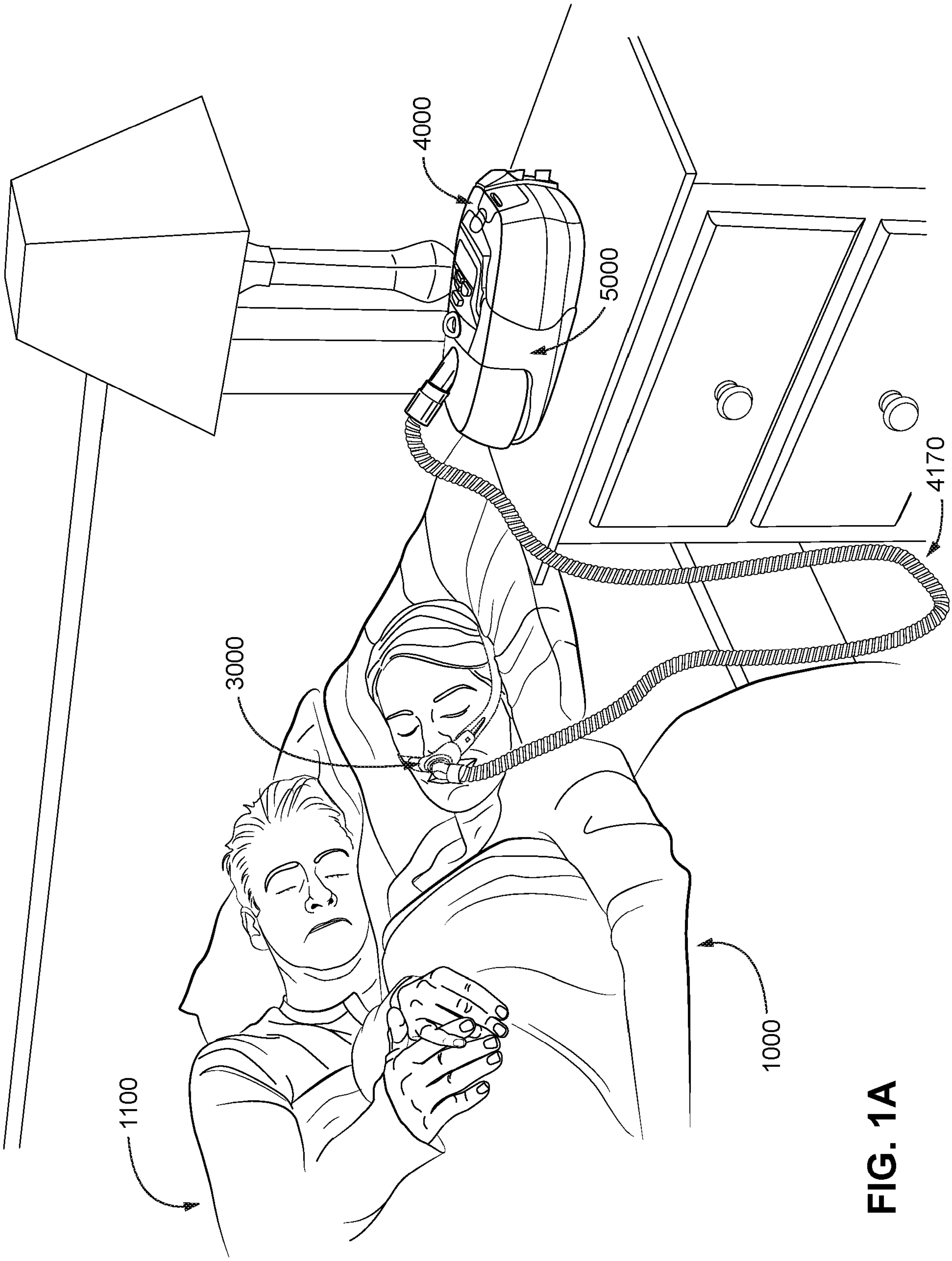


FIG. 1A

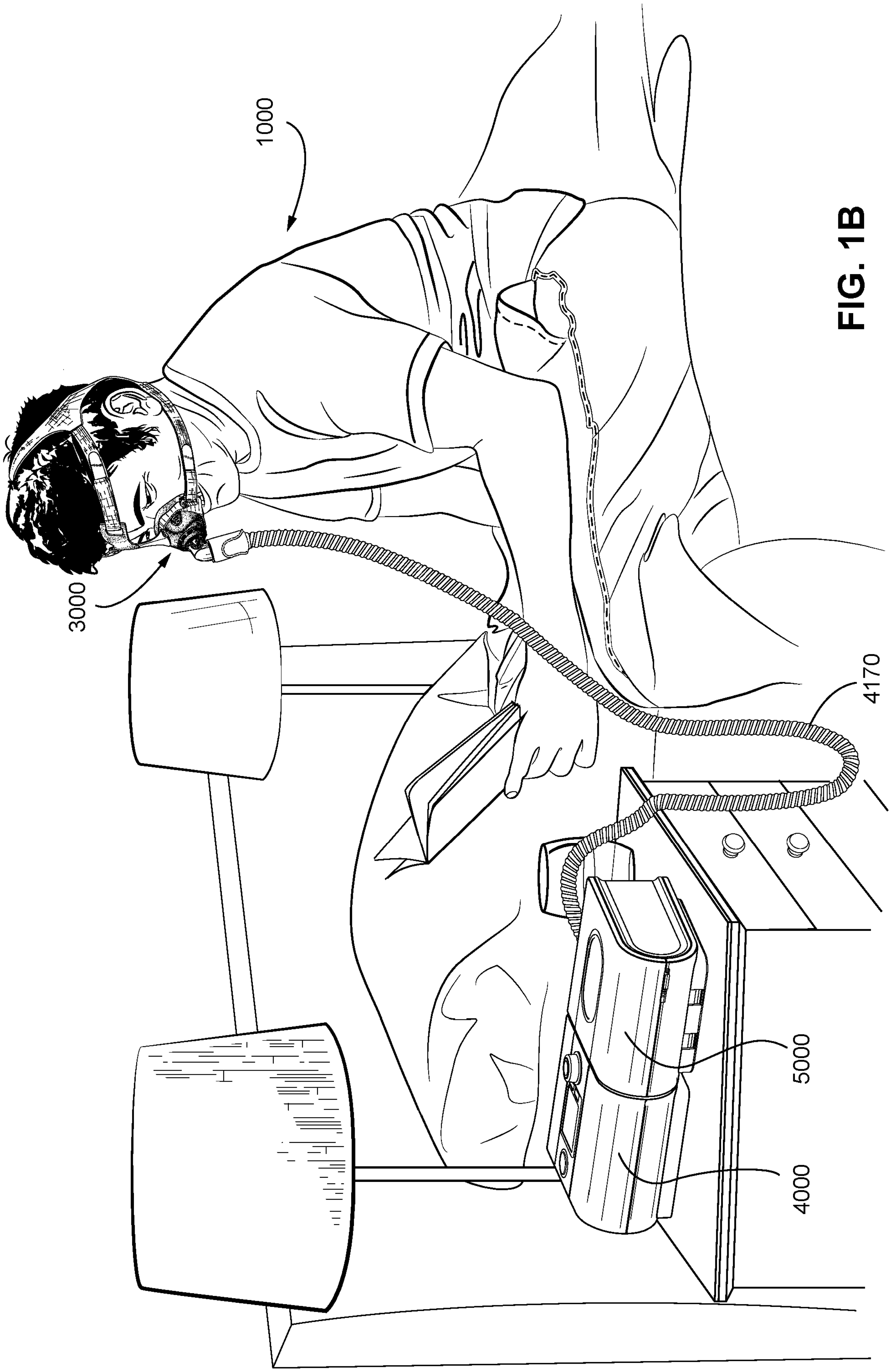


FIG. 1B

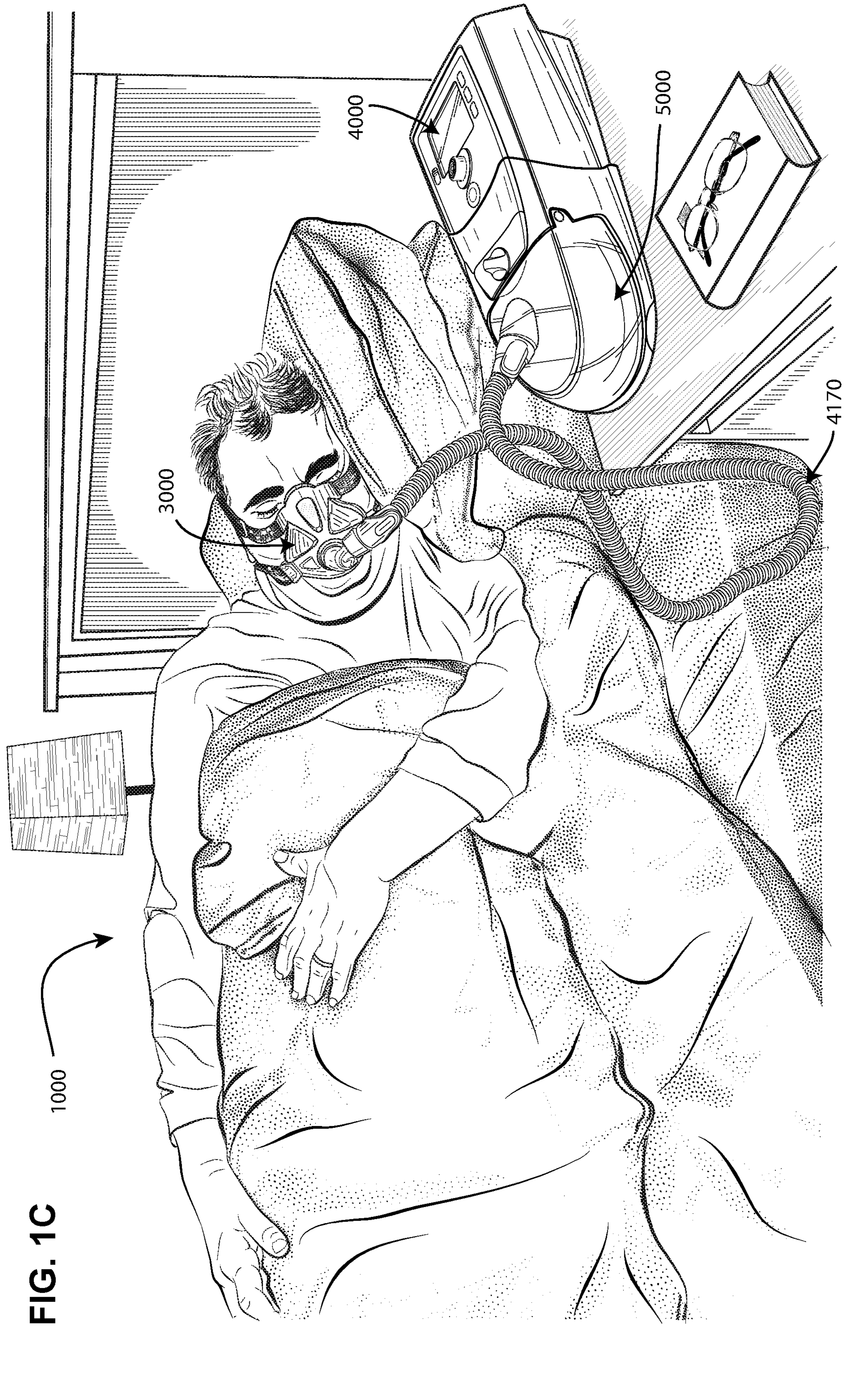


FIG. 1C

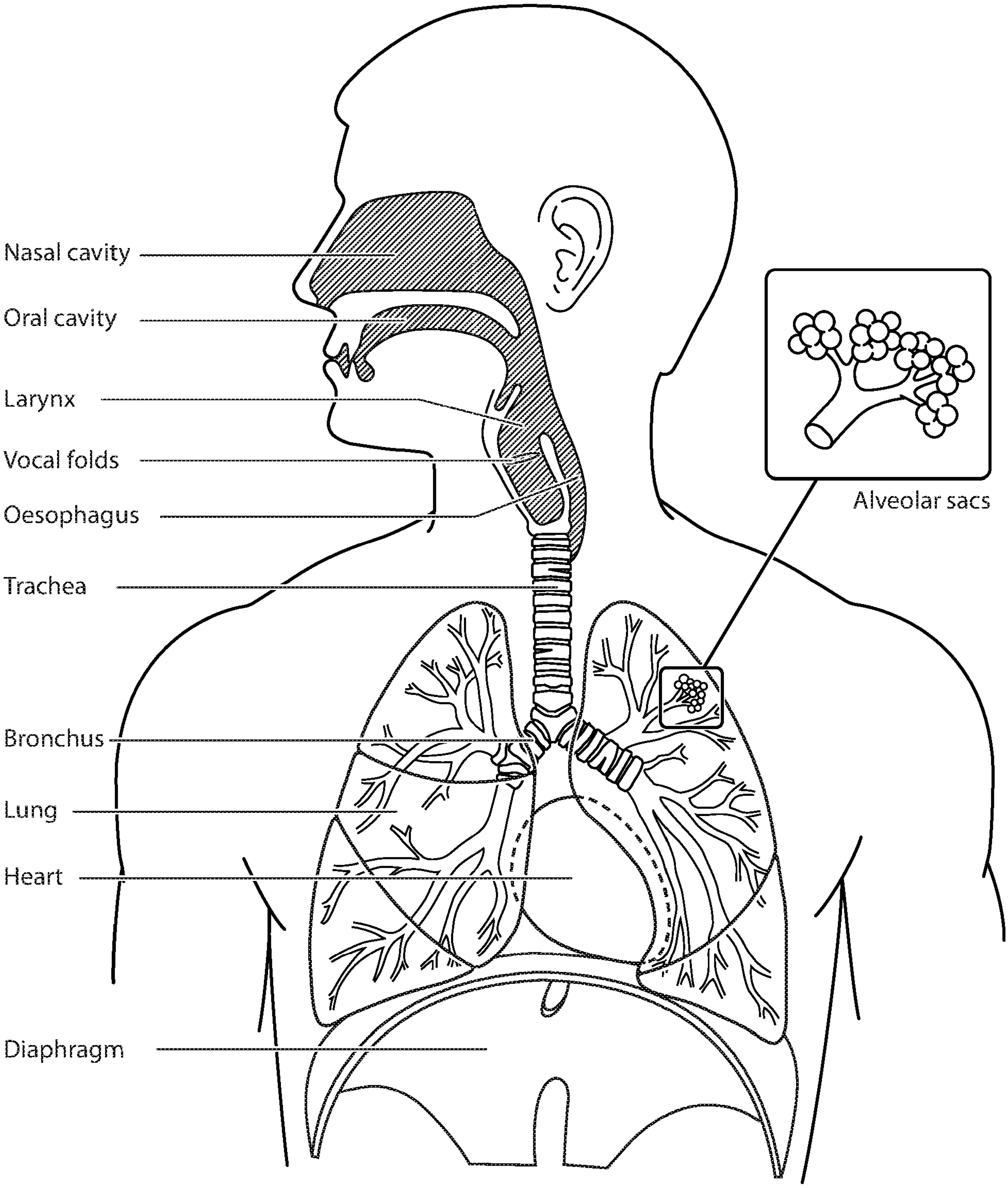
Nasal cavity
Oral cavity
Larynx
Vocal folds
Oesophagus
Trachea
Bronchus
Lung
Heart
Diaphragm
Alveolar sacs

FIG. 2A

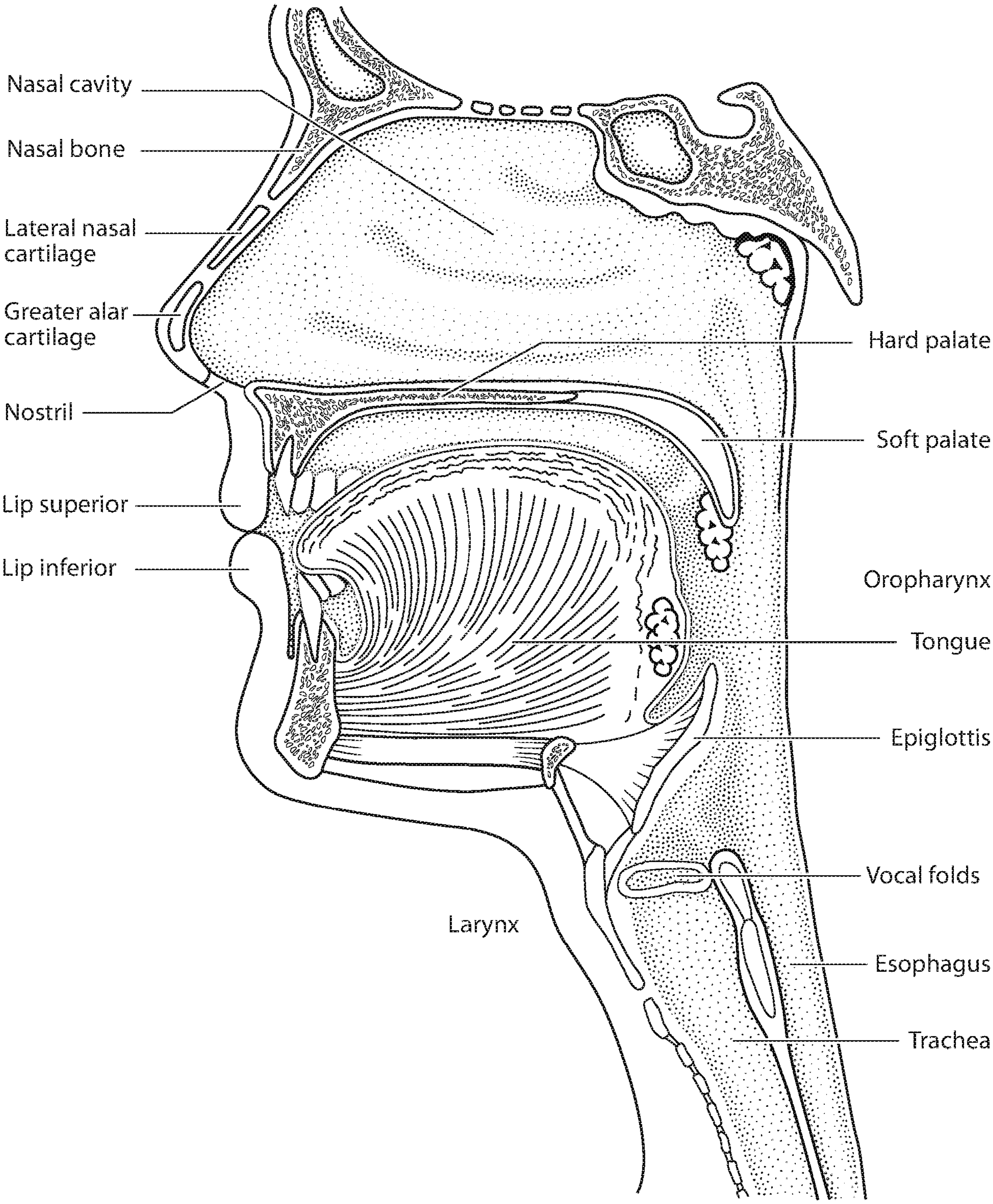
Nasal cavity
Nasal bone
Lateral nasal cartilage
Greater alar cartilage
Nostril
Lip superior
Lip inferior
Hard palate
Soft palate
Oropharynx
Tongue
Epiglottis
Vocal folds
Esophagus
Trachea
Larynx

FIG. 2B

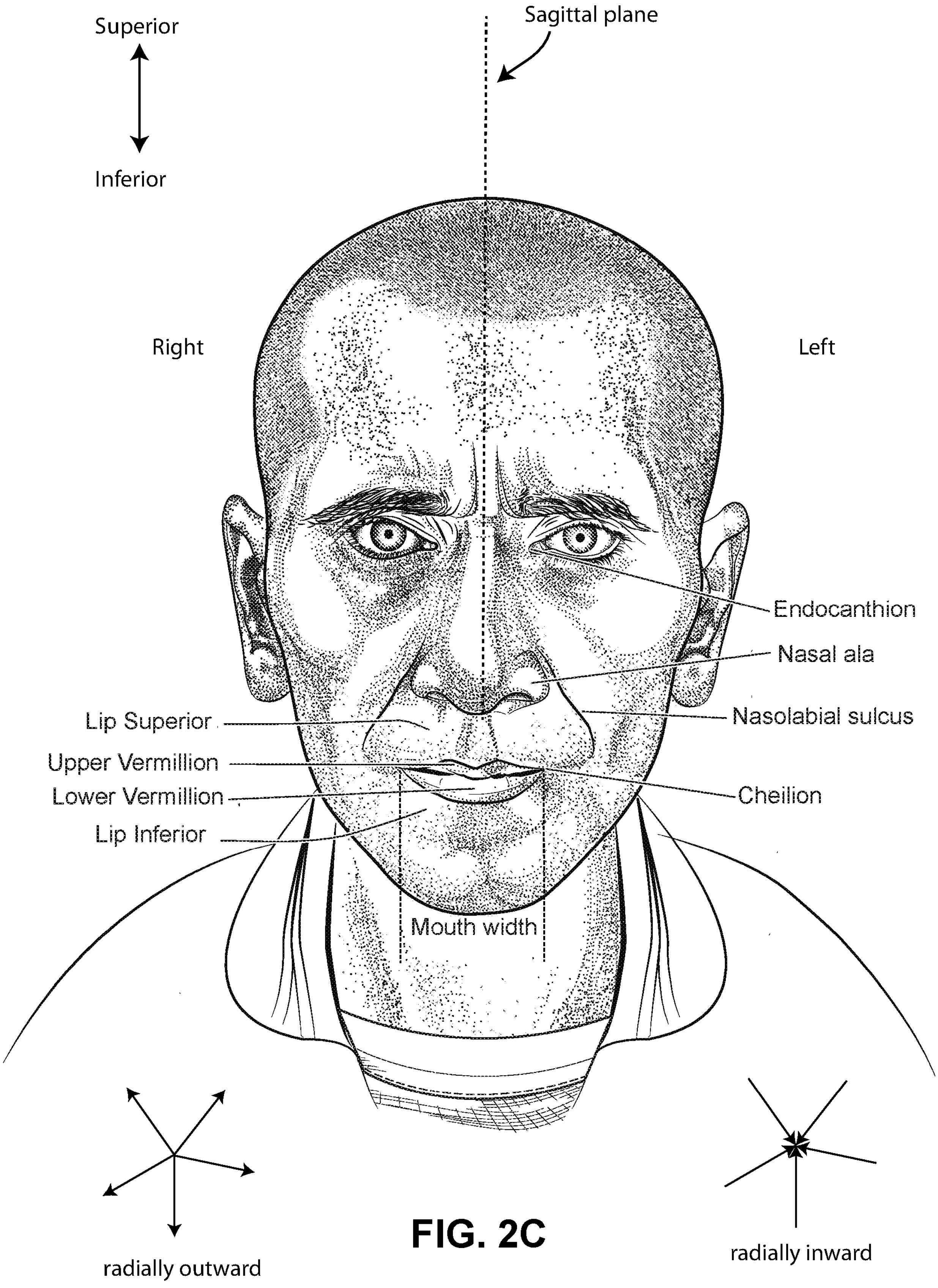
Superior
Inferior
Sagittal plane
Right
Left
Endocanthion
Nasal ala
Nasolabial sulcus
Lip Superior
Upper Vermillion
Lower Vermillion
Lip Inferior
Cheilion
Mouth width
radially outward
radially inward

FIG. 2C

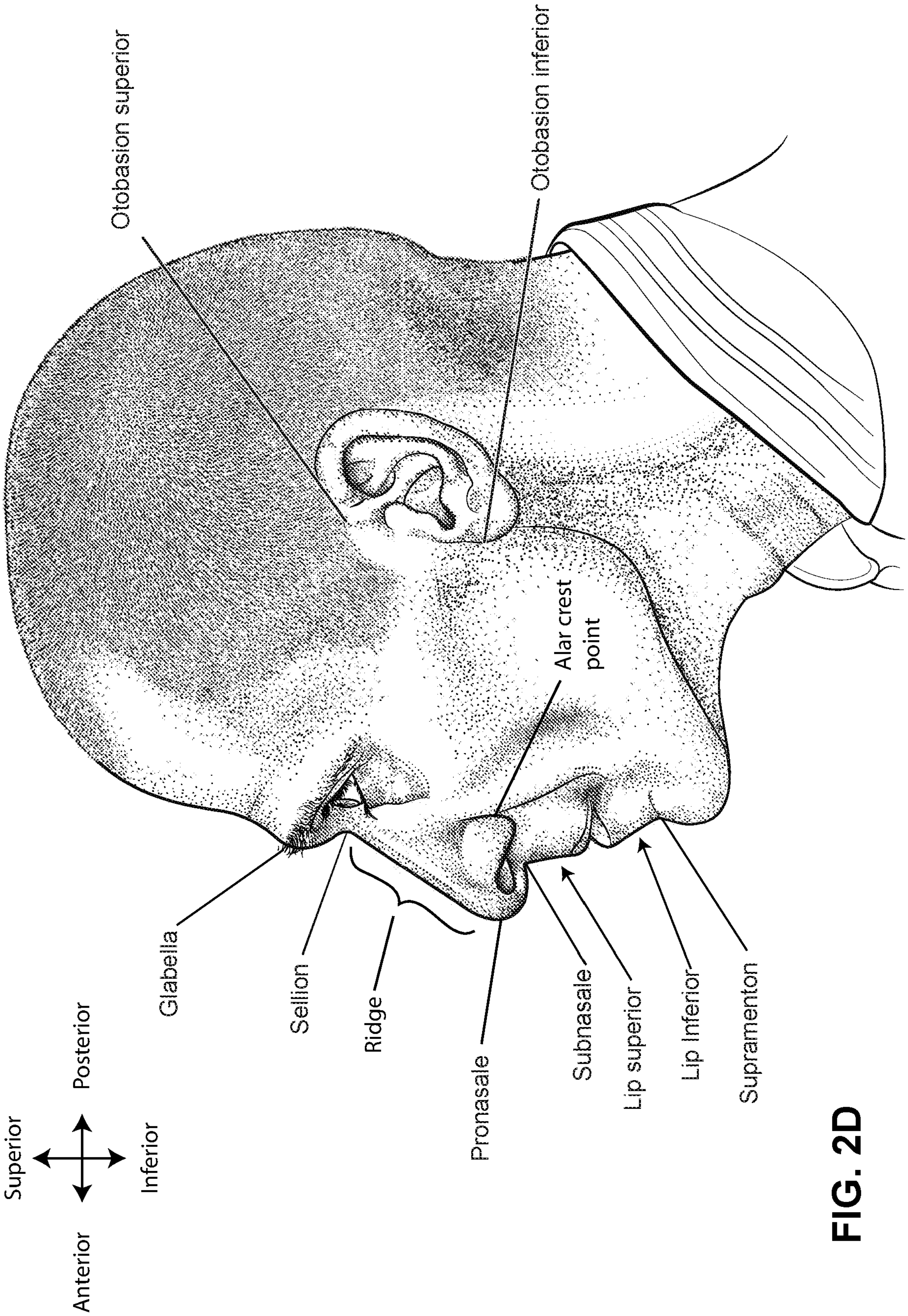
Otobasion superior
Otobasion inferior
Alar crest point
Glabella
Sellion
Ridge
Pronasale
Subnasale
Lip superior
Lip Inferior
Supramenton
Superior
Inferior
Anterior
Posterior

FIG. 2D

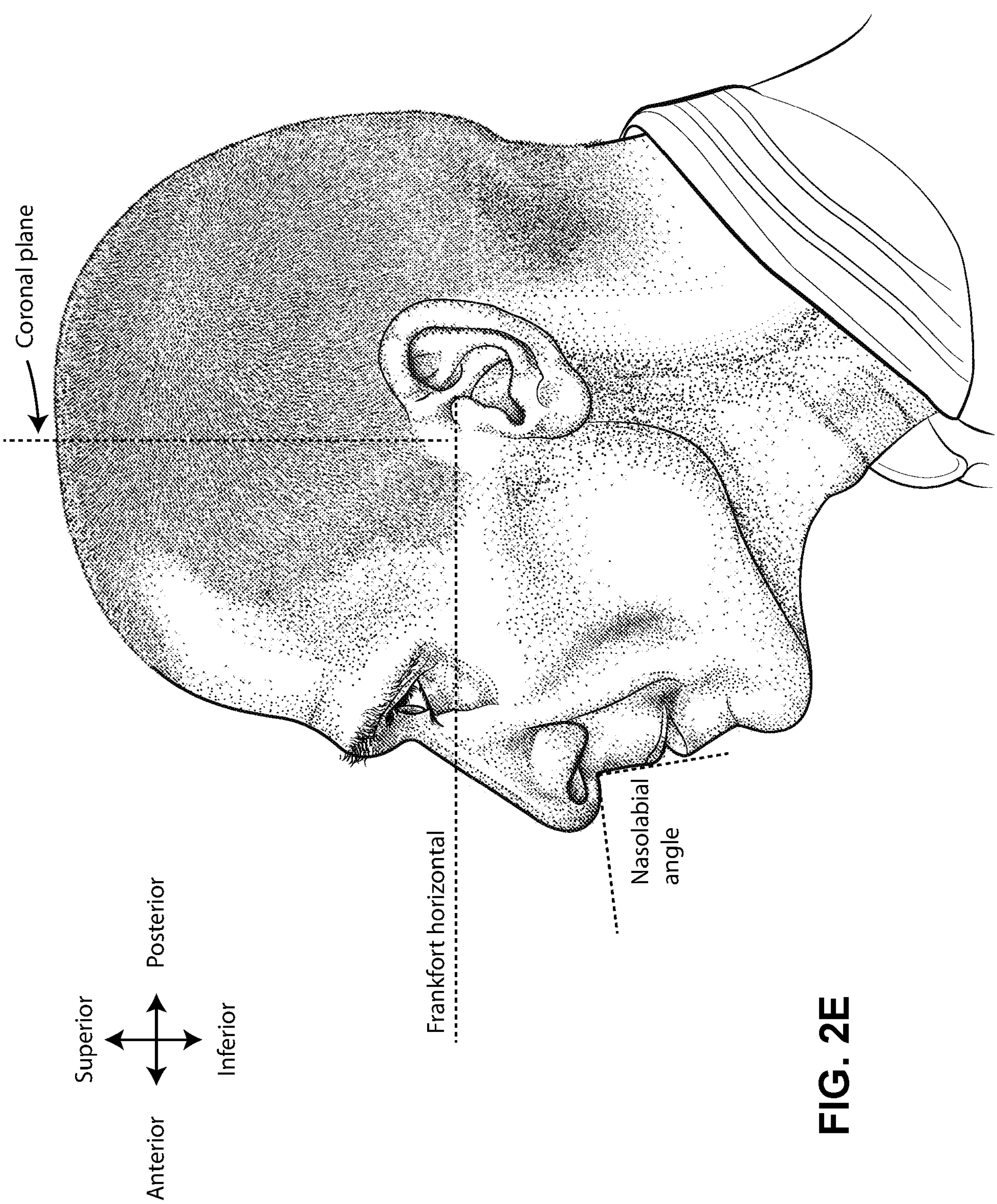
Coronal plane
Frankfort horizontal
Nasolabial angle
Superior
Inferior
Anterior
Posterior

FIG. 2E

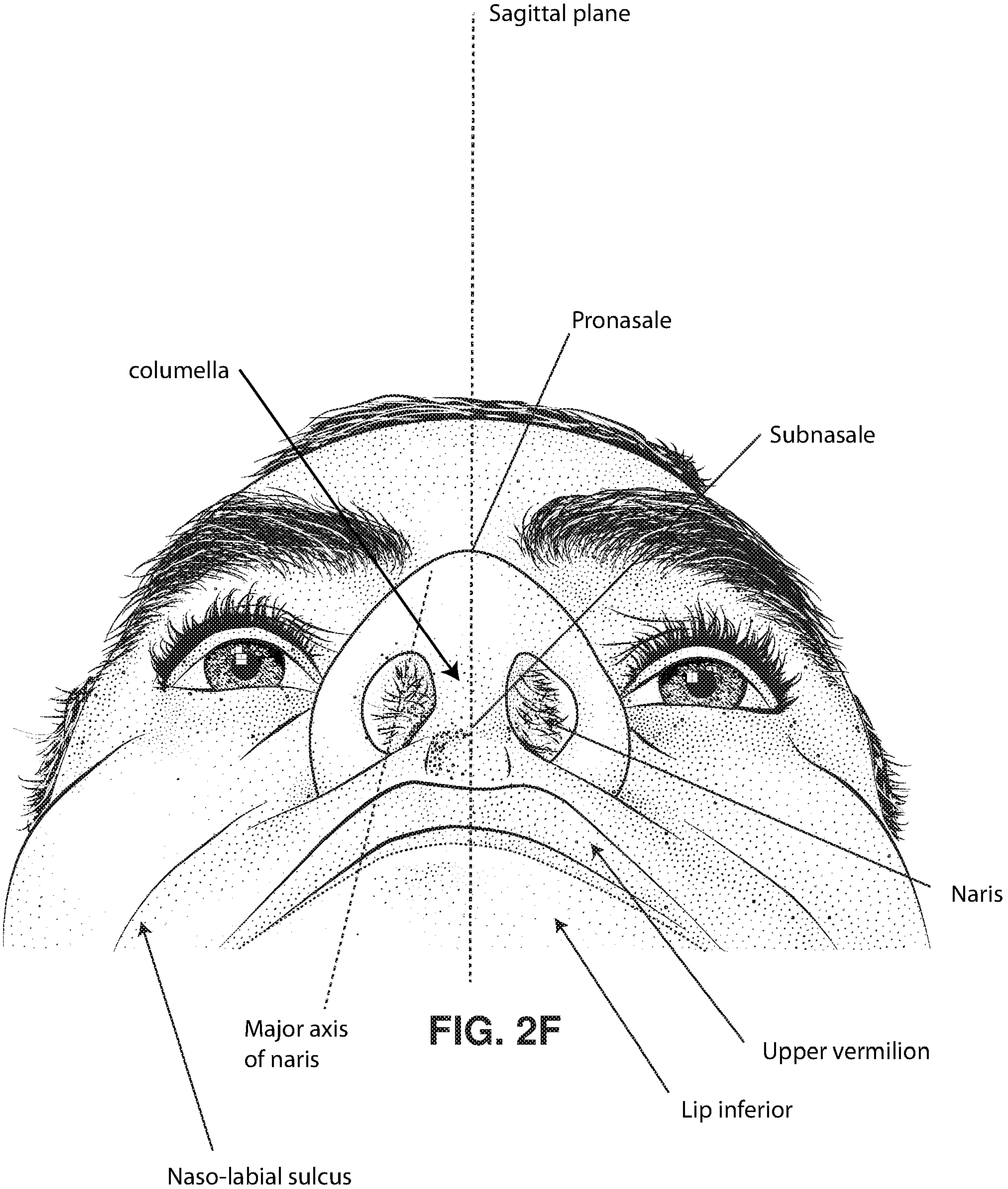
Sagittal plane
Pronasale
columella
Subnasale
Naris
Major axis
of naris
Upper vermilion
Lip inferior
Naso-labial sulcus

FIG. 2F

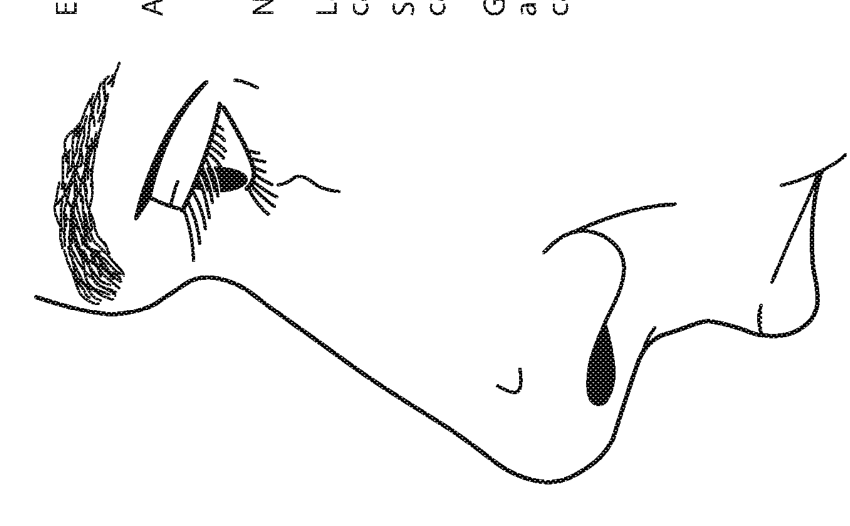

FIG. 2G

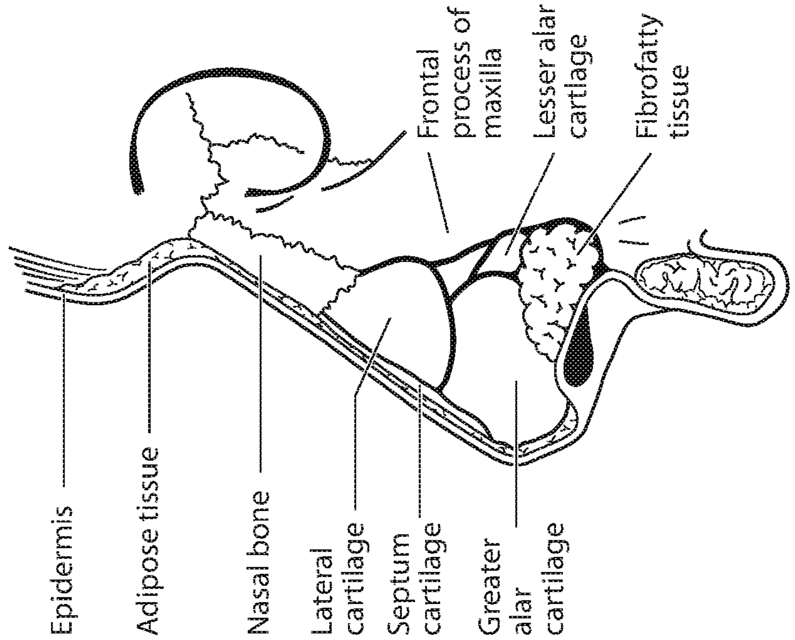


FIG. 2H

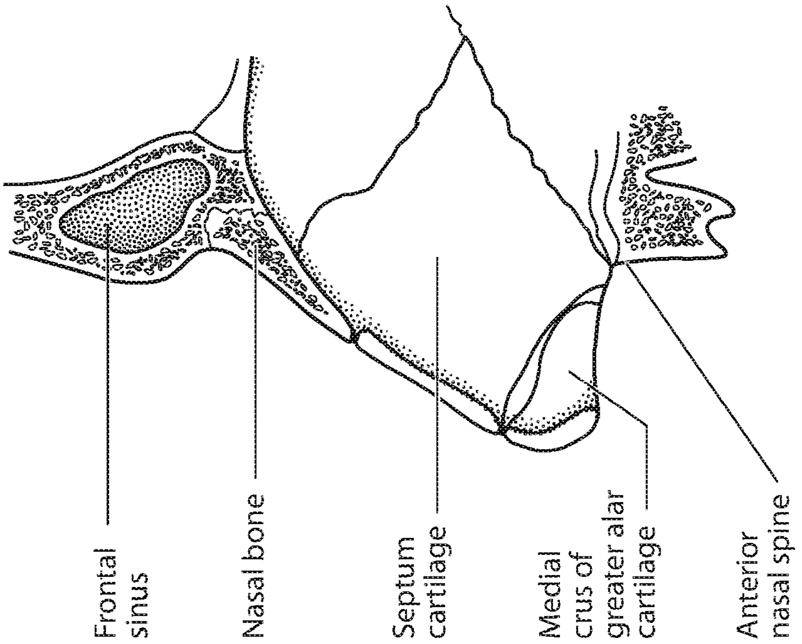


FIG. 2I

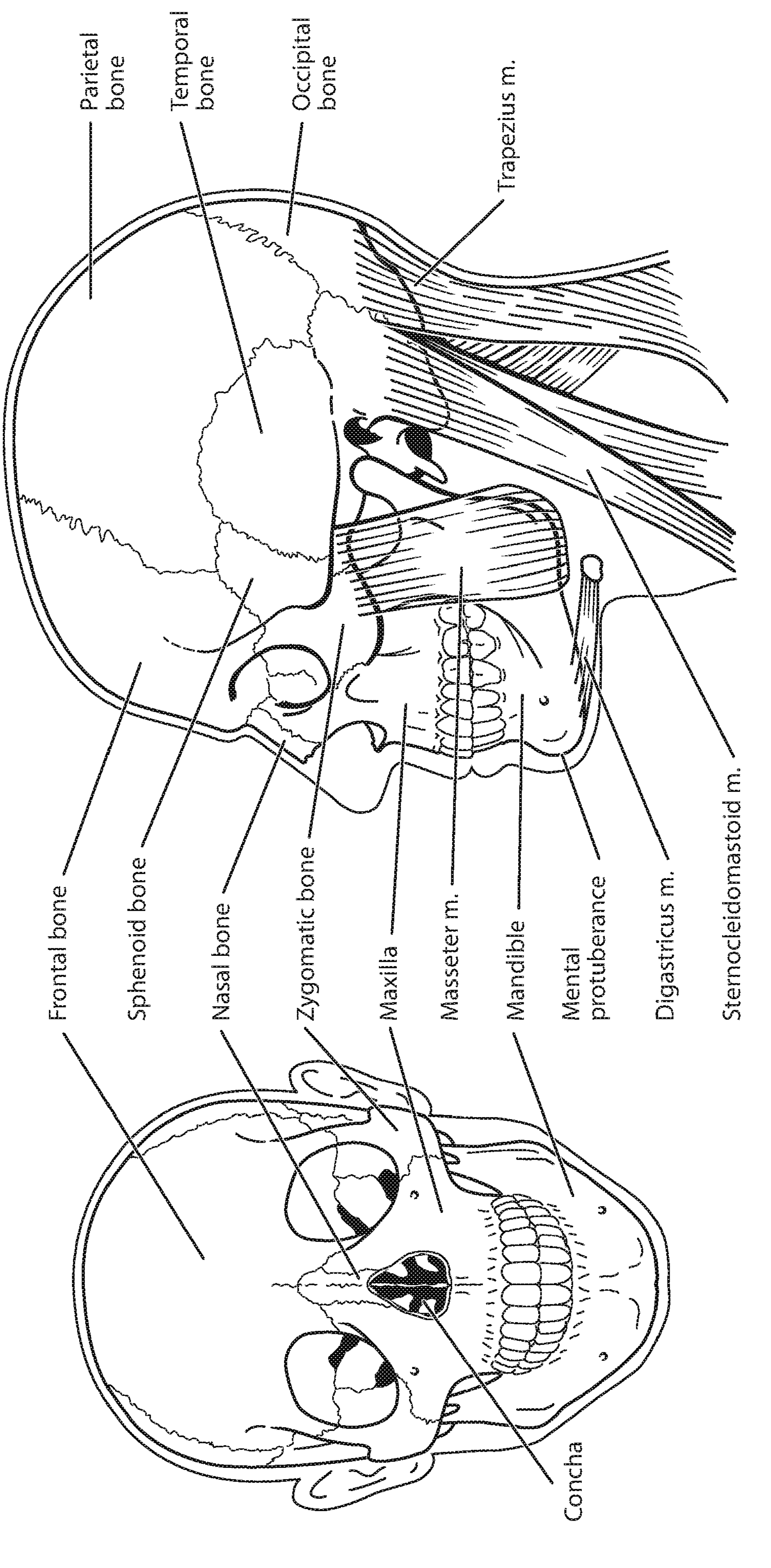
Parietal bone
Temporal bone
Occipital bone
Trapezius m.
Frontal bone
Sphenoid bone
Nasal bone
Zygomatic bone
Maxilla
Masseter m.
Mandible
Mental protuberance
Digastricus m.
Sternocleidomastoid m.
Concha

FIG. 2K

FIG. 2J

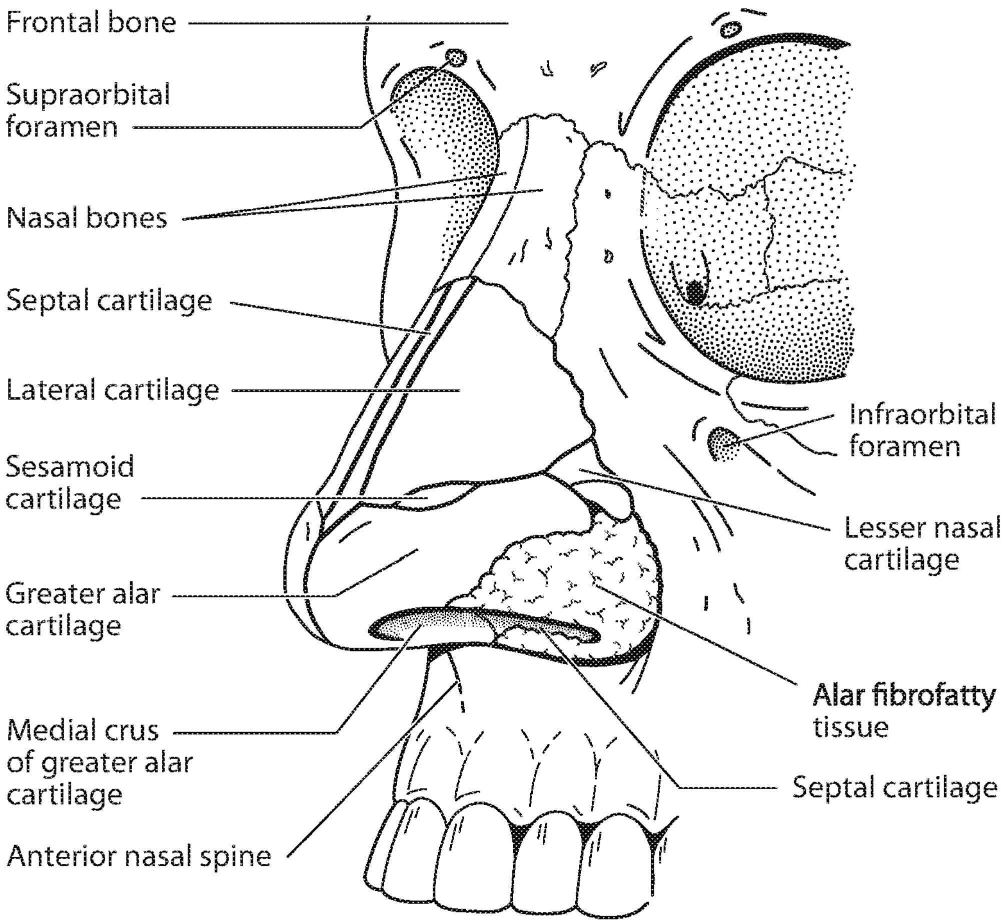
Frontal bone
Supraorbital foramen
Nasal bones
Septal cartilage
Lateral cartilage
Sesamoid cartilage
Greater alar cartilage
Medial crus of greater alar cartilage
Anterior nasal spine
Infraorbital foramen
Lesser nasal cartilage
Alar fibrofatty tissue
Septal cartilage

FIG. 2L

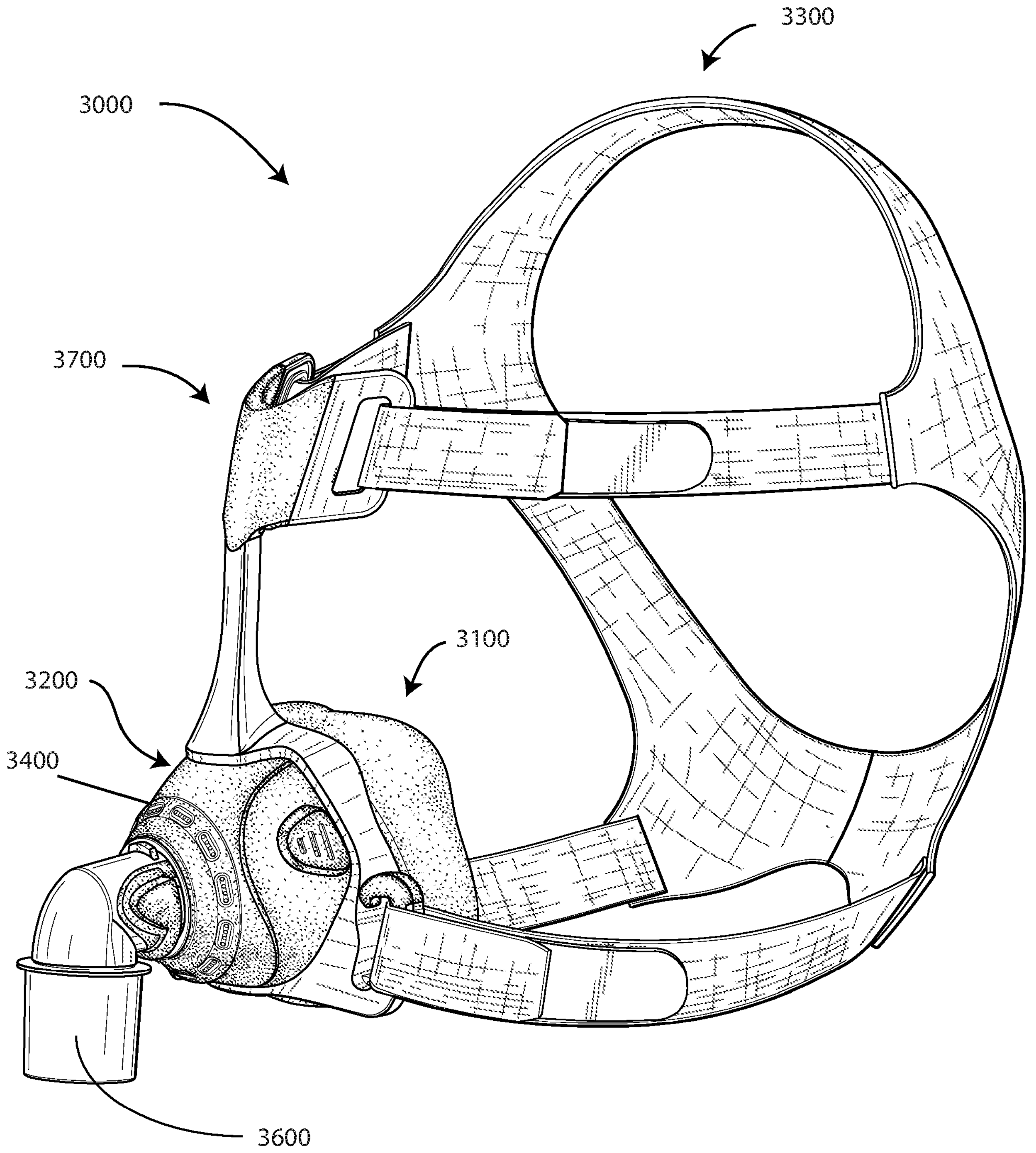


FIG. 3A

FIG. 3B

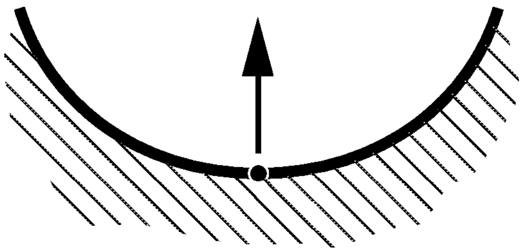

Relatively Large
Positive Curvature

FIG. 3C

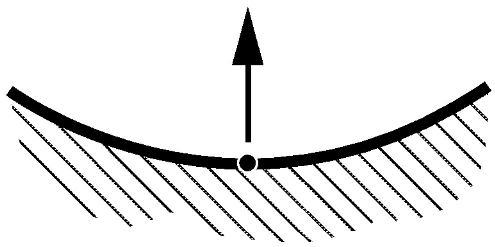

Relatively Small
Positive Curvature

FIG. 3D

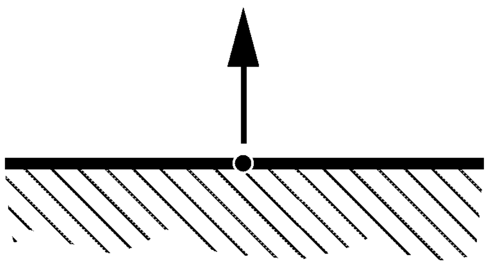

Zero Curvature

FIG. 3E

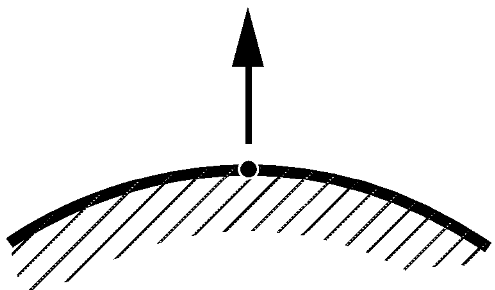

Relatively Small
Negative Curvature

FIG. 3F

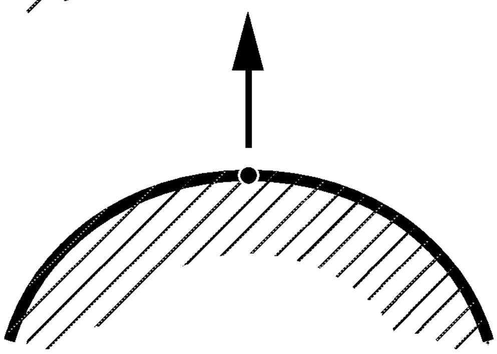

Relatively Large
Negative Curvature

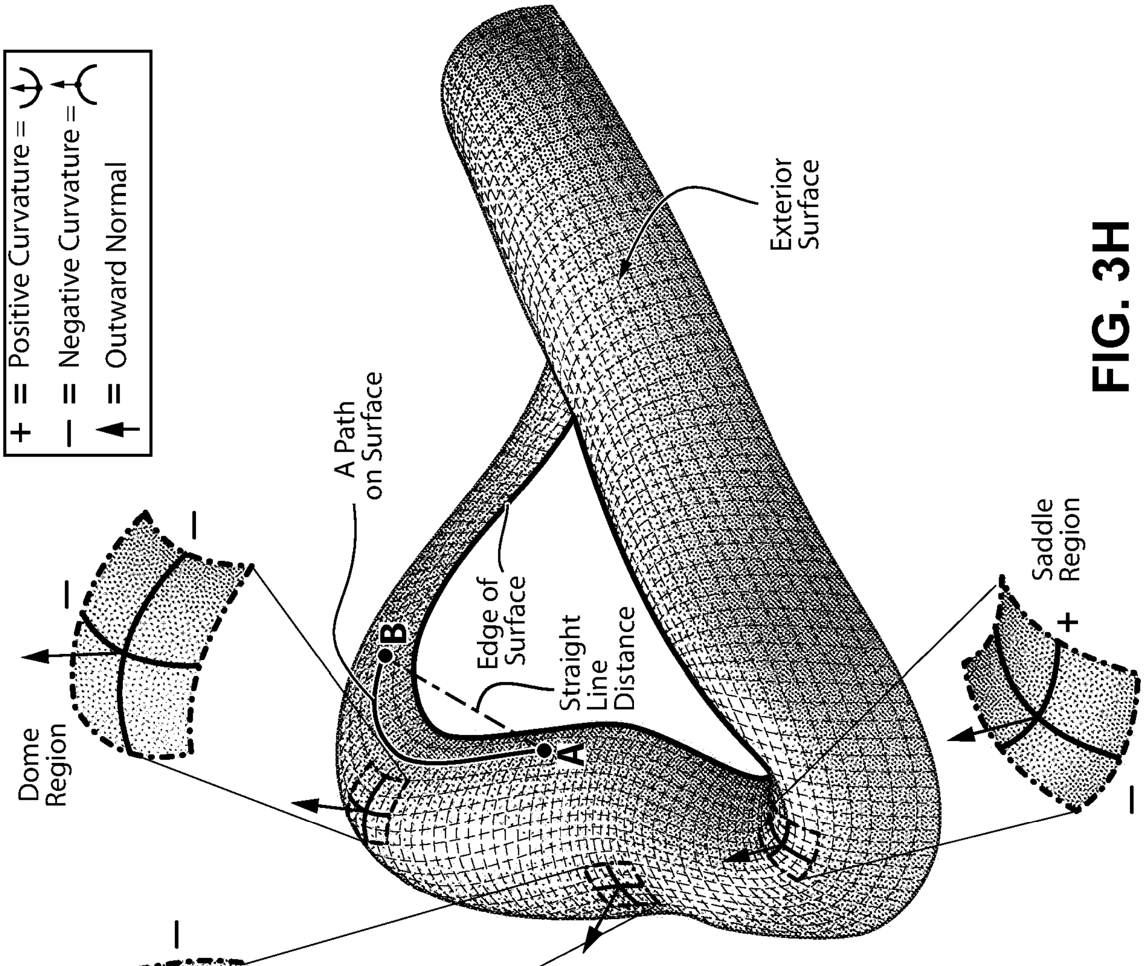
+ = Positive Curvature =
− = Negative Curvature =
= Outward Normal
A Path on Surface
Edge of Surface
Straight Line Distance
Dome Region
Saddle Region
Exterior Surface
A
B
+
−

FIG. 3H

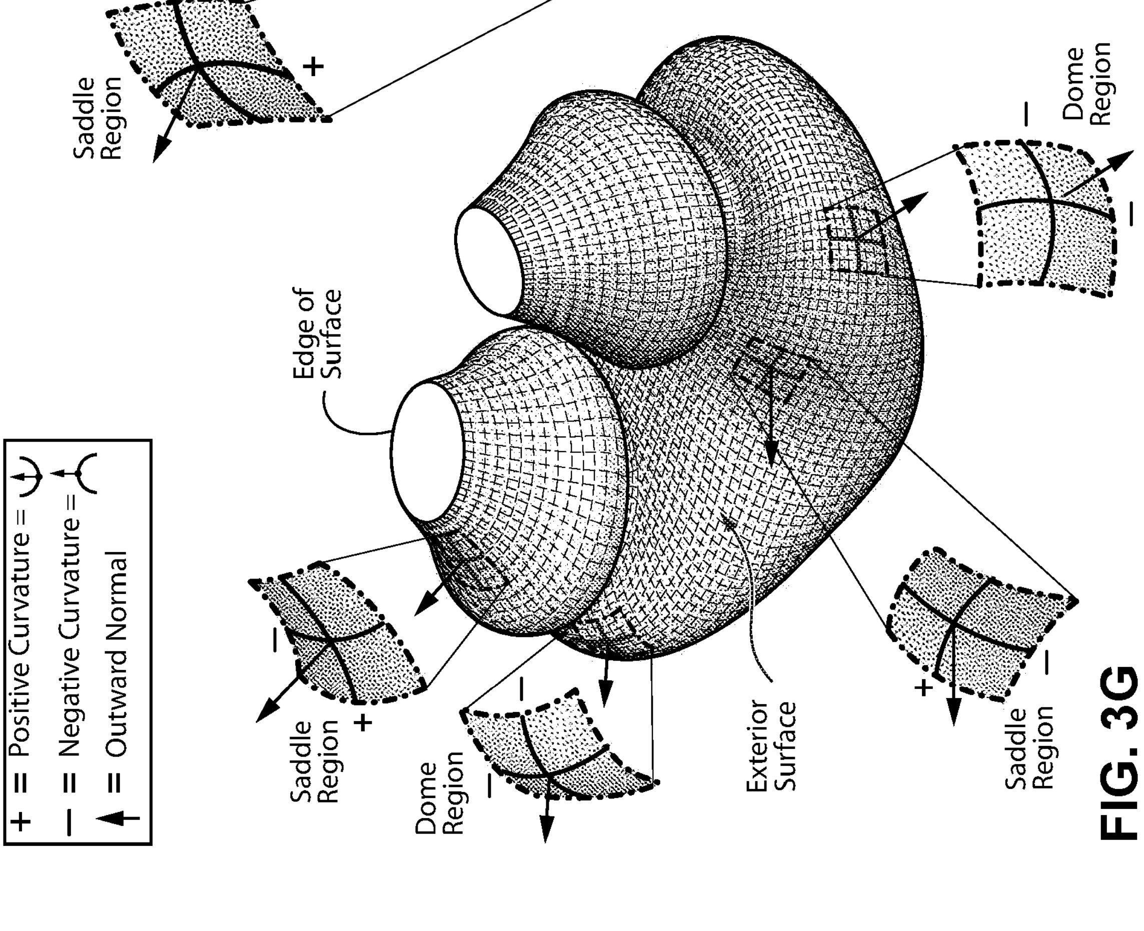
+ = Positive Curvature =
− = Negative Curvature =
= Outward Normal
Edge of Surface
Saddle Region
Dome Region
Exterior Surface
+
−

FIG. 3G

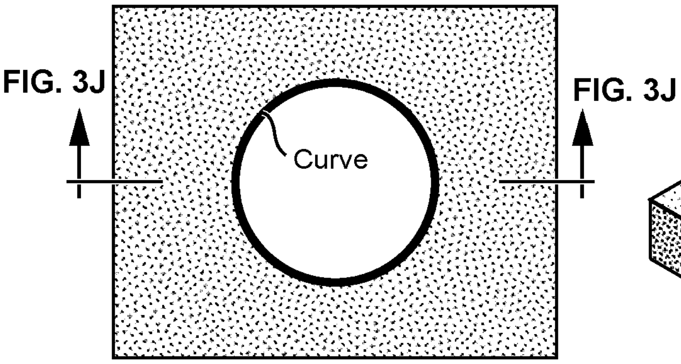


FIG. 3I

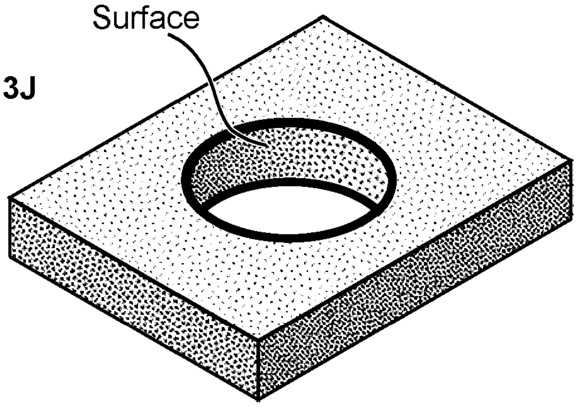


FIG. 3K

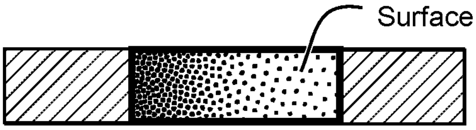


FIG. 3J

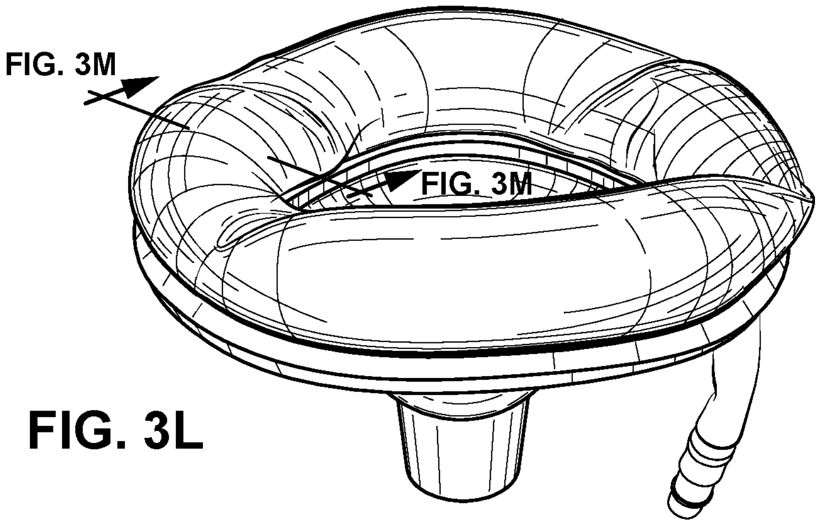


FIG. 3L

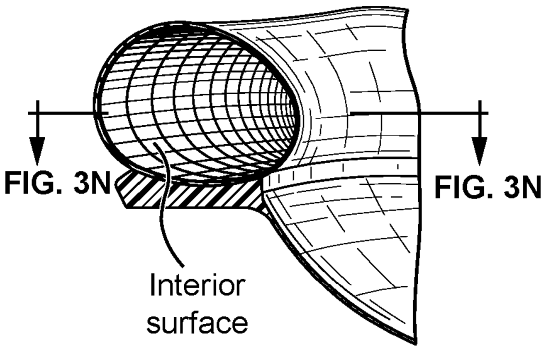


FIG. 3M

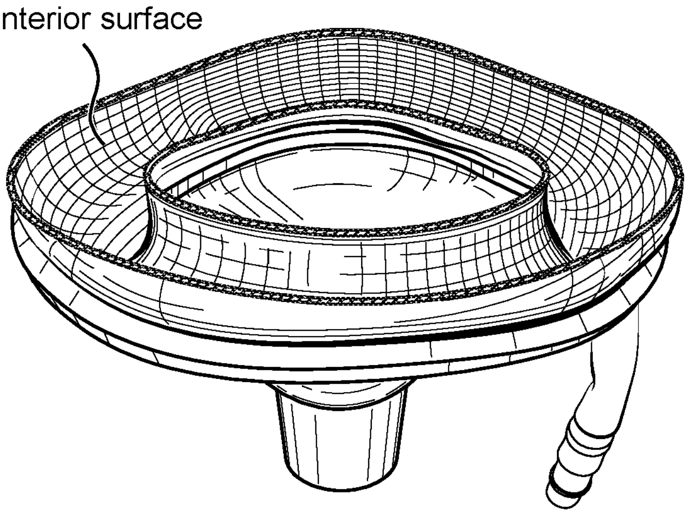


FIG. 3N

**Left-hand rule**

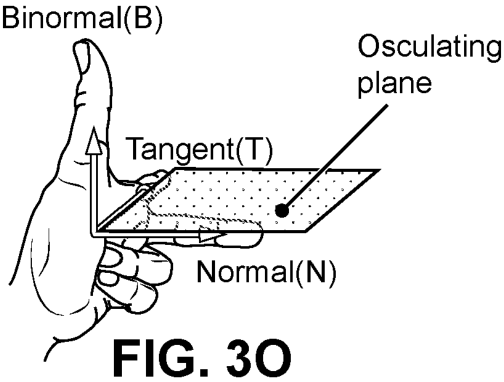


**FIG. 3O**

**Right-hand rule**

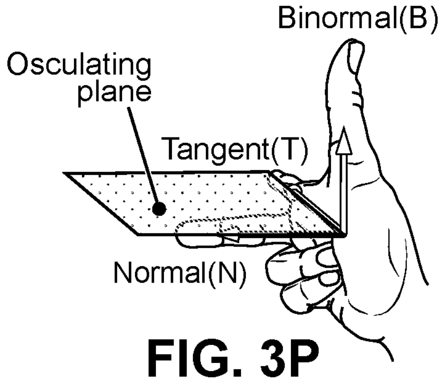


**FIG. 3P**

**Left ear helix**

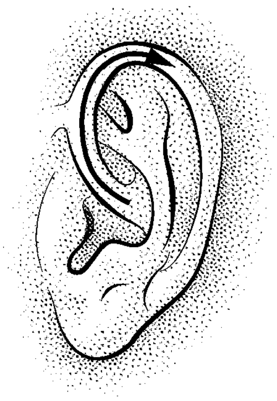

**FIG. 3Q**

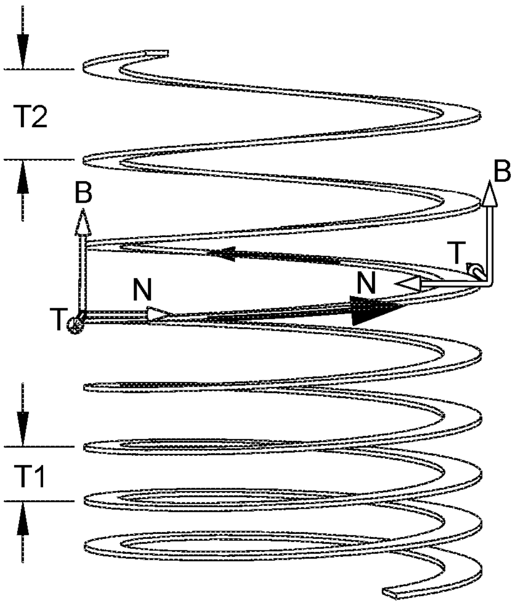


**Right-hand helix**
**Right-hand positive**

**FIG. 3S**

**Right ear helix**

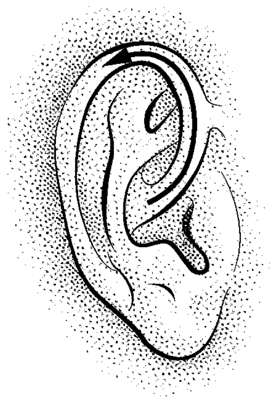

**FIG. 3R**

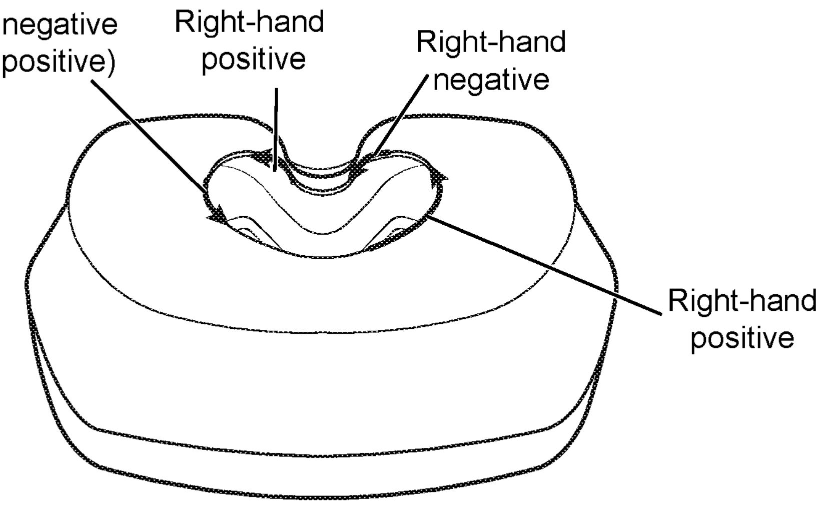


**FIG. 3T**

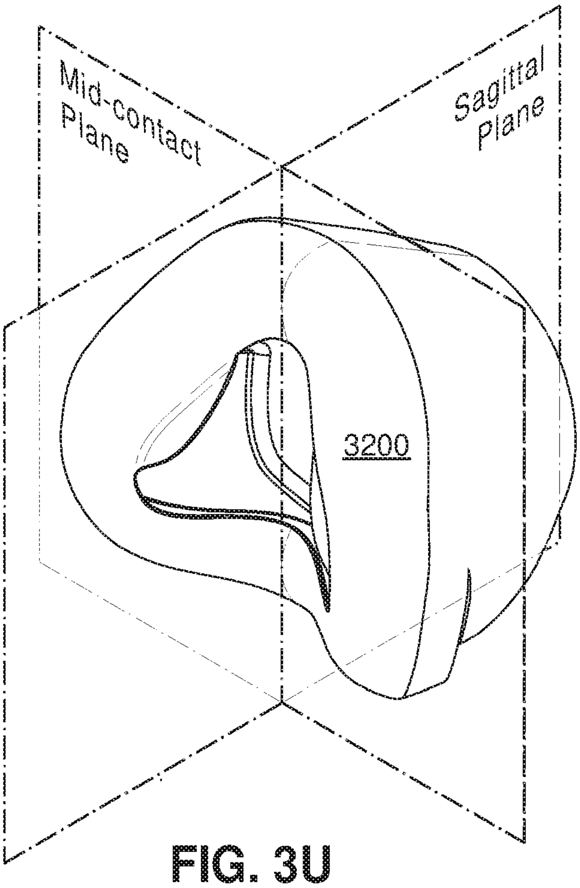


FIG. 3U

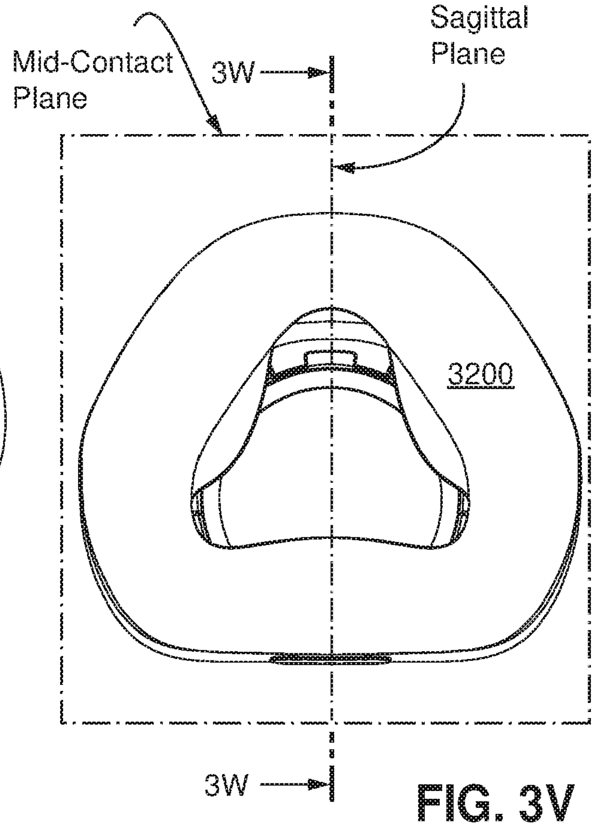


FIG. 3V

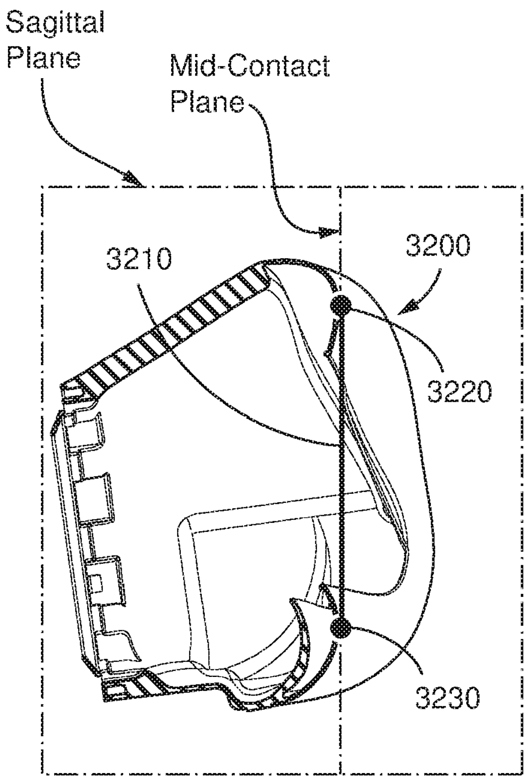


FIG. 3W

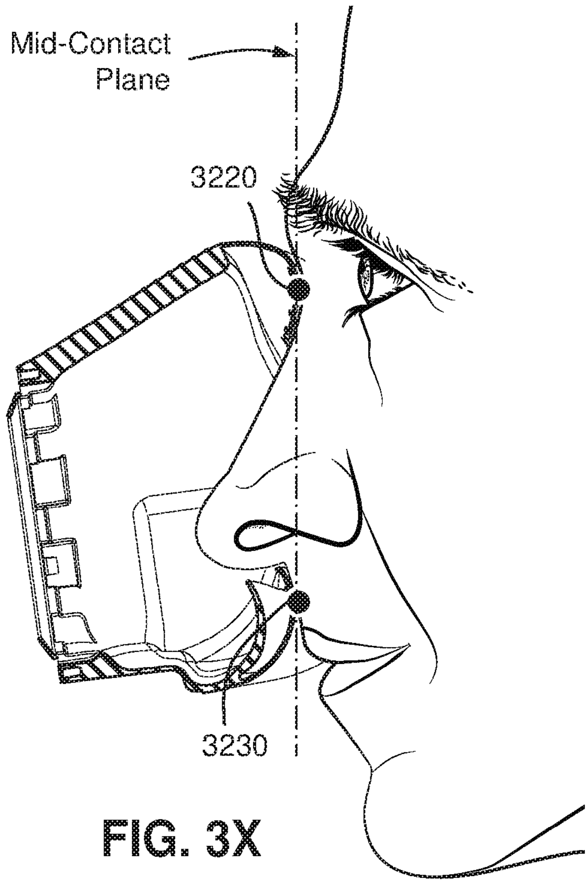


FIG. 3X

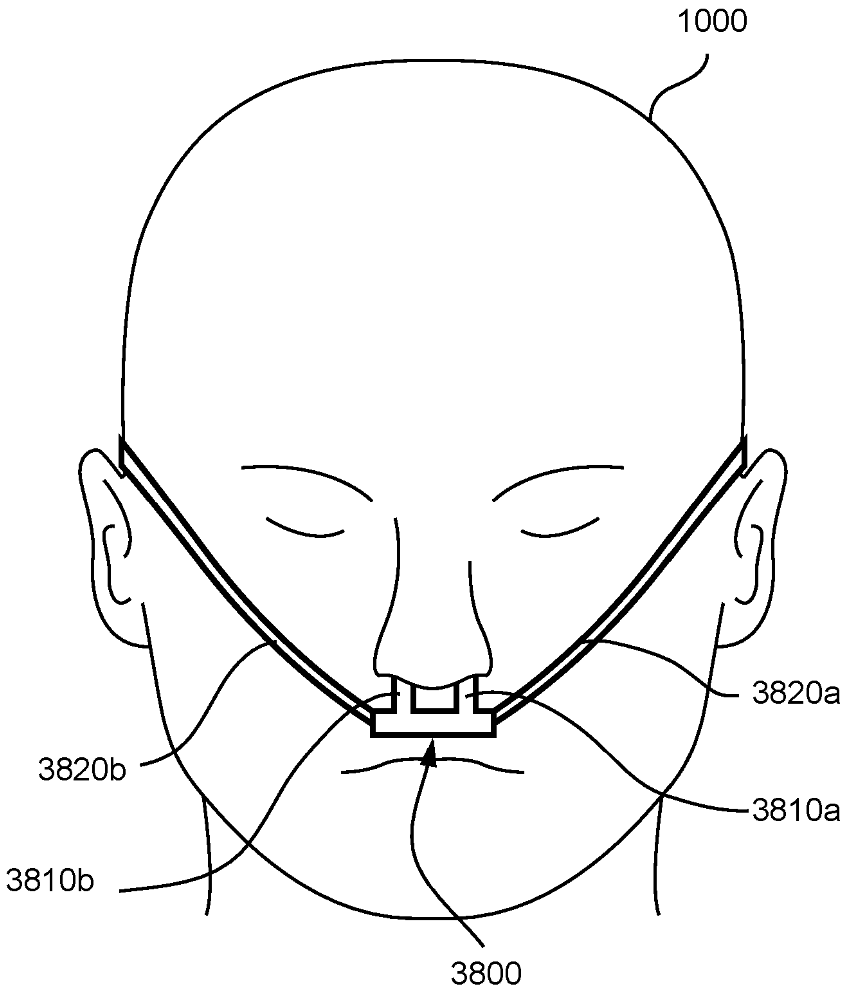


FIG. 3Y

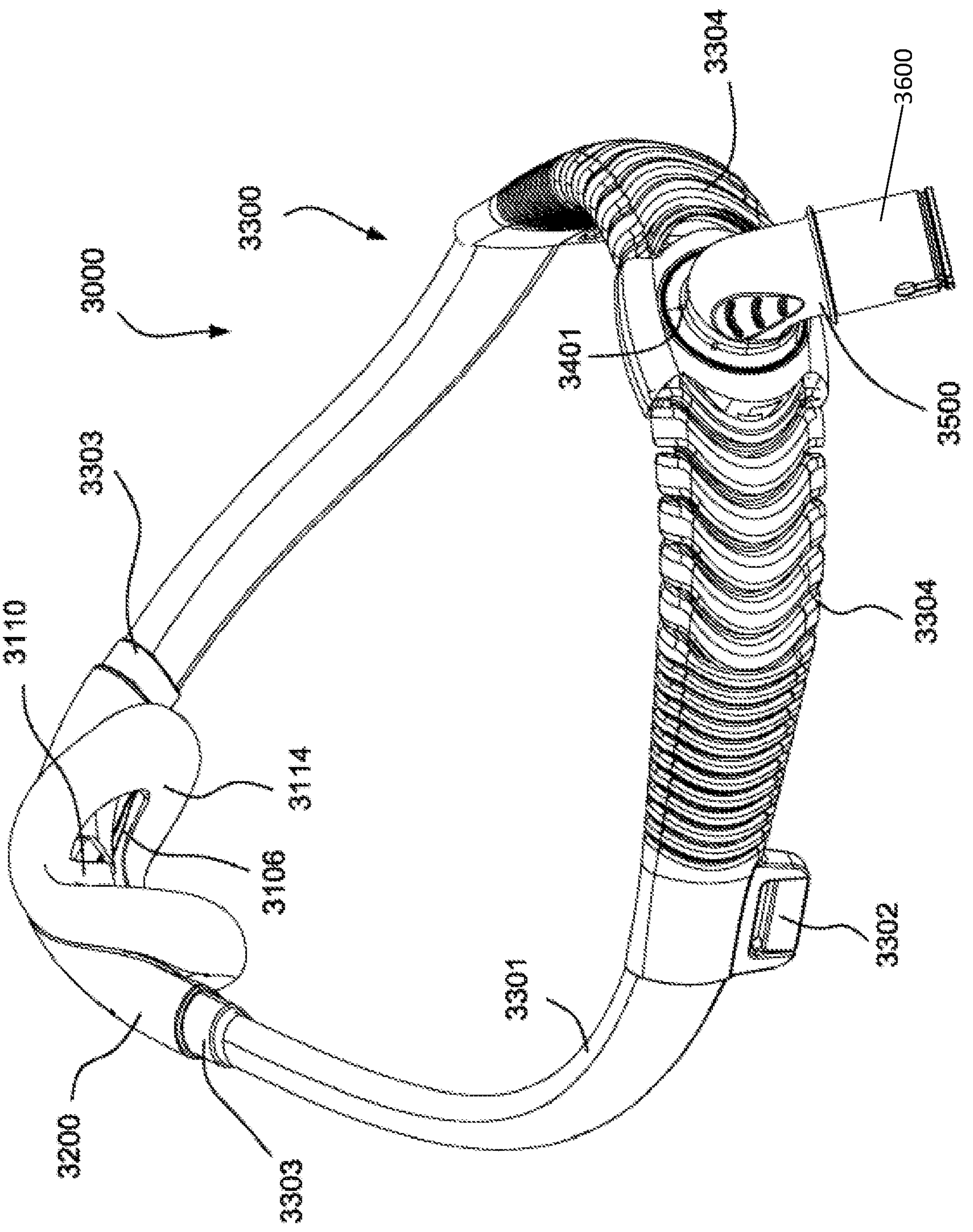
3000
3300
3304
3600
3401
3500
3303
3110
3114
3106
3304
3302
3301
3200
3303

FIG. 4

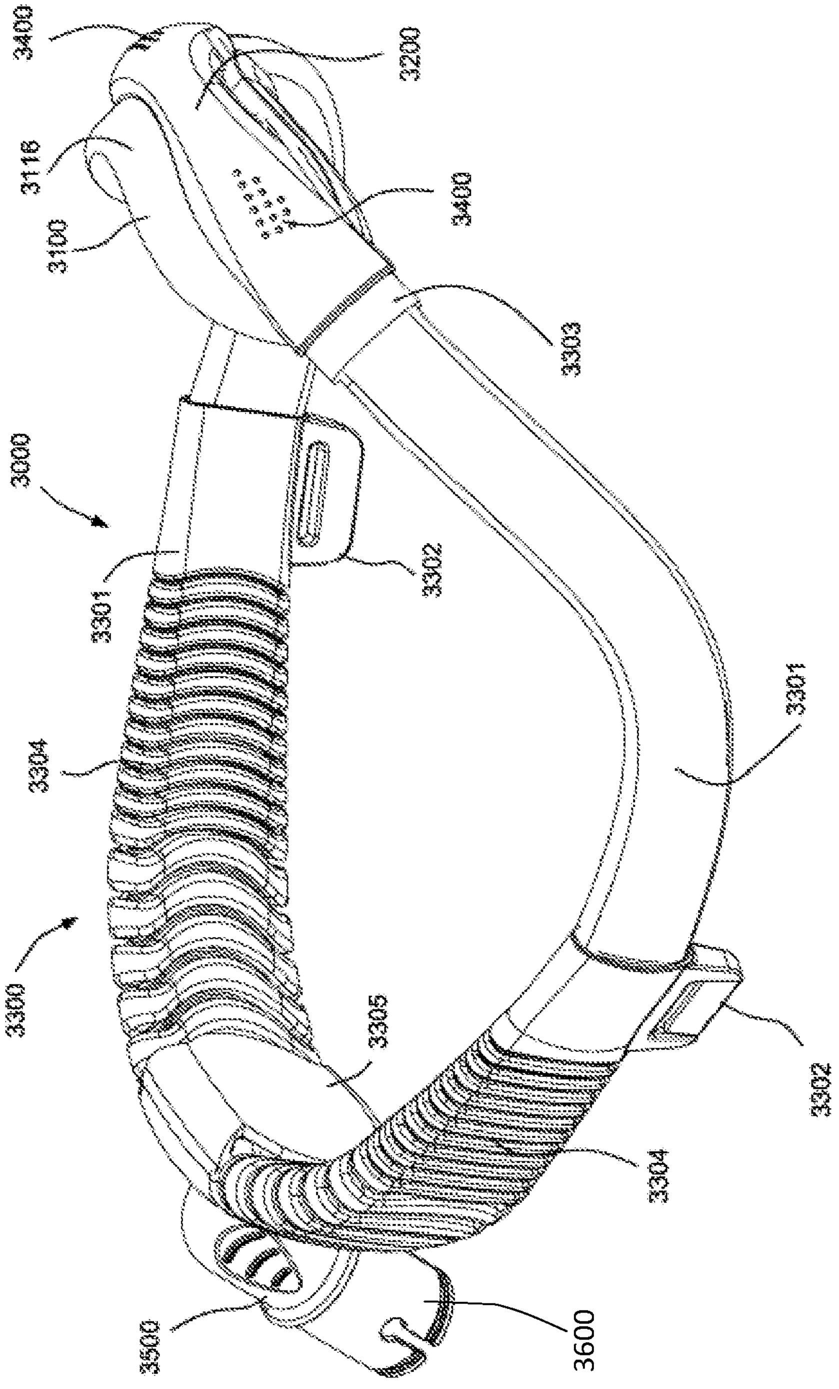
3400
3200
3116
3400
3100
3303
3000
3302
3301
3301
3304
3300
3305
3302
3304
3500
3600

FIG. 5

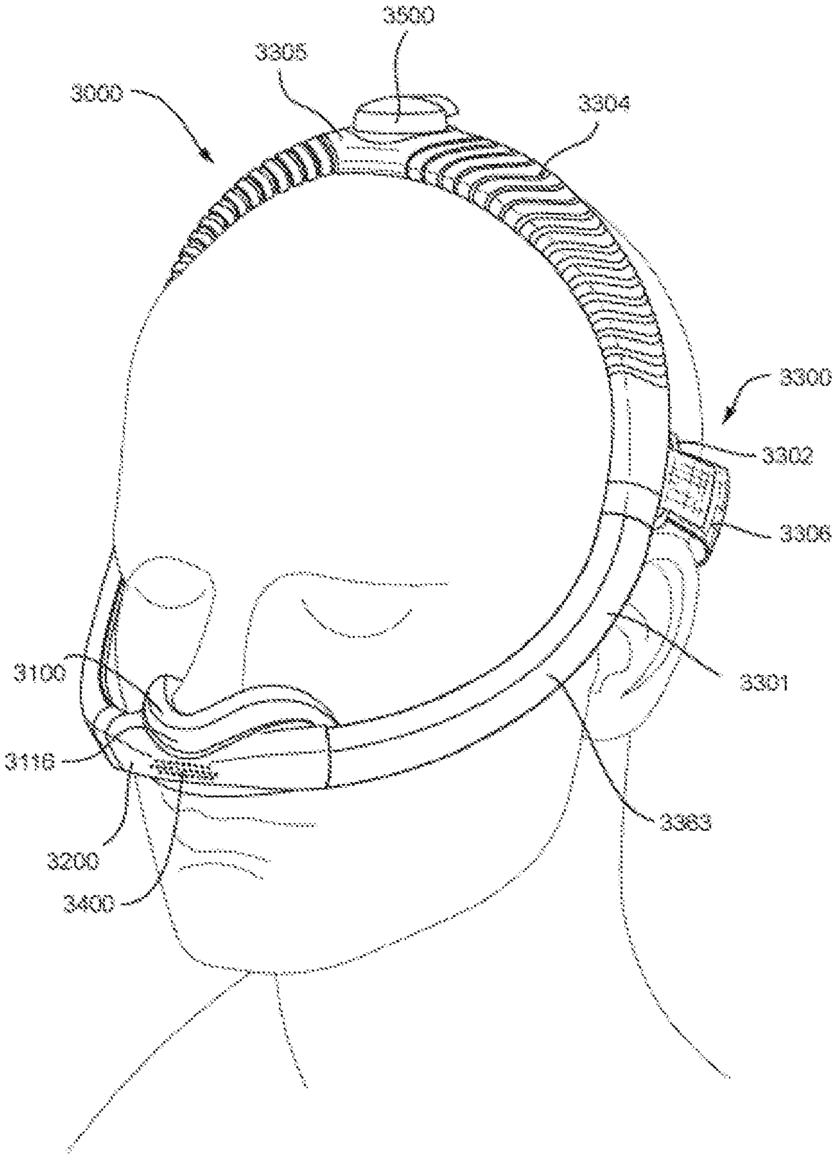
3500
3305
3000
3304
3300
3302
3306
3301
3100
3116
3200
3400
3363

FIG. 6A

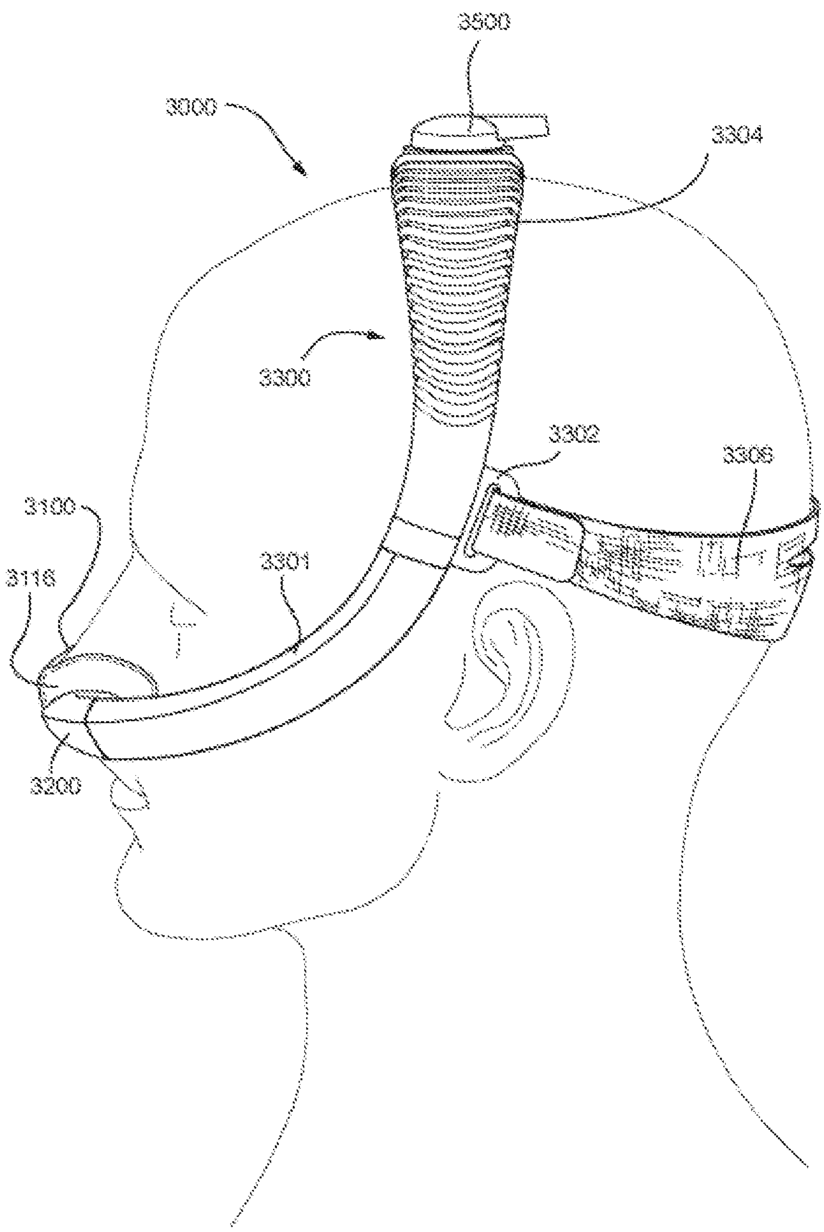


FIG. 6B

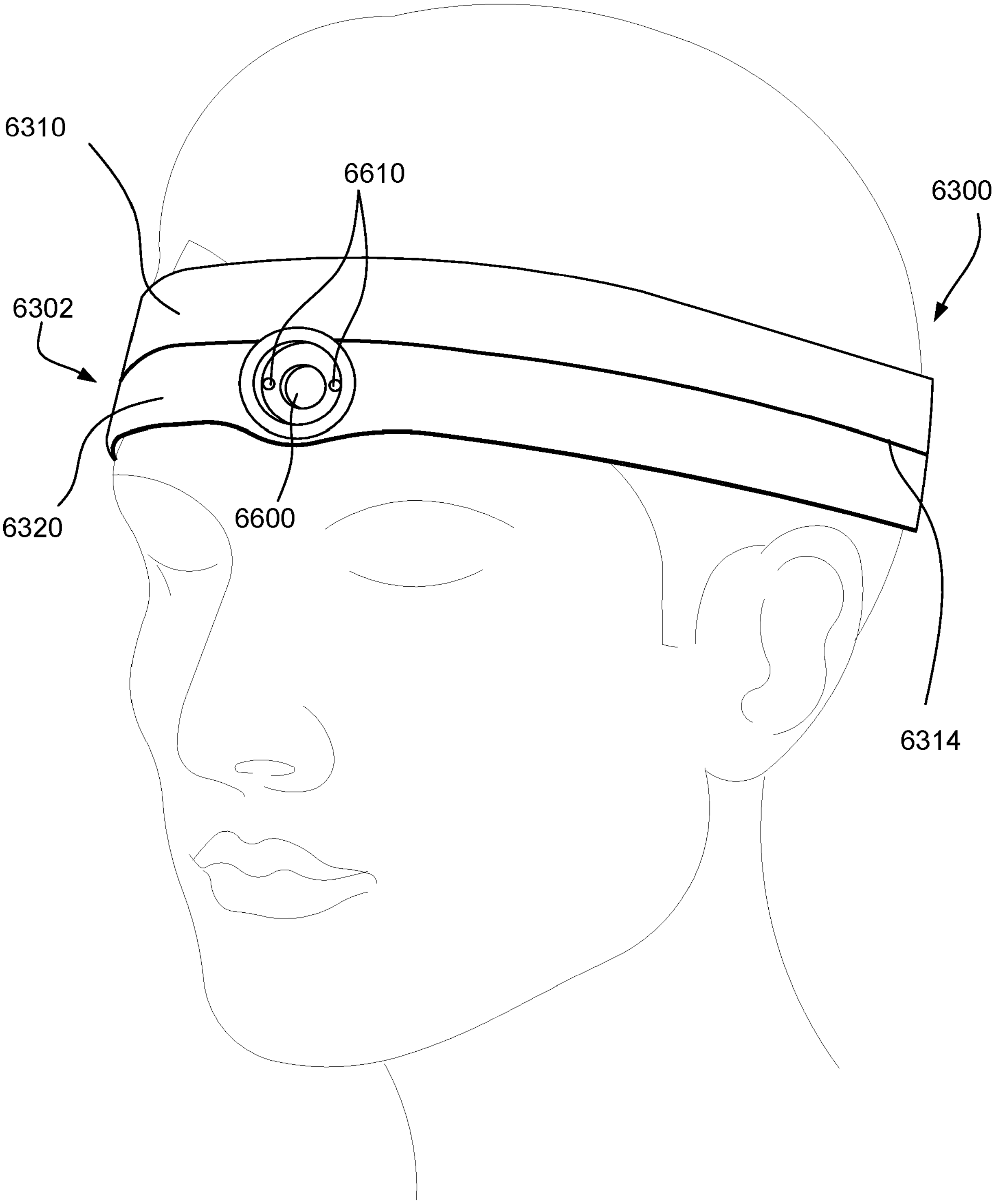


FIG 7A

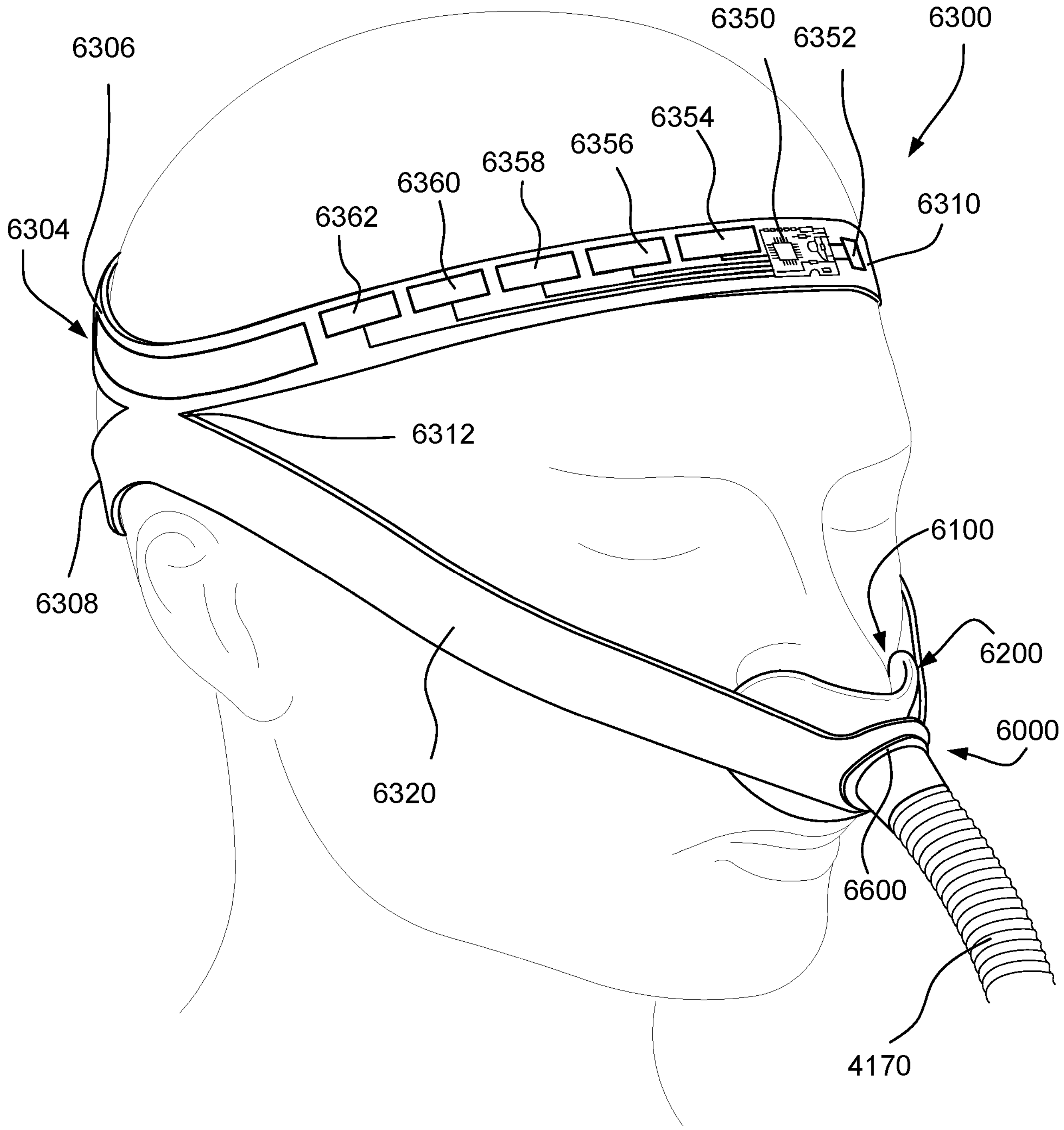
6306
6350
6352
6300
6354
6356
6358
6360
6362
6304
6310
6312
6100
6308
6200
6000
6320
6600
4170

FIG 7B

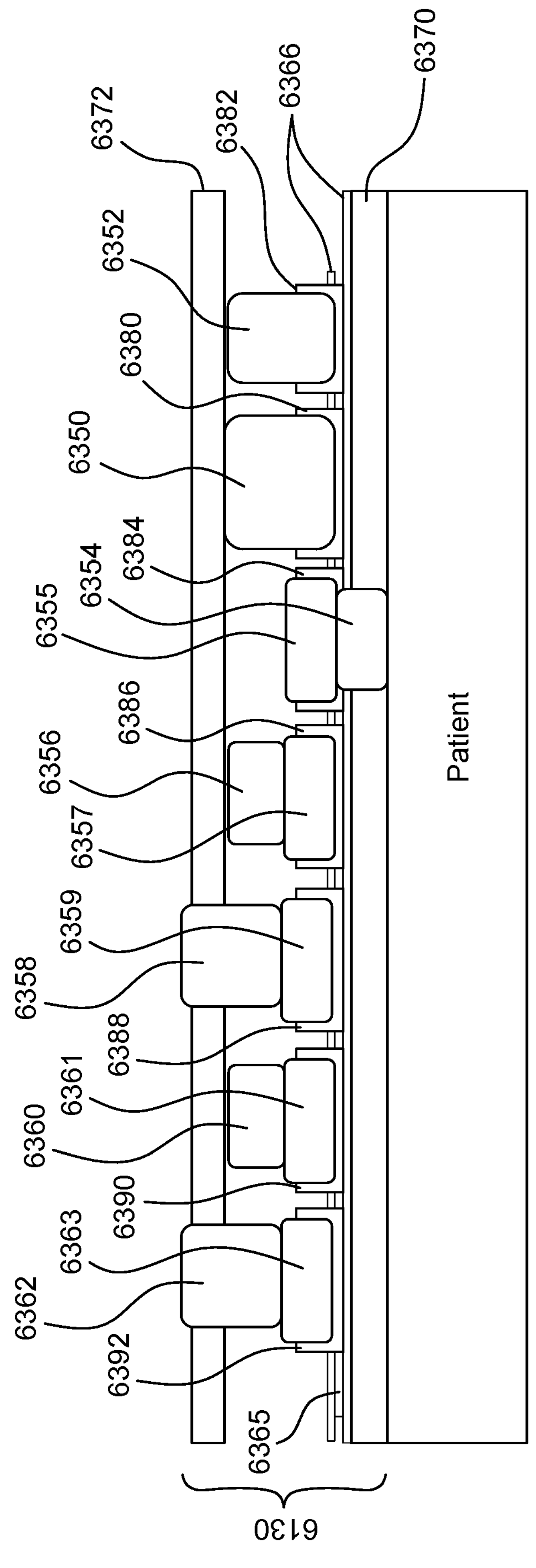
6372
6382
6366
6370
6352
6380
6350
6384
6354
6355
6386
6356
6357
6359
6358
6388
6361
6360
6390
6363
6362
6392
6365
6130
Patient

FIG 8

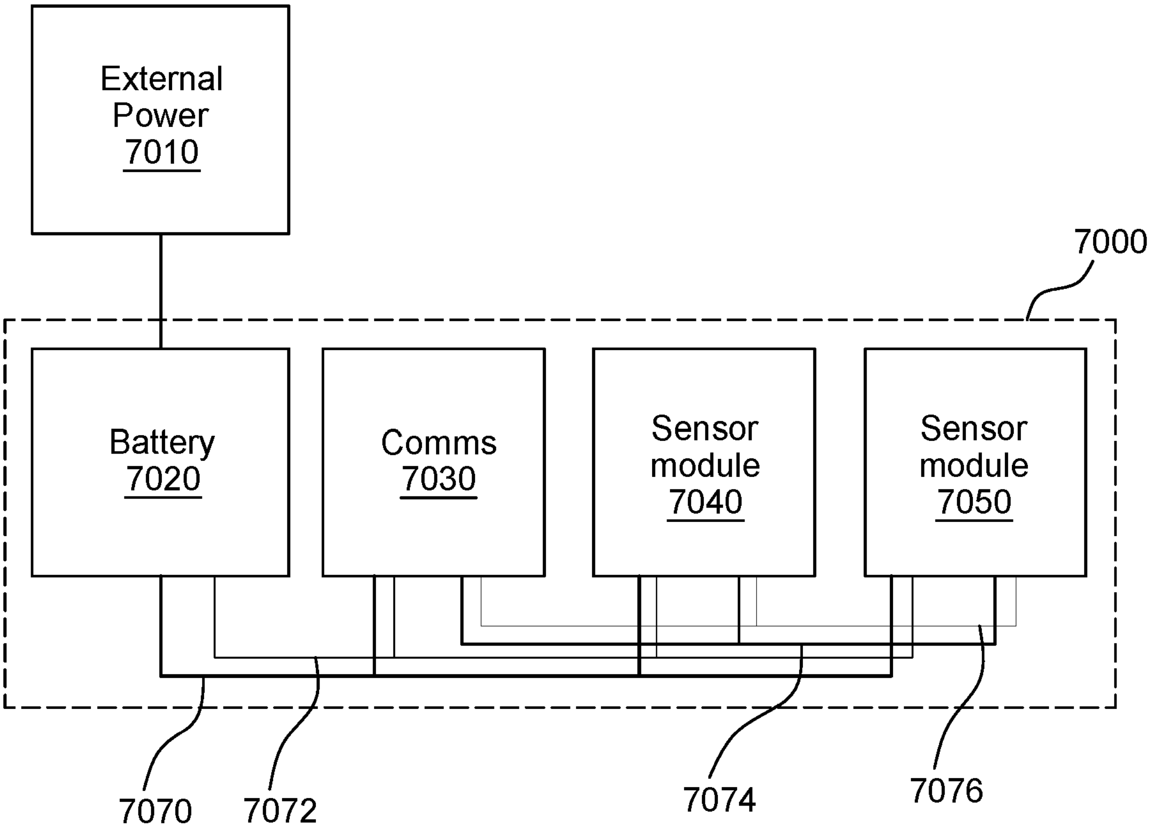
External Power 7010
7000
Battery 7020
Comms 7030
Sensor module 7040
Sensor module 7050
7070
7072
7074
7076

FIG 9A

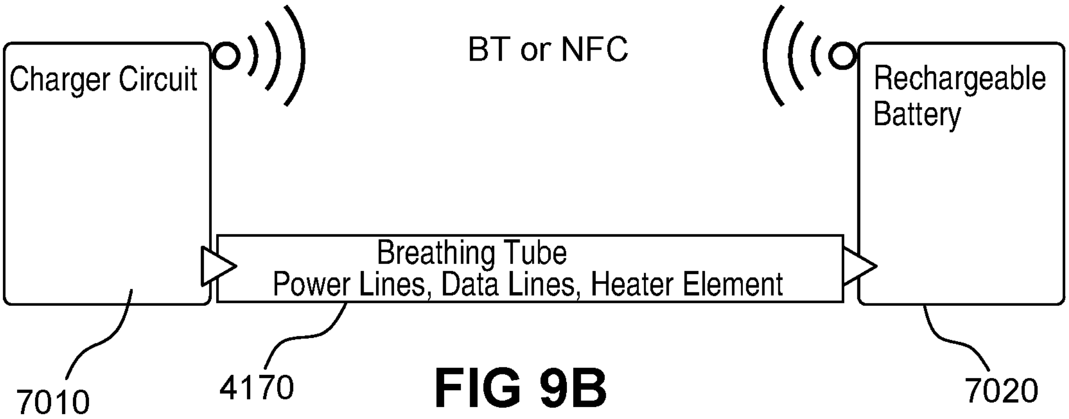


FIG 9B

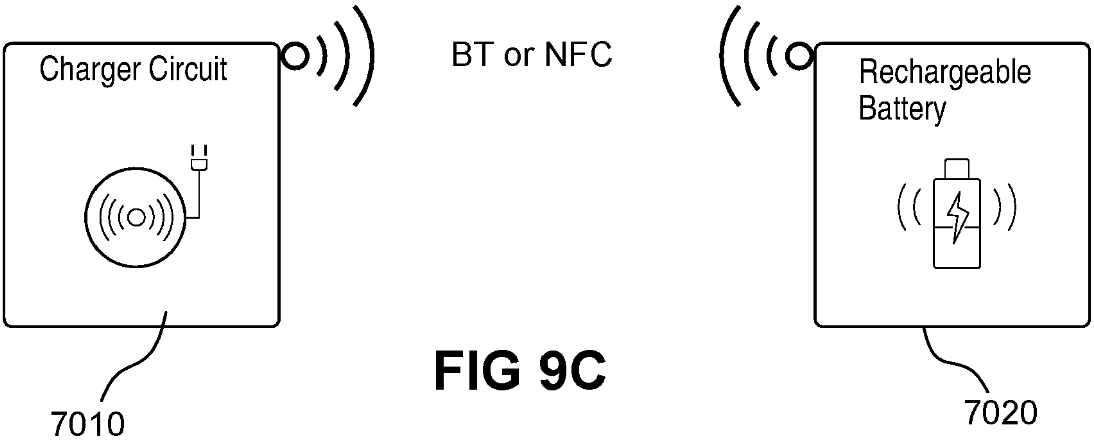


FIG 9C

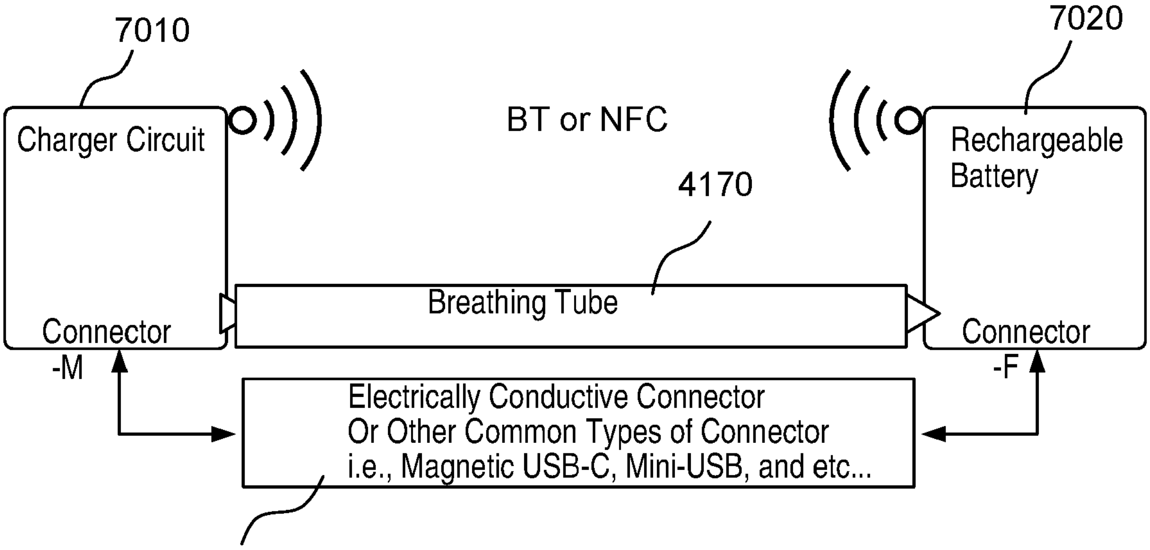


FIG 9D

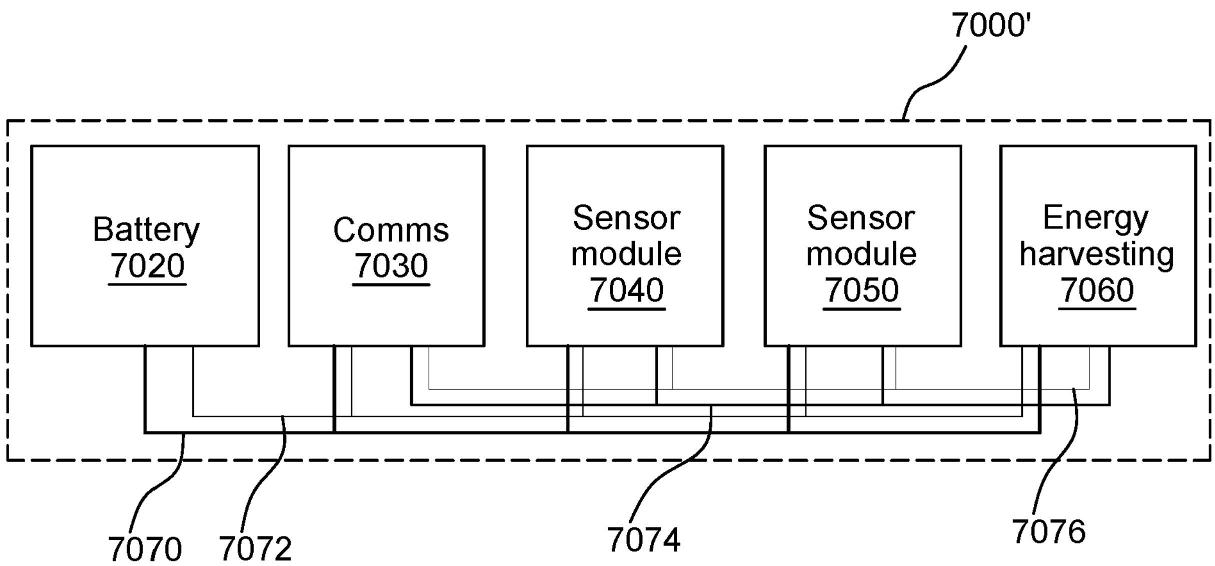
7000'
Battery
7020
Comms
7030
Sensor
module
7040
Sensor
module
7050
Energy
harvesting
7060
7070
7072
7074
7076

FIG 10

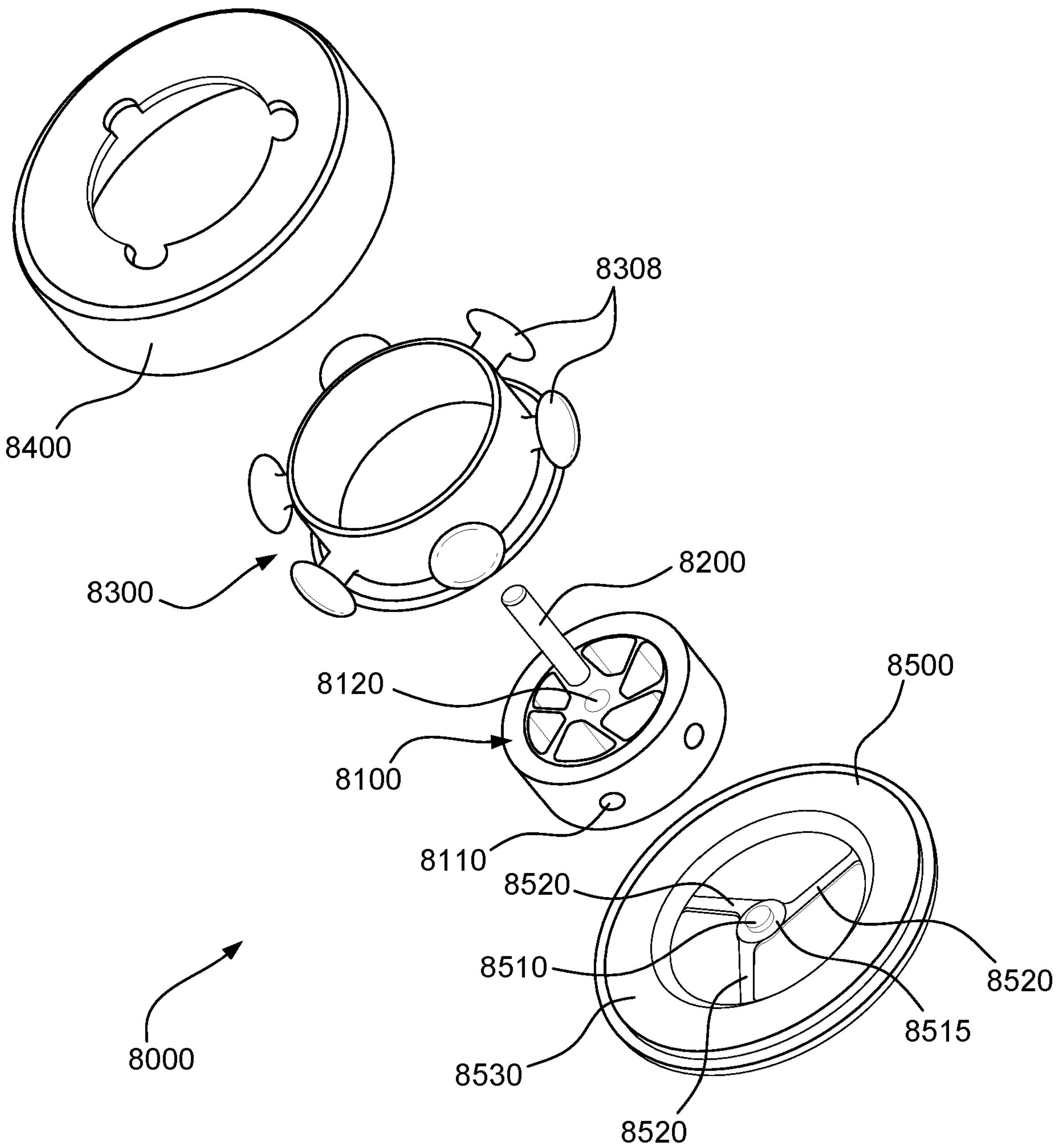
8308
8400
8300
8200
8120
8500
8100
8110
8520
8510
8520
8515
8000
8530
8520

FIG. 11A

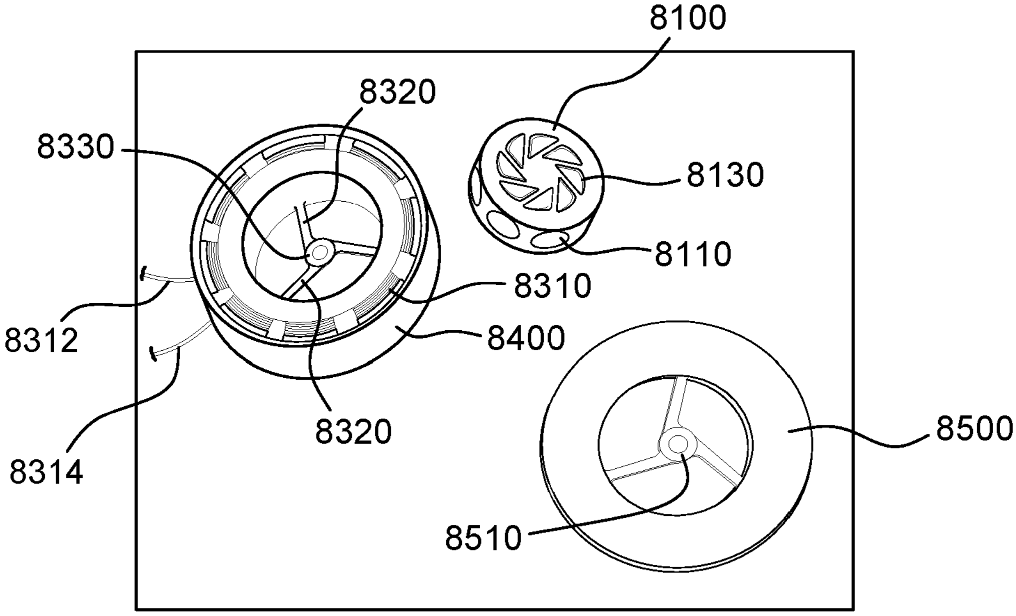


FIG 11B

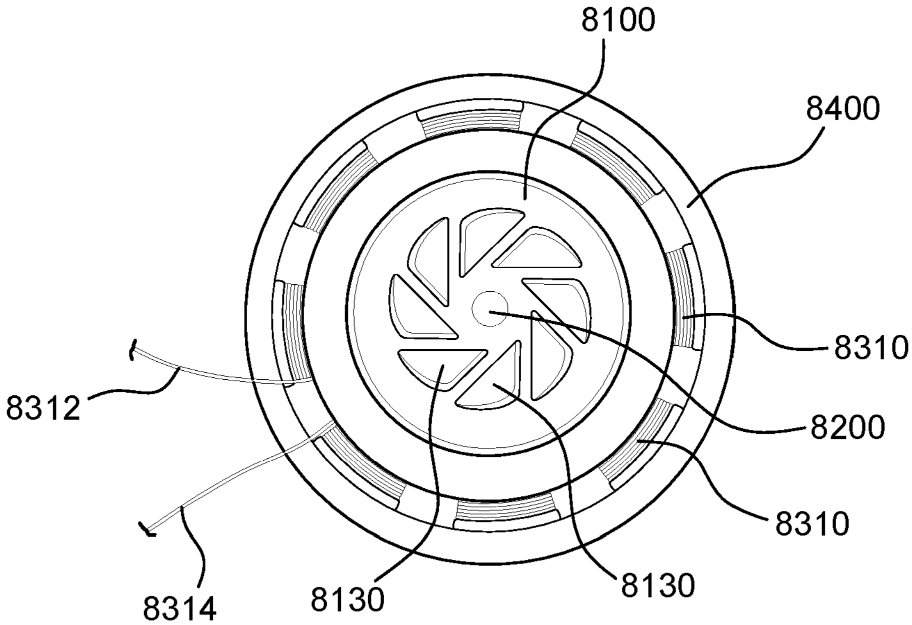


FIG 11C

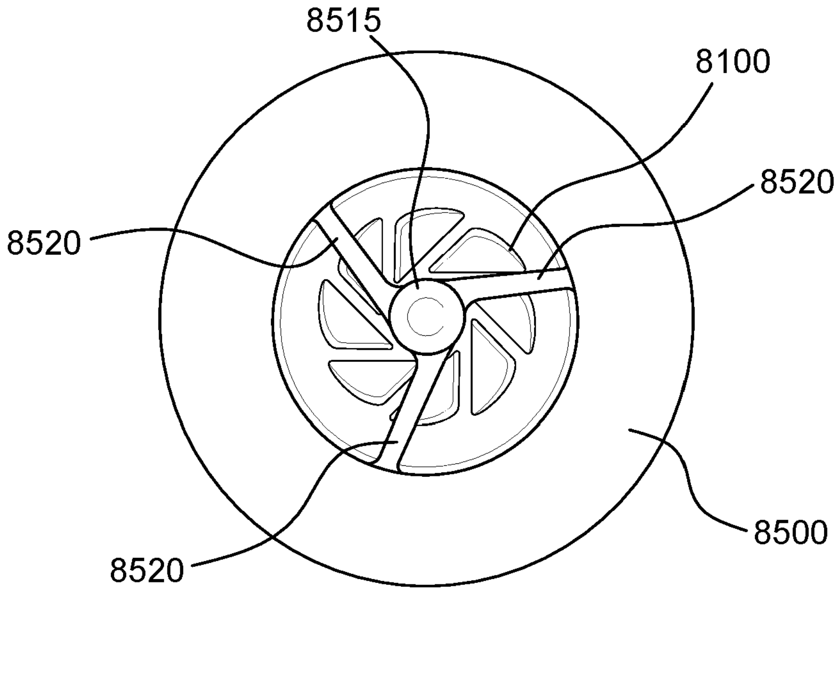


FIG 11D

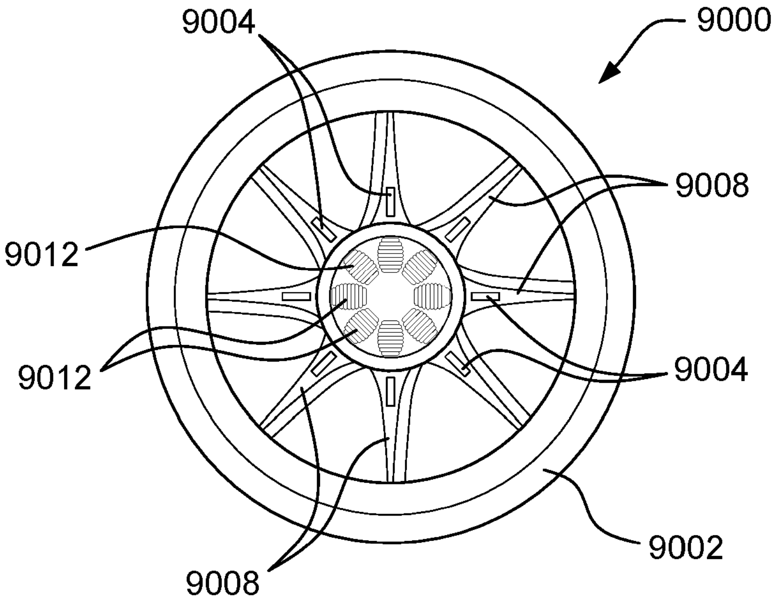


FIG 11E

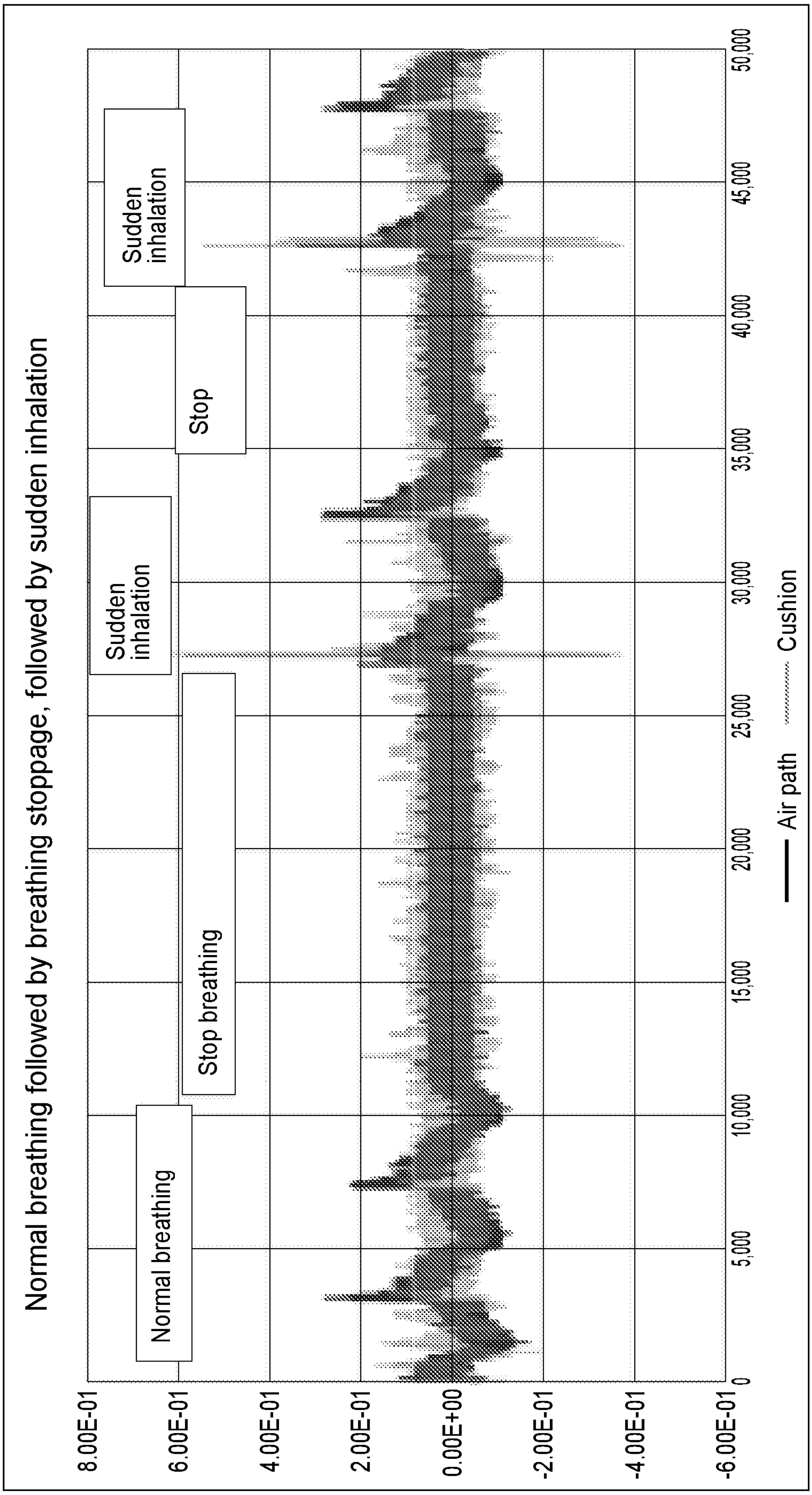
Normal breathing followed by breathing stoppage, followed by sudden inhalation
Normal breathing
Stop breathing
Sudden inhalation
Stop
Sudden inhalation
8.00E-01
6.00E-01
4.00E-01
2.00E-01
0.00E+00
-2.00E-01
-4.00E-01
-6.00E-01
0
5,000
10,000
15,000
20,000
25,000
30,000
35,000
40,000
45,000
50,000
Air path
Cushion

FIG. 12A

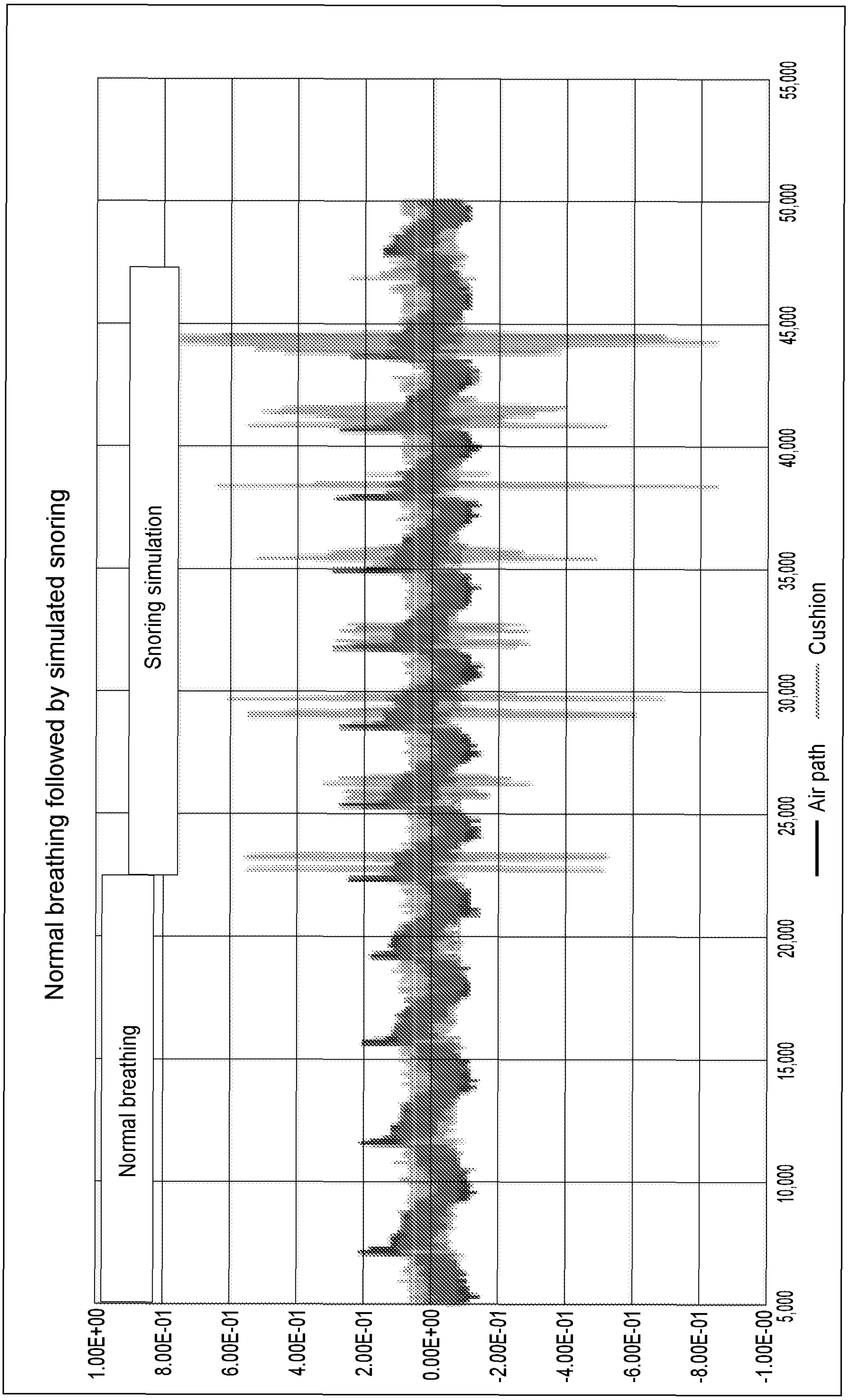
Normal breathing followed by simulated snoring
Normal breathing
Snoring simulation
1.00E+00
8.00E-01
6.00E-01
4.00E-01
2.00E-01
0.00E+00
-2.00E-01
-4.00E-01
-6.00E-01
-8.00E-01
-1.00E-00
5,000
10,000
15,000
20,000
25,000
30,000
35,000
40,000
45,000
50,000
55,000
Air path
Cushion

FIG. 12B

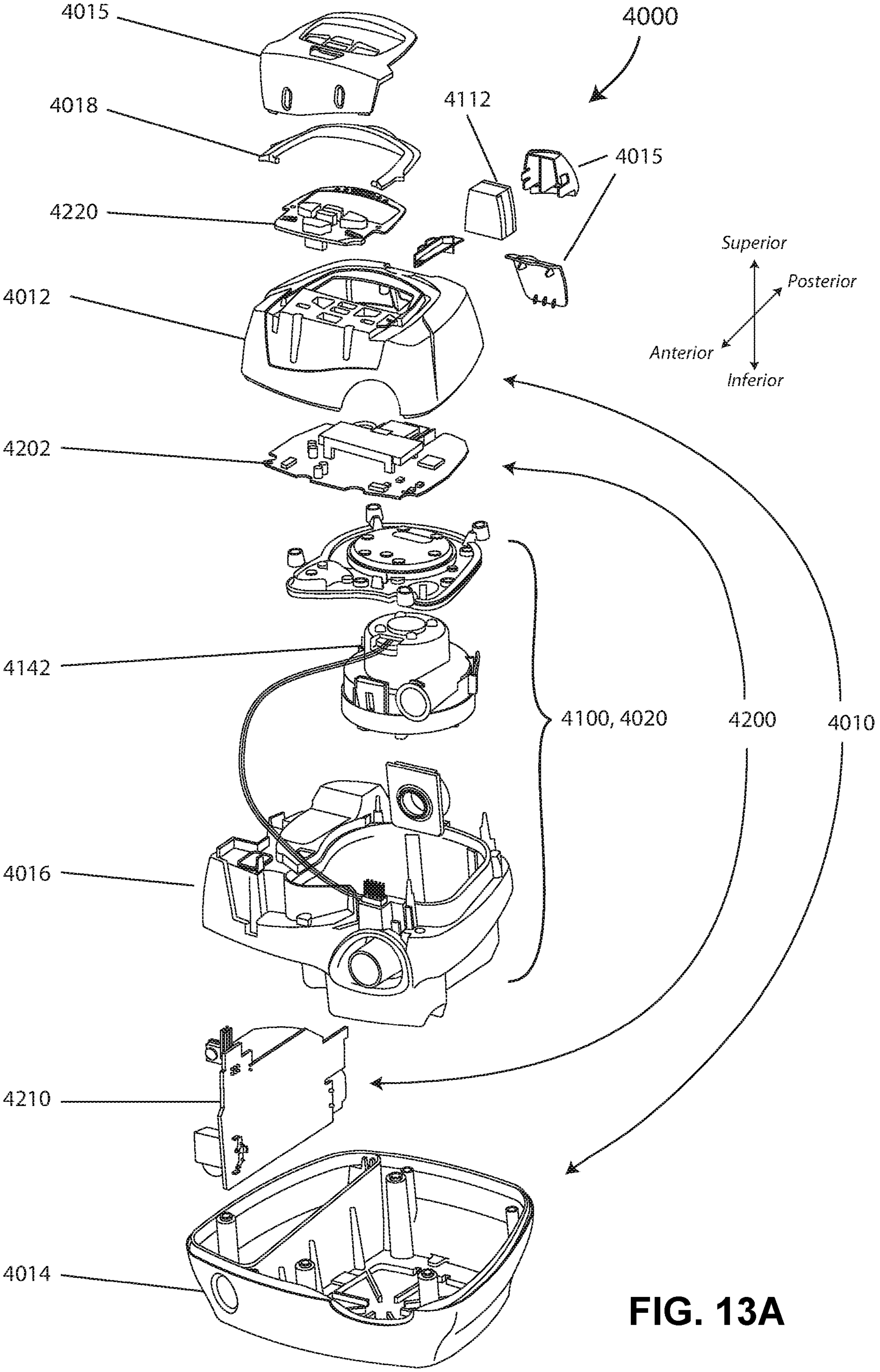
4000
4015
4018
4220
4012
4202
4142
4016
4210
4014
4112
4015
Superior
Posterior
Anterior
Inferior
4100, 4020
4200
4010

FIG. 13A

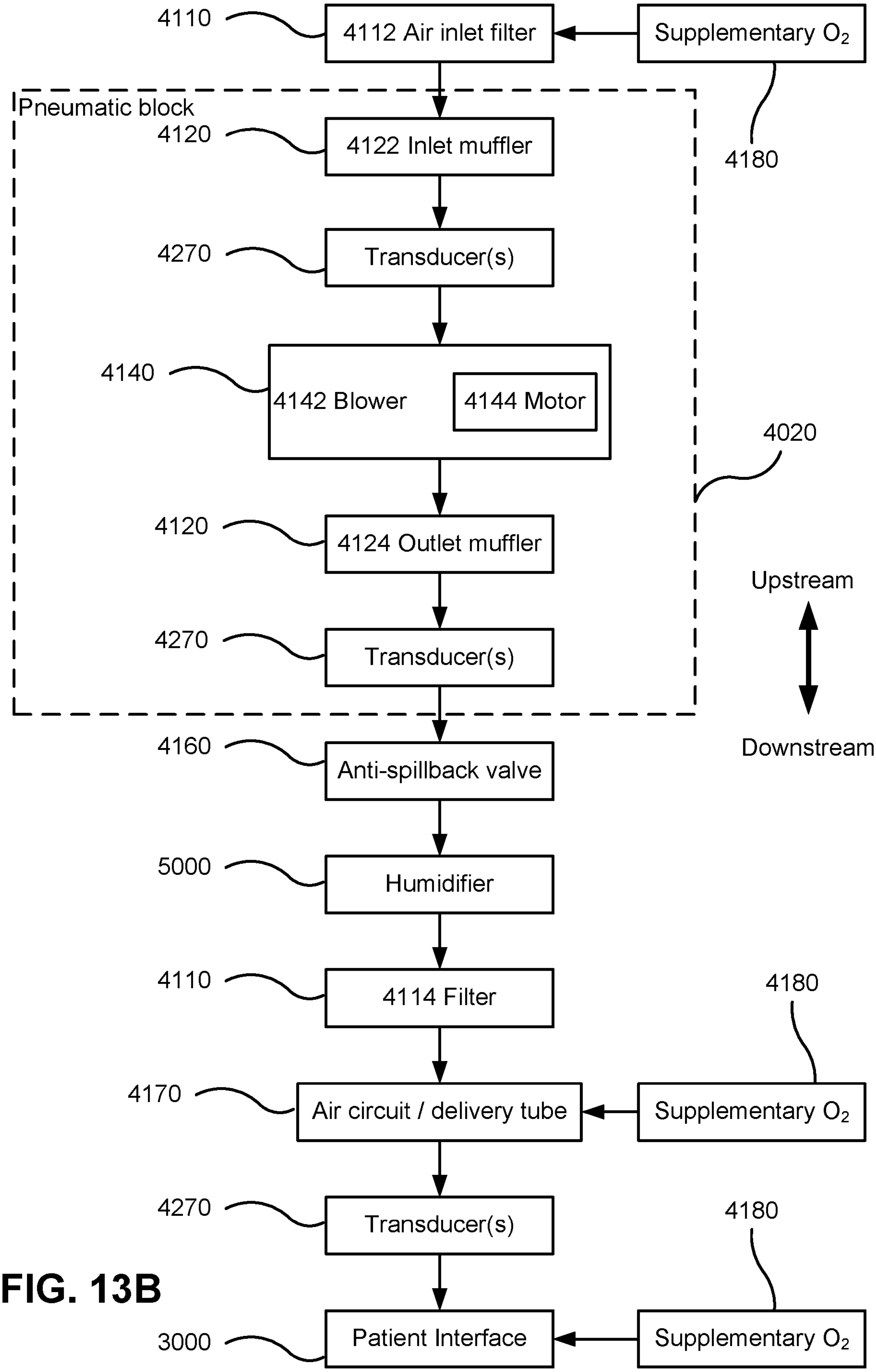
4110
4112 Air inlet filter
Supplementary O2
4180
Pneumatic block
4120
4122 Inlet muffler
4270
Transducer(s)
4140
4142 Blower
4144 Motor
4020
4120
4124 Outlet muffler
Upstream
4270
Transducer(s)
Downstream
4160
Anti-spillback valve
5000
Humidifier
4110
4114 Filter
4180
4170
Air circuit / delivery tube
Supplementary O2
4270
Transducer(s)
4180
3000
Patient Interface
Supplementary O2

FIG. 13B

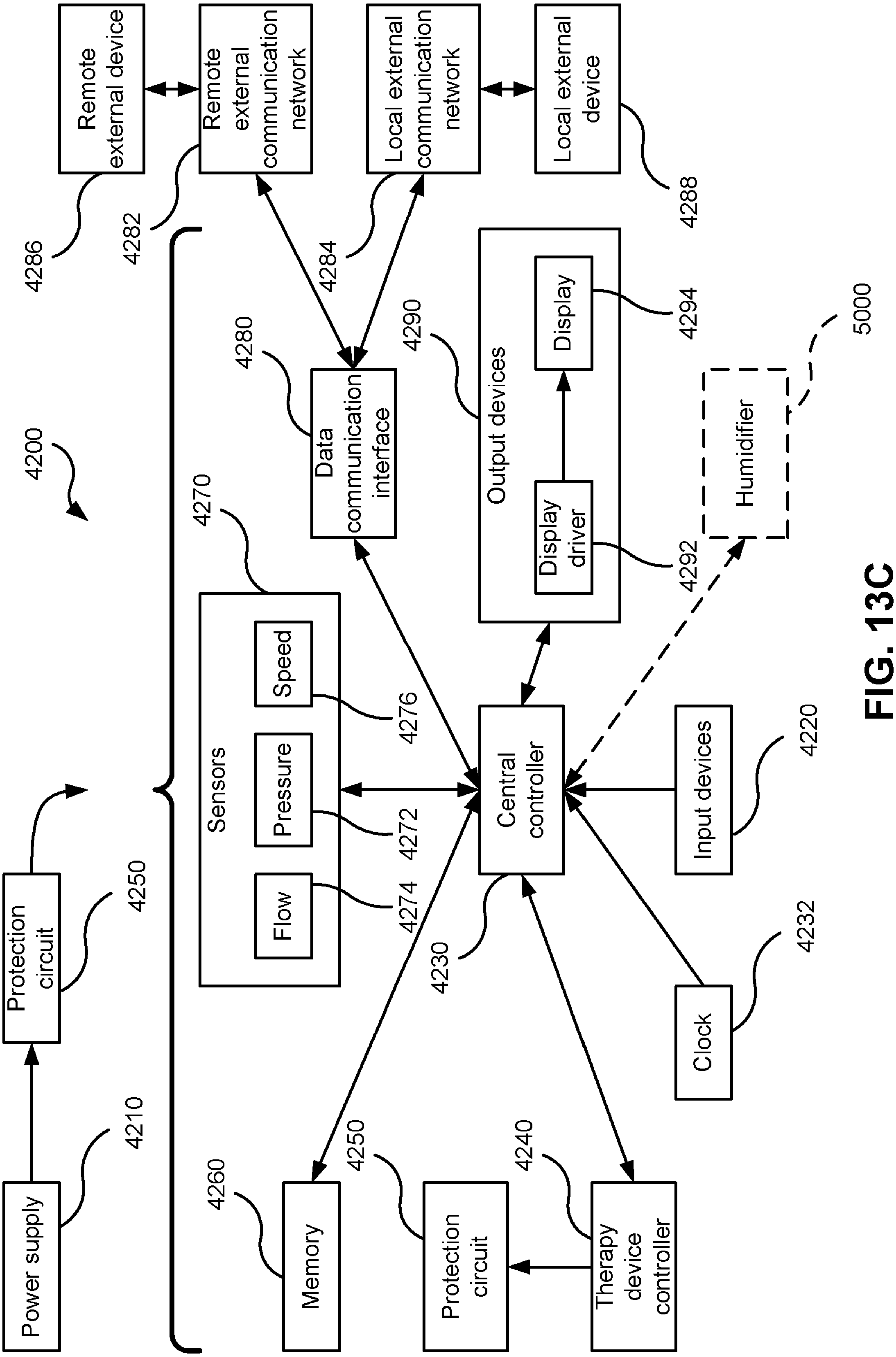
4200
Power supply
4210
Protection circuit
4250
Memory
4260
Protection circuit
4250
Therapy device controller
4240
Sensors
Flow
Pressure
Speed
4270
4274
4272
4276
Central controller
4230
Clock
4232
Input devices
4220
Data communication interface
4280
Output devices
4290
Display driver
4292
Display
4294
Humidifier
5000
Remote external communication network
4282
Remote external device
4286
Local external communication network
4284
Local external device
4288

FIG. 13C

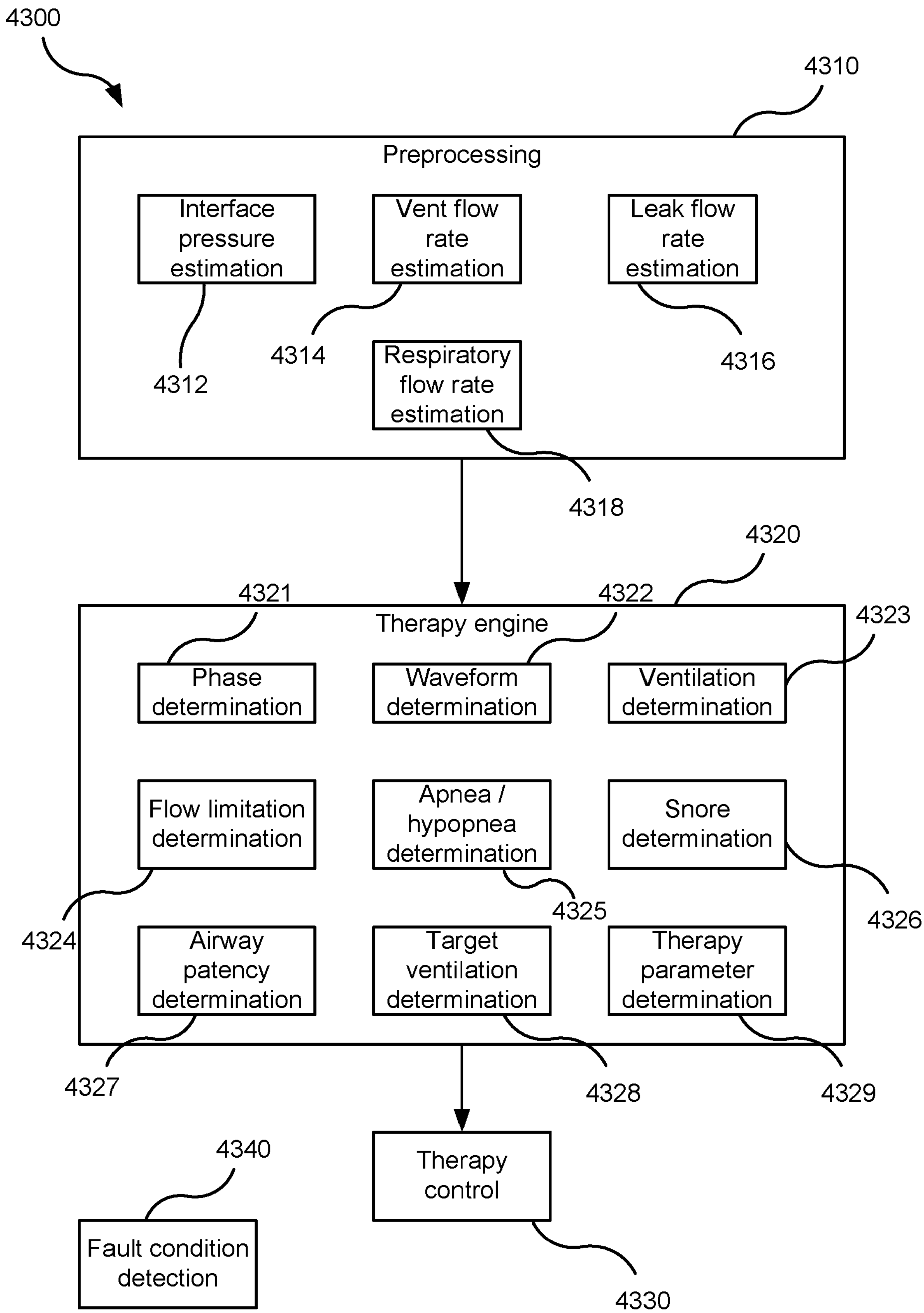
4300
4310
Preprocessing
Interface pressure estimation
Vent flow rate estimation
Leak flow rate estimation
4312
4314
4316
Respiratory flow rate estimation
4318
4320
4321
4322
4323
Therapy engine
Phase determination
Waveform determination
Ventilation determination
Flow limitation determination
Apnea / hypopnea determination
Snore determination
4324
4325
4326
Airway patency determination
Target ventilation determination
Therapy parameter determination
4327
4328
4329
4340
Therapy control
Fault condition detection
4330

FIG. 13D

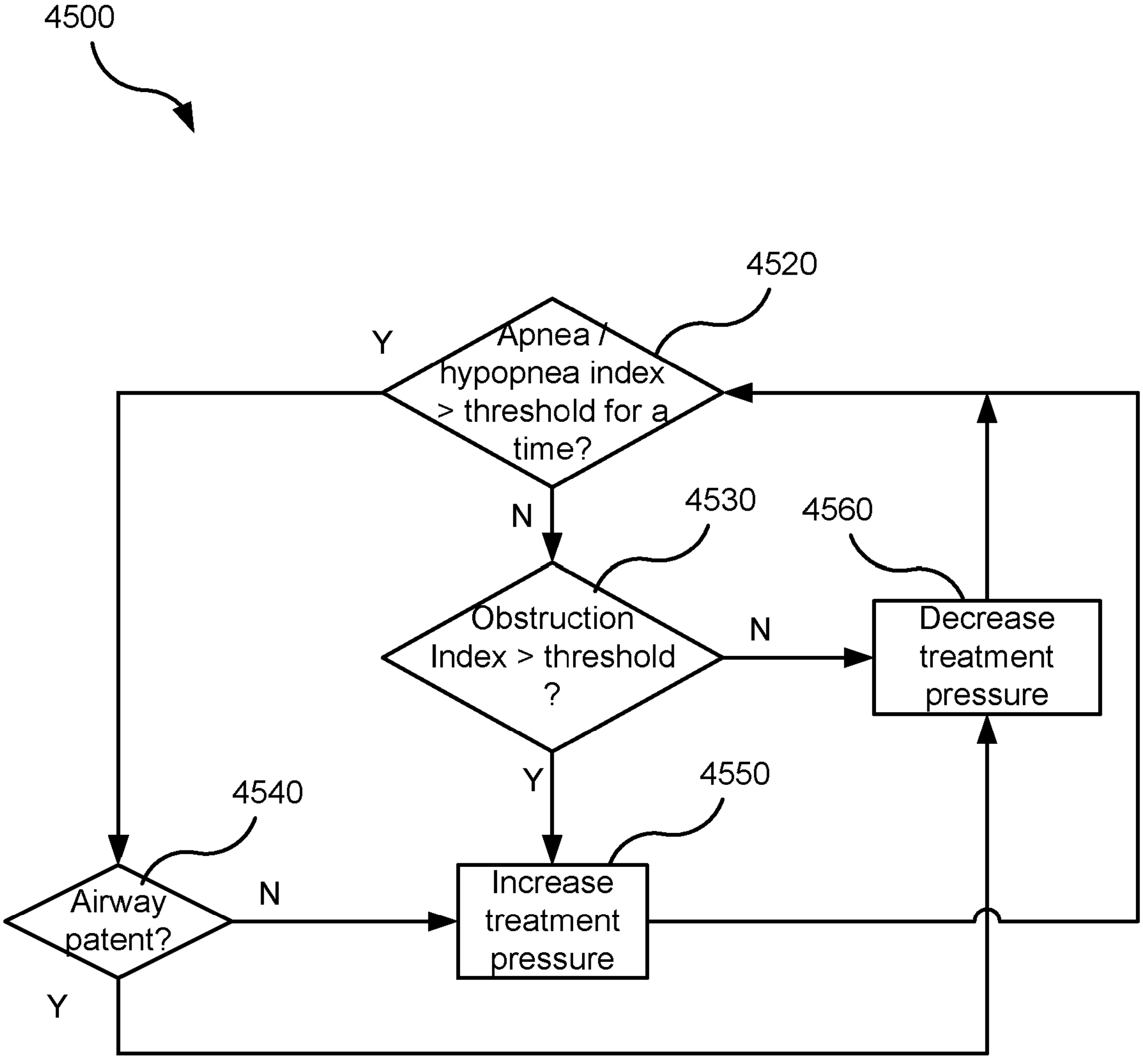
4500
4520
Apnea / hypopnea index > threshold for a time?
Y
N
4530
4560
Obstruction Index > threshold ?
N
Decrease treatment pressure
Y
4550
4540
Airway patent?
N
Increase treatment pressure
Y

FIG. 13E

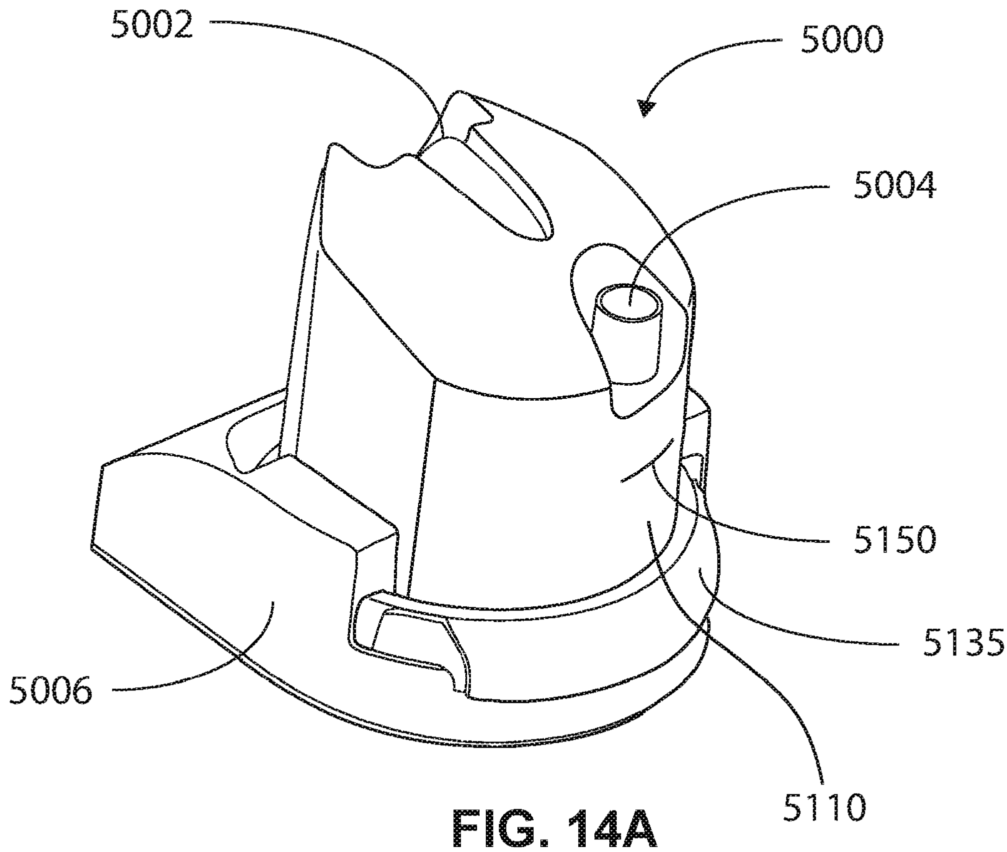


FIG. 14A

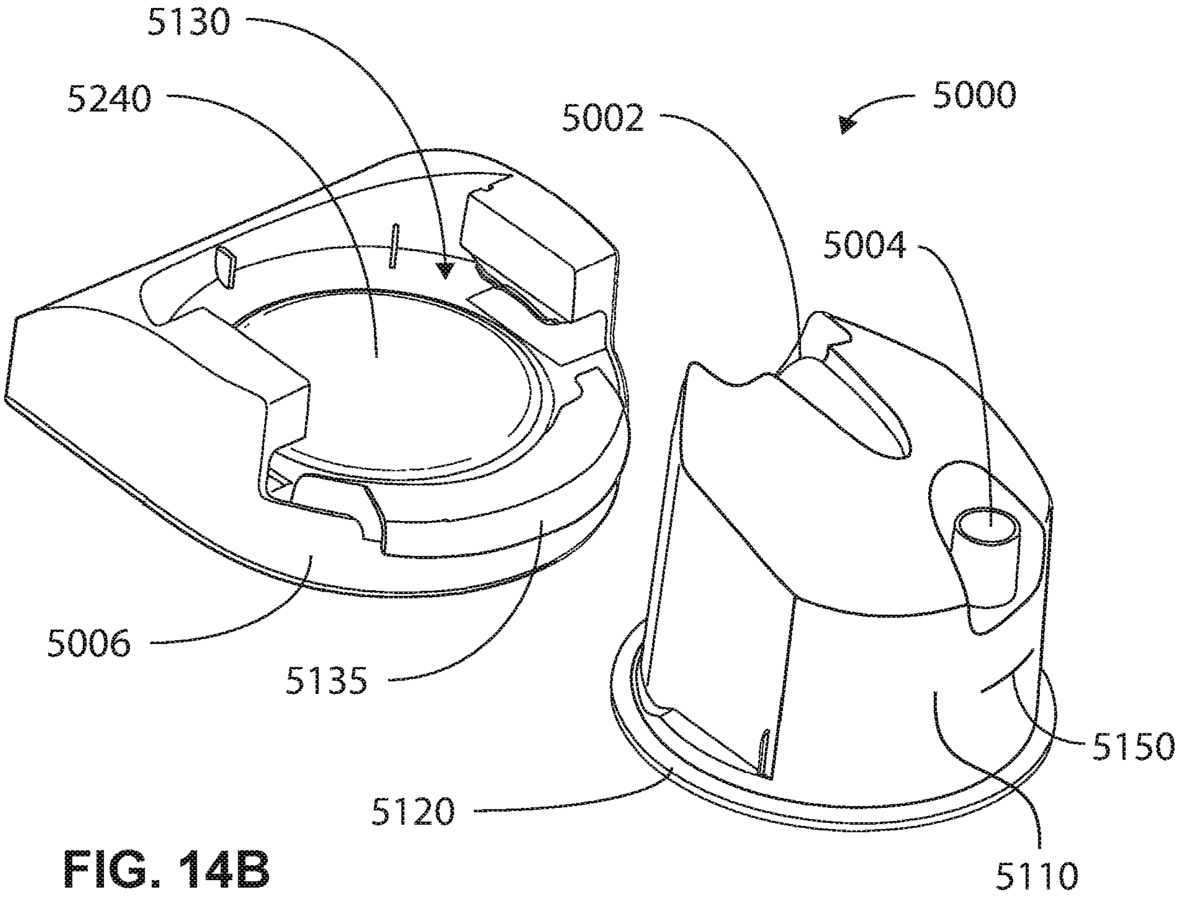


FIG. 14B

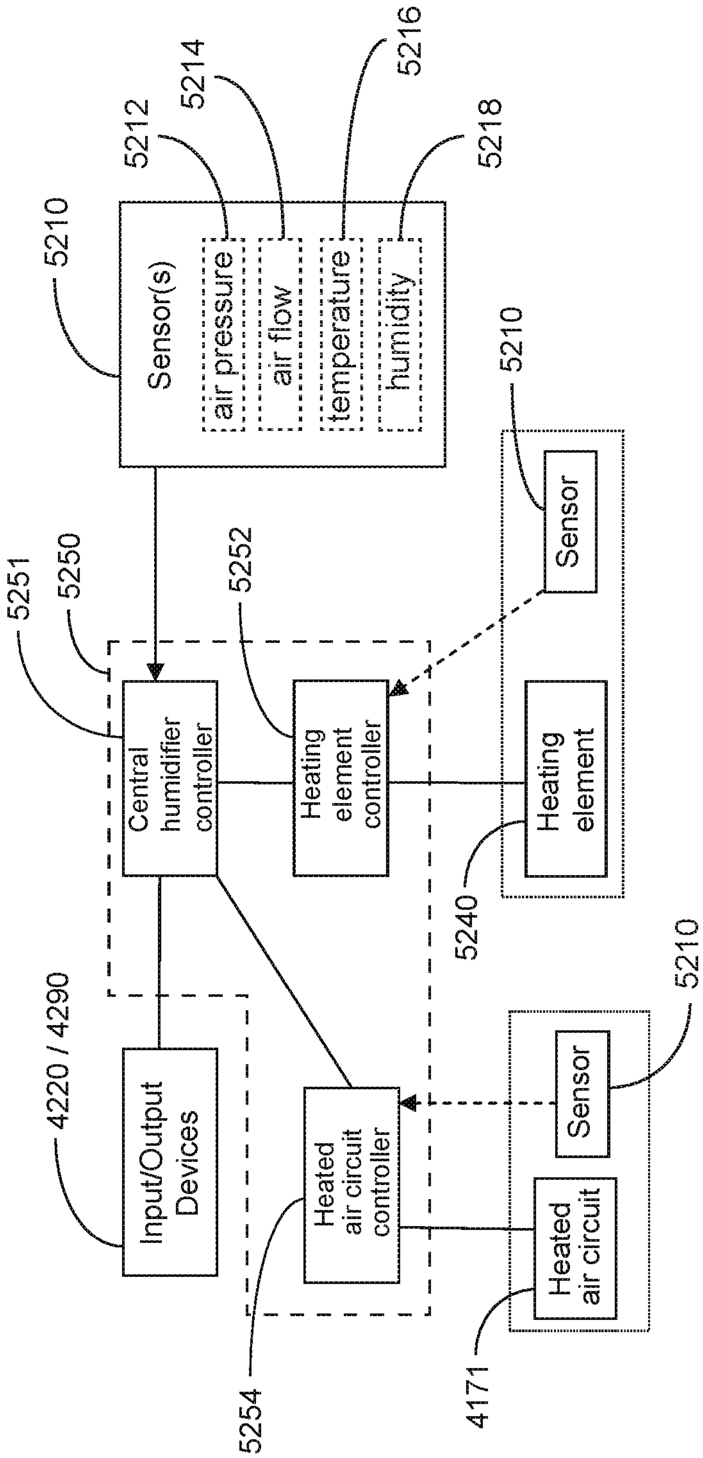
5210
Sensor(s)
air pressure
air flow
temperature
humidity
5212
5214
5216
5218
5251
5250
Central humidifier controller
5252
Heating element controller
Sensor
5210
Heating element
5240
4220 / 4290
Input/Output Devices
Heated air circuit controller
5254
Sensor
5210
Heated air circuit
4171

FIG. 14C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4944310 A, Sullivan
- US 6532959 B, Berthon-Jones
- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534 A
- WO 1998034665 A
- WO 2000078381 A
- US 6581594 B
- US 20090050156
- US 20090044808
- US 2014261425 A1
- US 2012234323 A1
- WO 2017185140 A
- US 7866944 B
- US 8638014 B
- US 8636479 B
- WO 2013020167 A
- US 8732349 B
- WO 201217072 A
- US 20090065007 A


### Non-patent literature cited in the description


- **JOHN B. WEST**. Respiratory Physiology. Lippincott Williams & Wilkins, 2012